# EUROPEAN PATENT APPLICATION

(11) **EP 4 725 502 A1**
(43) Date of publication of application: **15.04.2026**
(21) Application number: 25206743.4
(22) Date of filing: 04.10.2025
(51) Int. Cl.: A61K 40/17, A61K 41/00, C12N 5/0786, A61P 35/00, A61N 2/00, A61N 5/00, A61K 33/242, A61K 33/26

(54) **A METHOD TO CHANGE THE POLARIZATION OF TUMOR ASSOCIATED MACROPHAGES BY EXPOSING THEM TO A SOURCE OF RADIATION IN THE PRESENCE OF MAGNETOSOMES**

(30) Priority: 04.10.2024 EP 24020301; 23.10.2024 FR 2411540
(71) Applicant: AlphaOnco, 75016 Paris (FR); Université Paris Cité, 75006 Paris (FR); Centre National De La Recherche Scientifique, 75794 Paris Cédex (FR); Université de Franche-Comté, 25030 Besançon (FR)
(72) Inventor: Bécharef, Sonia, 75013 Paris (FR); Gazeau, Florence, 75013 Paris (FR); Roux, Stéphane, 25030 France (FR); Luciani, Nathalie, 75013 Paris (FR); Jabbour, Léa, 75013 Paris (FR); Amanda, Silva, 75013 Paris (FR); Alphandéry, Edouard, 75016 Paris (FR); Herbeuval, Jean-Philippe, 75270 Paris Cedex 06 (FR)
(74) Representative: Jorget, Quentin

(57) **Abstract**

The invention relates to a method to control or change the polarization of at least one macrophage associated with a tumor or pathological site (TAM), from being predominantly a TAM with either no polarization (M0) or a polarization i (Mi) before at least one step of the method, to a predominant polarization j (Mj), by associating at least one TAM with a nanoparticle, preferentially a magnetosome, and by exposing the assembly to a radiation, where Mi = M1 and Mj = M2 or Mi = M2 and Mj = M1.

## Description

### FIELD OF THE INVENTION

The field of the invention concerns the description of a method to change the polarization of tumor associated macrophages (TAM) by associating these cells with nanoparticles or magnetosomes and by exposing the assemblies to a source of radiation.

### STATE OF THE ART

It is well known that some types of immune cells, i.e. tumor associated with macrophages, can change their state from being unpolarized to polarized, and can then also switch polarization state from a first to a second polarization state. Such changes are important, since the polarization state is associated with some specific immune mechanisms, being for example associated with either pro or anti-tumor activity depending on polarization state, (Chen et al, Macrophages in immunoregulation and therapeutics, Signal Transduction and Targeted Therapy, 8:207 (2023)).

### DESCRIPTION OF THE INVENTION

Although polarization states of immune cells such as Tumor associated macrophages (TAM) have been described in the literature, a method to control such states with specific types of nanoparticles such as magnetosomes, as that presented in this disclosure, has not been described.

The invention relates to a method preferentially to control or change the polarization of preferentially at least one immune cell or macrophage or macrophage associated with a tumor or pathological site (TAM) or entity, from being predominantly a immune cell or macrophage or TAM or entity with either no polarization, preferentially M0, or preferentially a polarization i (Mi) preferentially before at least one step of the method, preferentially to a predominant polarization j (Mj) preferentially after at least one step of the method, wherein the method preferentially comprises the following steps or at least one or all of the following steps:
- Preferentially Step 1 providing i) and ii):
   i) At least one nanoparticle which is:
      - Preferentially a magnetosome synthesized by a magnetotactic bacterium or by at least one of its components, preferentially not comprising at least one non-denatured protein and/or lipid and/or LPS and/or DNA and/or RNA preferentially originating from or produced by or comprised in a magnetotactic bacterium or at least one of its components,
      - Preferentially a chemically synthesized nanoparticle, preferentially having at least one property in common with a magnetosome, preferentially selected in the group consisting of: i) a ferrimagnetic or ferromagnetic behavior or property, preferentially at a temperature below a blocking or melting or Curie temperature or below 273, 100, 50, 10, 5, 2 or 0.1 K, ii) an arrangement in chain or in a geometric figure, iii) a percentage in iron larger than 50, 75, 90, 95 or 99%, preferentially in mass or volume, preferentially in terms of metallic composition, preferentially in the core or crystallized part or most crystallized part of the nanoparticle, iv) the presence of a core, preferentially ferrimagnetic and/or metallic, preferentially faceted and/or crystallized, preferentially with at least one property in common with the core of a magnetosome not comprising partly or fully the biological magnetosome membrane synthesized by a magnetotactic bacterium, and v) the presence of a coating surrounding partly or fully the core, preferentially not synthesized by a magnetotactic bacterium,
         and/or
      - Preferentially a nanoparticle that has at least one property selected in the group consisting of:
         1) It is composed of or comprises at least 1 or 2 metal(s) or metal atom(s), which is or are preferentially surrounded by or mixed with or bound through or interacting through or assembled by part of or the entire of an organelle or cellular vesicle or endosome or exosome or lysosome or cellular compartment, preferentially intracellular, most preferentially inside an eukaryotic cell, preferentially belonging to or originating from a pathological cell,
            and
         2) It forms a nanoparticle or a particle with a size smaller than 1000, 100, 50, 20, 10, 5, 2 or 1 nm, preferentially in at least one dimension or cross section of the nanoparticle or particle, through the composition of at least 1 or 2 metals or metal atoms, which is/are preferentially surrounded by or mixed with or bound through or interacting through or assembled by or transformed into a nanoparticle or particle that preferentially has at least one property that differs from that of a single atom, or made crystallized or magnetic, preferentially diamagnetic, paramagnetic, superparamagnetic, ferromagnetic or ferrimagnetic, preferentially by part of or the entire of an organelle or cellular vesicle or endosome or exosome or lysosome or cellular compartment, preferentially intracellular, most preferentially inside an eukaryotic cell, preferentially belonging to or originating from a pathological cell,
            and
   ii) Preferentially at least one immune cell or macrophage or TAM or entity, preferentially having at least one property selected in the group consisting of: i) it is a TAM with either no polarization (M0) or with polarization i (Mi), ii) at least two TAM forming an assembly of TAM with predominantly polarization(s) (M0) and/or (Mi) and/or with a minority of or no polarization (Mj), ii) it is comprised in or is extracted from or originates from or is produced by or is differentiated in or by a pathological site or tumor or body part to be treated or its/their environment or microenvironment, partly or fully, iii) it is differentiated from a mast or stem or un-differentiated cell, preferentially before step 1, iv) it is moving or in movement or is migrating, preferentially from a site in which it is produced such as a bone marrow or another body part preferentially different from the body part to be treated or pathological site to the body part to be treated such as a pathological site or tumor or from one portion of the body part to be treated to another portion of the part to be treated, and v) it is provided, collected, harvested, detected, differentiated, isolated, preferentially from TAM or cells different from TAM, activated, polarized, preferentially by the method, preferentially while it is moving in or has moved to or from and/or is comprised in and/or is in interaction with the body part to be treated or pathological site or at least one of its/their compound(s) or component(s);
      - Preferentially Step 2 comprising assembling and/or mixing, and/or conjugating, and/or complexing at least one nanoparticle preferentially of or originating from step 1 with at least one immune cell, macrophage or TAM or entity preferentially of or originating from step 1 by:
         i) Administering and/or assembling and/or mixing and/or conjugating and/or complexing the at least one nanoparticle preferentially of or originating from step 1 preferentially to or with a body part preferentially to be treated preferentially comprising at least one immune cell, macrophage, TAM and/or one of its entity or compound or organelle, preferentially of or originating from step 1;
         ii) Bringing together the at least one immune cell, macrophage, TAM or entity and/or one of its entity or compound or organelle, preferentially of or originating from step 1, and at least of nanoparticle preferentially of or originating from step 1;
         iii) Internalizing at least one nanoparticle preferentially of or originating from step 1 in at least one immune cell, macrophage, TAM, or entity and/or one of its entity or organelle or compound preferentially of or originating from step 1;
         iv) Externalizing or expelling at least one nanoparticle preferentially of or originating from step 1 from at least one immune cell, macrophage, TAM or entity and/or one of its entity or compound or organelle, preferentially of or originating from step 1;
            and/or
         v) Associating and/or assembling and/or mixing and/or conjugating and/or complexing at least one nanoparticle preferentially of or originating from step 1 and at least of immune cell, macrophage, TAM or entity and/or one of its entity or compound or organelle, preferentially of or originating from step 1, wherein the at least one nanoparticle and immune cell, macrophage, TAM and/or one of its entity or compound or organelle, preferentially of or originating from step 1, interact and/or are brought and/or are assembled and/or are mixed and/or are conjugated and/or complexed preferentially together preferentially through:
            - dispersion, dipole-dipole, H-bonding, ion-dipole, covalent, ionic, intermolecular, and/or intramolecular interaction(s),
            - at least one first type of interaction having a strength or energy of interaction, preferentially in at least one first location of the assembly or for or in a first portion of the assembly that is weak or weak enough to allow the dissociation of the at least one nanoparticle from immune cell, macrophage, TAM or entity and/or one of its entity or compound or organelle, preferentially under or following irradiation of the assembly or of one of its components, or smaller than 1000, 100, 50, 5, 2 or 1 KJ/mol of interactions, and/or
            - at least one second type of interaction having a strength or energy of interaction, preferentially in at least one second location of the assembly or for or in a second portion of the assembly that is strong or strong enough to prevent the dissociation of the at least one nanoparticle from immune cell, macrophage, TAM or entity and/or one of its entity or compound or organelle, preferentially under or following irradiation of the assembly or of one of its components, or larger than 0, 1, 5, 10, 50, 100 or 1000 KJ/mol of interactions;
            - Preferentially Step 3 comprising exposing or having exposed or letting exposed, preferentially by the body part preferentially to be treated, or at least one compound or radiation in or outside the body part preferentially to be treated, the nanoparticle(s) and/or immune cell, macrophage, TAM or entity and/or one of its entity or compound or organelle, preferentially of or originating from step 1 and/or assembly, and/or mixture, and/or conjugate, and/or complex and/or composite and/or compound and/or blend of nanoparticle(s) and/or immune cell, macrophage, TAM and/or one of its entity or compound or organelle, preferentially of or originating from step 2 and/or preferentially its/their environment or microenvironment to at least one of:
               i) a variation of at least one radiation parameter, preferentially a variation of the type, intensity, power, power density, frequency, wavelength, penetration depth, duration of application, and/or number of application times of the radiation that is/are preferentially larger than or equal to 10-²⁰, 10⁻¹⁰, 10⁻⁵, 10⁻¹, 0, 1, 2 or 5 preferentially Watt, Hz, KHz, MHz, nm, second, hour, day, preferentially per cell, TAM or entity, nanoparticle and/or body part, preferentially per cm, cm2 or cm3 of cell, immune cell, macrophage, TAM or entity and/or one of its entity or compound or organelle, nanoparticle and/or body part;
               ii) a movement and/or displacement, preferentially by more than 10⁻²⁰, 10⁻⁵, 10⁻¹, 0, 1 or 5 m, cm or nm, preferentially a movement and/or displacement that is more important for at least one immune cell, macrophage, TAM and/or one of its entity or compound or organelle than at least one nanoparticle, preferentially following engulfment or capture or internalization of the at least one nanoparticle in at least one TAM or entity, optionally following exposure of the TAM or entity and/or nanoparticle to radiation;
               iii) a temperature gradient, preferentially of more than 10⁻²⁰, 10⁻⁵, 10⁻¹, 0, 1 or 5 °C or K;
               iv) a pH gradient, preferentially of more than 10⁻⁵, 10⁻¹, 0, 1 or 5 pH unit(s);
                  and/or
               v) a composition gradient, preferentially by more than 0, 1, 5, 10, 20, 10², 10³ or 10⁵ preferentially per nanoparticle, immune cell, macrophage, TAM or entity and/or one of its entity or compound or organelle, body part, nm³ or cm³.
wherein preferentially the environment of the nanoparticle(s) and/or immune cell, macrophage, TAM and/or one of its entity or compound or organelle is preferentially at least one region or volume comprising the nanoparticle(s) and/or immune cell, macrophage, TAM or entity and/or one of its entity or compound or organelle, that is preferentially smaller than the individual or body part of the individual preferentially to be treated or diameter or thickness of body part of the individual or than 1, 0.1 or 0.001 meter.

Optionally step 4 of:
Stopping the application of radiation preferentially after step 3 or at least one step of the method; or
Exposing the assembly or mixture or conjugate or complex, or compound, or composite, or blend, preferentially resulting or originating from step 3 of the method or from at least one step of the method, to a different source of radiation than that applied in step 3 or in at least one step of the method or to the same source of radiation as that applied in step 3 or in at least one step of the method, but with at least one of its parameter with a different value than in step 3 or in at least one step of the method. Optionally step 5 of detecting or measuring or differentiating at least one cell or cell component, being a monocyte or immune cell, macrophage, TAM or entity, M0, M1, M2 and/or one of its entity or compound or organelle or property associated with the at least one state of the cell or cell component, such as the morphology, size, directional movement, production of type, size, number of immune entities or the cell, preferentially associated with its undifferentiated, differentiated, unpolarized, or polarized state, M0, M1 and/or M2.
   - Optionally step 6 of differentiating at least one cell, preferentially a immune cell, preferentially undifferentiated, preferentially a monocyte, into an unpolarized cell, preferentially an unpolarized immune cell, preferentially a macrophage or TAM or entity by:
      i) associating or making or letting interact or contact at least one cell or immune cell or entity, preferentially undifferentiated, preferentially a monocyte with at least one entity that differentiates the at least one cell or immune cell, preferentially undifferentiated, preferentially a monocyte, into a differentiated cell or entity, preferentially a TAM,
         and/or
      ii) associating or making or letting interact or contact at least one nanoparticle preferentially of or originating from step 1 with at least one cell or entity or immune cell, preferentially undifferentiated, preferentially a monocyte, and of exposing the assembly of the at least one nanoparticle and at least one cell or immune cell, preferentially undifferentiated, preferentially a monocyte, to at least one radiation, wherein the at least one cell or entity or immune cell, preferentially undifferentiated, preferentially a monocyte, is preferentially a mast or stem or embryonic or fetal or Yalk sac or Erhythro-myeloid progenitor or bonne marrow or hematopoietic stem or monocyte-derived macrophage cell,
         wherein preferentially the immune cell or entity, macrophage, TAM and/or one of its entity or compound or organelle, is or belongs to or originates from or is produced by or diffuses in or from or to: a differentiated cell or a cell of an organ or body part or tissue or lung, heart, liver, brain, intestine, skin, kidney, blood, and/or spleen,
         wherein preferentially Mi = M1 and Mj = M2 or Mi = M2 and Mj = M1,
         wherein preferentially the pathological site is a site preferentially comprised in a body part preferentially to be treated that preferentially comprises at least one tumor or pathological cell, virus, bacterium, and/or pathological material,
         wherein the method preferentially does not comprise or involve magnetogenetics.

In some cases, an organelle or cellular vesicle or endosome or exosome or lysosome has at least one property selected in the group consisting of:
i) it is responsible for the formation or forms the nanoparticle, preferentially by binding the at least 2 metals or metal atoms together,
ii) it protects the nanoparticle against degradation,
iii) it polarizes TAM or entity,
iv) it forms a layer or multilayer or assembly around or with the nanoparticle or at least two metals or metal atoms,
   and
v) it can be separated from the nanoparticle preferentially its metallic part preferentially by being heated together with the at least two metals or metal atoms.

As used herein, the terms "predominant", "predominantly", "principal", "principaly" used interchangeably in relation to macrophage polarisation can refer to the majority or dominant polarisation state of the macrophage at a given time. This can mean that more than 50%, 60%, 70%, 80%, 90%, 95% or 99% of macrophages have adopted a certain polarisation profile (for example, M0, Mi, Mj).

In some cases "predominant polarisation (M0)" can mean that the majority of macrophages are in a non-polarised state (M0), whereas "predominant polarisation j (Mj)" can mean that the majority of macrophages have adopted Mj polarisation.

In one embodiment of the invention, the at least one immune cell or macrophage or macrophage associated with a tumor or pathological site (TAM) or entity, is predominantly an immune cell or entity or macrophage or TAM with either no polarization, preferentially M0, or preferentially a polarization i (Mi) preferentially when:
an assembly of immune cells or entity or macrophages or macrophages associated with a tumor or pathological site (TAM) comprise more than 0, 1, 5, 10, 50, 75, 80 or 90% of cell(s) with no polarization, preferentially M0, or preferentially a polarization i (Mi),
and/or
the at least one immune cell or entity or macrophage or macrophage associated with a tumor or pathological site (TAM) occupies or comprises more than 0, 1, 5, 10, 50, 75, 80 or 90% of the time during which it is observed or active or moving or producing at least one immune entity or interacting with at least one nanoparticle and/or radiation, no polarization, preferentially M0, or preferentially a polarization i (Mi).

In another embodiment of the invention, the at least one immune cell or entity or macrophage or macrophage associated with a tumor or pathological site (TAM), is predominantly a immune cell or macrophage or TAM or entity with a predominant polarization j (Mj), when:
preferentially an assembly of immune cells or entity or macrophages or macrophages associated with a tumor or pathological site (TAM) comprise more than 0, 1, 5, 10, 50, 75, 80 or 90% of cell(s) with a polarization j (Mj),
preferentially the at least one immune cell or entity or macrophage or macrophage associated with a tumor or pathological site (TAM) occupies or comprises more than 0, 1, 5, 10, 50, 75, 80 or 90% of the time during which it is observed or active or moving or producing at least one immune entity or interacting with at least one nanoparticle and/or radiation, a polarization j (Mj).

In one embodiment of the invention, the nanoparticle comprises at least one compound or an assembly of compound(s) that can dissociate, preferentially following the application of radiation.

In one embodiment, the nanoparticle is produced in the body part, preferentially by at least one organelle or vesicle or endosome or exosome or lysosome, which preferentially crystallizes free or dissolved or decomposed metal(s), preferentially iron and/or gold, preferentially originating from at least one nanoparticles or magnetosomes, preferentially administered in the body part, preferring being or comprising an aurosome or ferrosome or metalosome or a nanoparticle with at least one property in common with an aurosome or ferrosome or metalosome, where a metalosome has at least one property in common with an aurosome or ferrosome, but is formed by involving at least one other metal than gold or iron.

In some cases, the nanoparticle, preferentially the aurosome or ferrosome or metalosome, or salt is composed of or comprises at least 1 or 2 metal(s) or metal atom(s), preferentially bound to each other, of the same type in some cases, of different cases in some other cases, preferentially crystallized, preferentially magnetic and/or plasmonic, which are surrounded by part of or the entire of an organelle, preferentially of or originating from an eukaryotic cell or body part, preferentially belonging to or originating from the pathological site, preferentially a vesicle or endosome or exosome or lysosome. In some cases, the salt is a particle or nanoparticle or assembly of at least 1, 2, 5 or 10 ion(s), atom(s), molecule(s), preferentially forming a particle or nanoparticle, preferentially an assembly as preassembly defined that has more empty space or does not comprise so well-ordered atom(s) or ion(s) or has a less positive or less negative charge or surface charge than (in) a nanoparticle, preferentially a crystallized and/or charged nanoparticle.

In some other cases, the salt comprises cations and/or anions preferentially forming a neutral product or particle or nanoparticle, wherein these ions can be mineral, for example chloride Cl-, organic, for example acetate CH3-COO-, monoatomic, for example fluoride F-, and/or polyatomic, for example sulphate SO42-.

In some cases, the salt can have at least one property in common with the nanoparticle.

In some other cases, salt can have at least one property that differs from a property of the nanoparticle, for example an amorphous composition, preferentially predominantly.

In one embodiment of the invention, the immune cell or entity, macrophage, TAM and/or one of its entity or compounds or organelle, comprises at least one state or state of polarization that can preferentially be changed or controlled and/or is polarizable.

The invention also relates to the method according to the invention, wherein at least one or two of its step, preferentially at least the step of differentiation of a monocyte into a TAM and/or at least one step of TAM polarization from an unpolarized state M0 into a polarized state M1 or M2, comprise(s) at least one lapse of time and/or one location, during or in which par of or all of the at least one or two step(s) occur simultaneously or concomitantly in time and/or space,
wherein the location is preferentially at least one region comprised in or comprised outside or being the body part to be treated, tumor, or pathological or healthy site,
wherein the lapse of time is preferentially a duration of more than 0, 10⁻²⁰, 10⁻⁵, 1, 5, 10 or 10³ second(s). In some other cases, the lapse of time can be a duration of less than 10²⁰, 10⁵, 1, 5, 0, 10⁻¹ or 10⁻³ second(s).

The invention also relates to the method nanoparticle(s) and/or cell(s), wherein preferentially the differentiation of a monocyte into a TAM and/or the TAM polarization from an unpolarized state M0 into a polarized state M1 or M2, occurs during at least one lapse of time and/or one location, preferentially simultaneously or concomitantly in time and/or space.

The invention also relates to method according to the invention, wherein at least one or two of its step, preferentially at least the step of differentiation of a monocyte into a TAM and at least one step of TAM polarization from an unpolarized state M0 into a polarized state M1 or M2, comprise at least one lapse of time and/or one location, during or in which par of or all of the at least two steps occur non-simultaneously or non-concomitantly in time and/or space,
wherein the location is preferentially at least one region comprised in or comprised outside or being the body part to be treated, tumor, or pathological site,
wherein the lapse of time is preferentially a duration of more than 0, 10⁻²⁰, 10⁻⁵, 1, 5, 10 or 10³ second(s). The invention also relates to the method nanoparticle(s) and/or cell(s), wherein preferentially the differentiation of a monocyte into a TAM and/or the TAM polarization from an unpolarized state M0 into a polarized state M1 or M2, occur during at least one different lapse of time and/or one location, during or non-simultaneously or non-concomitantly in time and/or space.

The invention also relates to the method and/or cell and/or entity and/or nanoparticle according to the invention, wherein M2 and/or cell and/or nanoparticle and/or entity, preferentially immune entity, has at least one property selected in the group consisting of:
i) It is anti-inflammatory;
ii) It is or is associated with or comprises partly or fully tumor-promoting, pathogen-promoting, angiogenesis-promoting, neovascularization-promoting, stroma-activating, tumor or pathological or tumor site or environment or microenvironment remodeling, growth or protection, or increasing or activating or triggering pro-tumor or pro-pathological site inflammatory reactions or immune entity(ies) of first category or decreasing or de-activating anti-tumor or anti-tumor-site inflammatory reaction(s) or immune entity(ies) of second category,
iii) it comprise(s) or generate(s) or is/are associated with or activate(s) or is activated by or is polarized by or originate(s) from:
   - at least one immune entity of first category;
   - a larger number or size or concentration or different types of immune entity(ies) of first category than for M1 or in the presence than in the absence of at least one nanoparticle, preferentially exposed to at least one radiation, preferentially by a factor of at least 0, 0.1, 1, 2 or 5;
   - a larger number or size or concentration or different immune entity(ies) of first than second category, preferentially by a factor of at least 0, 0.1, 1, 2 or 5;
   - a larger number or concentration of immune entity(ies) of first category than 10²⁰, 10⁵, 10³, 100, 50, 20, 10, 5, 2 or 1, preferentially per cell preferentially and/or per nanoparticle, preferentially per nm³, µm³, mm³, cm³ or m³ of TAM, nanoparticle(s), and/or body part;
   - a size of immune entity(ies) of first category that is larger than 10²⁰, 10⁵, 10³, 100, 50, 20, 10, 5, 2 or 1 nm,
   - a number of different types of immune entity(s) of first category that is larger than 10²⁰, 10⁵, 10³, 100, 50, 20, 10, 5, 2 or 1,
   - at least one immune entity of first category that changes the polarization at least one TAM from an un-polarized state to M2 or from M1 to M2, preferentially in the presence of at least one nanoparticle, preferentially exposed to at least one radiation,
wherein the entity, preferentially immune entity, most preferentially immune entity of first category is preferentially selected among: at least one stimulation factor, preferentially LPS, ICs, GCs, LIF, adenosine, TGF-β, IFN-γ, IL-4, IL-10, IL-13, IL-33, Th cytokine, M-CSF, and/or helminth, at least one cytokine, preferentially Low IL-12, IL-23, high IL-1RA, IL-10, and/or IL-4/IL-13, at least one marker, preferentially CD86, CD163, MHC II, SR, MMR/CD206, CD200R, TLR1, TLR8, VEGF, MMP9, TGM2, DecoyR, IL-1R, IIMR, Fizz-1, and/or Ym1, at least one chemokine, preferentially CCL1, CCL2, CCL5, CxCL10, CCL13, CCL14, CCL17, CCL18,CCL22, CCL23, CCL24, CCL26, CXCL16, CCR2, CCR3, and/or CCR4, at least one Reprogramming factor, preferentially Reprogramming from M1 to M2; hyaluronic acid-poly (ethyleneimine), plasmid DNA NPs (HA-PEI/pDNA), miR-223-expressing plasmid DNA-encapsulated, HAPEI NPs (HA-PEI/miR-223 NPs), alginate NPs, Au, and/or Ti, at least one TAM induced product, preferentially STAT3, STAT6, IRF4, p50, iron release, folate uptake and metabolism, and/or arginase 1 to polyamine, at least one immune entity inducing M2 TAM polarization, at least one interleukin such as IL-4, IL-10, IL-13, at least one receptor of an interleukin such as IL-4Rα, IL10-R1, IL10-R2, IL1-R2, at least one regulator factor such as IRF4, at least one Janus or tyrosine kinase such as JAK1 or JAK3, at least one protein of Signal Transducer(s) and Activator(s) of Transcription such as STAT6, at least one peroxisome or one of its component such as peroxisome proliferator-activated receptor γ (PPARy), at least one protein such as arginase 1 (Arg 1), Ym1, at least one enzyme such as Chitinase 3-like 3, at least one non-enzymatic protein such as Chi313, at least one cysteine rich protein such as resistin-like-α, at least one ligand of chemokine such as CCL17, at least one cluster of differentiation such as CD206, at least one TAM receptor or TAM mannose receptor such as Mrc1, at least one fatty acid or fatty acid receptor, at least one transcription factor such as that of STAT3, at least one suppressor of cytokine signaling such as SOCS3, at least one hormone such as glucocorticoid or glucocorticoid hormone, at least one lipophilic compound, at least one receptor such as a glucocorticoid receptor (GR), at least one transcription factors, such as nuclear factor-kappa B NF-kB or activator protein such as AP-1, and the whole or part of a biological pathway or immune response, iv) it has at least one parameter (P) or property preferentially selected in the group consisting of: i) activity, ii) concentration, iii) morphology, iv) size, v) stiffness, vi) strength, vii) movement, viii) existence, xi) oxygen or oxygen concentration or metallic or nanoparticle or salt concentration, which is preferentially changed or controlled or altered or remodeled or altered, preferentially by or following the method and/or at least one step of the method according to the invention.

The invention also relates to a method to control or change or alter or remodel at least one parameter (P) of the cell and/or entity and/or nanoparticle

The invention also relates to the method and/or cell and/or nanoparticle according to the invention, wherein the cell and/or entity and/or M1 and/or nanoparticle has at least one property selected in the group consisting of:
i) it is pro-inflammatory;
ii) it is or is associated with or comprises partly or fully pathogen or tumor resistant preferentially immune entity(ies), anti-angiogenic, acting against the development of the vasculature or neovasculature of a tumor or pathological site, anti-pathogenic or anti-tumoral or increasing or activating or triggering anti-tumor or anti-pathological site immune reaction, or deactivating the tumor or pathological site or its environment or micro-environment, or decreasing or de-activating pro-pathological site inflammatory reactions or immune entity(ies) of second category,
iii) it comprises or generates or is associated with or activate(s) or is activated by or is polarized by or originate(s) from:
   - at least one entity, preferentially an immune entity, most preferentially an immune entity of second category;
   - a larger number or size or concentration or different types of immune entity(ies) of second category than for M2 or in the presence than in the absence of at least one nanoparticle, preferentially exposed to at least one radiation, preferentially by factor of at least 0, 0.1, 1, 2 or 5;
   - a larger number or size or concentration or different types of immune entity(ies) of second category than for M2, preferentially by factor of at least 0, 0.1, 1, 2 or 5;
   - a larger number or concentration of immune entity(ies) of second category than 10²⁰, 10⁵, 10³, 100, 50, 20, 10, 5, 2 or 1, preferentially per cell preferentially of first and/or second type(s) and/or per nanoparticle, preferentially per nm³, µm³, mm³, cm³ or m³ of body part;
   - a size of immune entity(ies) of second category that is larger than 10²⁰, 10⁵, 10³, 100, 50, 20, 10, 5, 2 or 1 nm,
   - a number of different types of immune entity(s) of second category that is larger than 10²⁰, 10⁵, 10³, 100, 50, 20, 10, 5, 2 or 1,
   - at least one entity, preferentially an immune entity, most preferentially an immune entity of second category that changes the polarization at least one TAM from an un-polarized state to M1 or from M2 to M1, preferentially in the presence of at least one nanoparticle, preferentially exposed to at least one radiation,
wherein the entity, preferentially immune entity, most preferentially immune entity of second category is preferentially selected in the group consisting of: at least one stimulation factor, preferentially LPS, IFN-γ, GM-CSF, and/or TNFα, at least one cytokine, preferentially High TNFα, IL-1β, IL-6, IL-12, IL-18, IL-23, at least one marker, preferentially CD86, CD80, CD68, MHC II, IL-1R, TLR2, TLR4, iNOS, and/or SOCS3, at least one chemokine, preferentially CXCL1, CXCL3, CXCL5, CXCL8, CXCL9, CXCL10, CXCL11, CXCL13, CXCL16 CX3CL1, CXCR3; CCL2, CCL3, CCL4, CCL5, CCL8, CCL11, CCL15, CCL19, CCL20; NOS2, CD64, IDO, and/or SOCS1, at least one reprogramming factor, preferentially Reprogramming from M2 to M1; Superparamagnetic iron oxide nanoparticle (SPIONs), Glycocalyx-mimicking NPs, (glycol-NPs), Ag, Au, Co, ZnO, TiO2, and/or SiO2, at least one TAM induced product, preferentially STAT1, STAT2, IRF5, p65, iron uptake, and metabolism; high ROS, ROIs, NO, and MHC I, and/or low IL-10, at least one immune entity inducing M1 TAM polarization, at least one interferon such as IFN-γ, IFN-α or IFN-β or at least one of its receptor, at least one Janus kinase (Jak) adapters, at least one protein of signal transducer and transcription activator (STAT1), IFN-α, at least one Interferon-regulatory facto IRF, at least one nuclear factor such as NF-kB, at least one Nitric oxide synthase such as iNOS or NOS2, at least one interleukin such as IL-1β or IL-12, at least one major histocompatibility complex such as (MHC) II, at least one suppressor of cytokine signaling (SOCS), at least one LPS or derivative, at least one Pattern recognition receptor (PRR) such as a Toll-Like receptor TLR or TLR4, at least one entity triggering a differentiation, at least one interferon-responsive factor such as IRF3, at least one pathway such as nuclear factor kappa-B (NF-kB) pathway p65 or p50, at least one transcription factor, at least one gene that makes a protein involved in a signaling pathway with a immune cell such as MyD88, at least one protein activator such as AP-1, at least one protein kinase such as Mitogen-activated protein kinase MAPK, at least one cytokine such as a tumor necrosis factor (TNF), at least one chemokine such as CXCL10, CXCL11, at least one co-stimulating protein, at least one protein that processes or produces antigen(s), and the whole or part of a biological pathway or immune response.

In some other cases, the cell and/or M1 and/or nanoparticle has at least one property selected in the group consisting of:
- a smaller number or size or concentration or different types of entities or immune entity(ies) of second category than for M2 or in the presence than in the absence of at least one nanoparticle, preferentially exposed to at least one radiation, preferentially by factor of at least 0, 0.1, 1, 2 or 5,
- a smaller number or size or concentration or different types of entities or immune entity(ies) of second category than for M2, preferentially by factor of at least 0, 0.1, 1, 2 or 5,
- a smaller number or concentration of entities or immune entity(ies) of second category than 10²⁰, 10⁵, 10³, 100, 50, 20, 10, 5, 2 or 1, preferentially per cell preferentially of first and/or second type(s) and/or per nanoparticle, preferentially per nm³, µm³, mm³, cm³ or m³ ₒf body part,
- a size of entities or immune entity(ies) of second category that is smaller than 10²⁰, 10⁵, 10³, 100, 50, 20, 10, 5, 2 or 1 nm, and
- a number of different types of entities or immune entity(s) of second category that is smaller than 1020, 105, 103, 100, 50, 20, 10, 5, 2 or 1.

The invention also relates to the method according to the invention, which is reversible, wherein preferentially at least one TAM first changes its polarization from being predominantly a TAM with either no polarization (M0) and/or a polarization i (Mi) before the method, to a predominant j polarization (Mj) after one step of the method, and second changes back its polarization from being predominantly polarized into Mj before at least one step of the method to being predominantly with no polarization (M0) and/or with a polarization i (Mi) after one step of the method.

The invention also relates to the method and/or radiation according to the invention, wherein the radiation is not a laser or magnetic field or an electromagnetic wave.

The invention also relates to the method and/or radiation according to the invention, wherein the radiation is selected in the group consisting of: i) wave(s) such as electromagnetic, sound, acoustic or particle wave(s), ii) electromagnetic radiation, iii) acoustic radiation forces, iv) radiation forces, v) radiation pressures, vi) irradiation, vii) a magnetic or electric field, viii) light not produced by a laser, ix) light produced by a lamp, x) light emitted at a single wavelength, xi) light emitted at multiple wavelengths, xii) a ionizing radiation, xiii) microwave, xiv) radiofrequencies, xv) alpha particles or radiation, xvi) beta particles or radiation, xvii) gamma particles or radiation, xviii) X-ray particles or radiation, xix) neutron particles or radiation, xx) proton particles or radiation, xxi) electron particles or radiation, xxii) ion particles or radiation, xiii) neutrino particles or radiation, xiv) muon particles or radiation, xv) meson particles or radiation, xvi) photon particles or radiation, xvii) infra-sounds, xviii) sounds, xix) ultra-sounds, xx) hyper sounds. xxi) heating radiation, xxii) a cooling radiation, xxiii) a ionizing radiation, xxii) a light not being a laser, xxiii) an alternating magnetic field, xxiv) a uniform radiation, and xxv) radiation with a gradient or variation, preferentially spatial and/or temporal.

The invention also relates to the nanoparticle and/or cell or entity and/or method according to the invention, wherein part or the entire of the at least one or two nanoparticle(s) and/or cell(s) has or have at least one property selected in the group consisting of:
1) Part or the entire of the at least one nanoparticle and/or cell or entity comprises a metallic, crystalline, plasmonic, super-paramagnetic, ferromagnetic and/or ferrimagnetic composition or property,
2) Part or the entire of the at least two nanoparticles and/or cell(s) or entity form an assembly of at least two nanoparticles and/or cell(s) within a geometric figure such as a chain, circle, rectangle, or square,
3) Part or the entire of the at least one nanoparticle and/or cell or entity does or do not comprise at least one photosensitizer or ROS or radical species generator or radiation-generator, radiation-amplifier, or photo-generator, preferentially in its core and/or coating;
4) Part or the entire of the at least one nanoparticle and/or cell or entity differentiates or polarizes at least one cell or TAM, preferentially from an un-differentiated and/or un-polarized state(s) to a differentiated and/or polarized state(s);
5) Part or the entire of the at last one nanoparticle and/or cell(s) or entity comprises at least one compound such as at least one or two metal(s) and/or part or the entire of an organelle or cellular vesicle or endosome or exosome or lysosome or cellular compartment, preferentially intracellular, most preferentially inside or originating from an eukaryotic cell, preferentially belonging to or originating from a pathological cell or site, which preferentially differentiates and/or polarizes at least one cell or TAM or entity.

In one embodiment, the nanoparticle comprises or originates from or is composed of at least one metallic salt or at least one salt comprising at least one metal.

In some cases, the at least one nanoparticle or its assembly can comprise or be made of platelet and/or chain.

In some other cases, the nanoparticle can comprise or be made of or be associated with or in interaction or in contact with, preferentially directly or indirectly, at least one vesicle or organelle or cell component or immune entity, preferentially extracellular or intracellular.

In still some other cases, the at least one nanoparticle or its assembly can comprise or be made or be associated or in interaction or in contact, preferentially directly or indirectly, with at least one vesicle. In still some other cases, the at least one nanoparticle, vesicle, organelle, cell component, entity and/or immune entity, can be or be comprised in, originate from, be produced by, by expelled by, be internalized in, activated, deactivated, and/or diffuse from the intracellular and/or extracellular matrix, plasma, liquid, gas, solid, blood, tissue, organ, body part, partly or fully.

In sill some other cases, the at least one nanoparticle or its assembly can comprise or be made or be associated or in interaction or in contact, preferentially directly or indirectly, with at least one cellular or organelle or membrane or vesicle or body part fragment.

In still some other cases, the at least one nanoparticle, vesicle, organelle, cell component and/or immune entity, can be or be comprised in, originate from, be produced by, by expelled by, be internalized in, and/or diffuse from at least one virus or virus particle or metallic salt.

The invention also relates to the method and/or at least one cell according to the invention, wherein the at least one cell or TAM or entity has at least one of its polarization states that is changed by varying, increasing or decreasing at least one property or parameter selected in the group consisting of :
- the radiation, preferentially applied on the nanoparticle and/or cell(s), preferentially selected in the group consisting of: i) the type or number of radiation i) the intensity or power or power density of radiation, ii) the frequency or wavelength of radiation, iii) the penetration depth of radiation, and iv) the duration of application of the radiation, and v) the number of times that the radiation is applied;
- the nanoparticle, preferentially assembled with the cell(s), preferentially selected in the group consisting of: the type, number, concentration, charge, surface charge, composition, type of assembly, coating or core type or composition, magnetic property, metallic and/or carbonaceous composition, a-pyrogenicity, sterility, stability, type of interaction or binding with the at least one cell, differentiable and/or polarizable cell, location of the at least one nanoparticle, preferentially inside or outside the least one cell or cell component;
- a physico-chemical disturbance, preferentially applied on the nanoparticle(s) and/or TAM or entity, preferentially during at least one step of the method, preferentially selected in the group consisting of: a variation of the environment, pH, preferentially by more than 10⁻⁵, 0.1, 0, 1 or 5 pH unit(s), temperature, preferentially by more than 10⁻⁵, 0.1, 0, 1 or 5 °C, of composition, preferentially by more than 10⁻⁵, 0.1, 0, 1 or 5 atom(s), and/or potential redox, preferentially by more than 10-5, 0.1, 0, 1 or 5 mV or V, of the nanoparticle(s) and/or cell or TAM or of its/their environment;

wherein preferentially the variation of the environment of the nanoparticle(s), entity and/or cell or TAM is a variation of the composition, number of atoms, pH, temperature of at least one region or volume comprising the nanoparticle(s) and/or TAM, preferentially smaller than the individual or body part of the individual or diameter or thickness of body part of the individual or than 1, 0.1 or 0.001 meter.
wherein preferentially the radiation or physico-chemical disturbance is preferentially sufficiently intense or induces a sufficiently large amount of heat, cold or radical species or has at least one of its parameter(s) with a sufficiently large value to trigger the change or at least one perturbation that changes at least one state of polarization of at least one TAM;
wherein preferentially in some cases the type, intensity, power, power density, frequency, wavelength, penetration depth, duration of application, and/or number of application times of the radiation is/are preferentially larger than 10⁻²⁰, 10⁻¹⁰, 10⁻⁵, 10⁻¹, 0, 1, 2 or 5 preferentially Watt, Hz, KHz, MHz, nm, second, hour, day, preferentially per cell and/or nanoparticle and or body part, preferentially per cm, cm2 or cm3 of cell, nanoparticle and/or body part,
wherein preferentially the radiation or physico-chemical disturbance is preferentially sufficiently mild or produces a sufficiently moderate amount of heat, cold or radical species or has at least one of its parameter(s) with a sufficiently low value to avoid or prevent the destruction or inactivation of at least one entity or TAM,
wherein preferentially in some other cases, the type, intensity, power, power density, frequency, wavelength, penetration depth, duration of application, and/or number of application times of the radiation is/are smaller than 10²⁰, 1010, 105, 100, 10, 5, 2, 1 or 0, preferentially Watt, Hz, KHz, MHz, nm, second, hour, day, preferentially per cell and/or nanoparticle and or body part, preferentially per cm, cm² or cm³ of cell, nanoparticle and/or body part.

In some other cases, the variation of the cell and/or nanoparticle and/or entity and/or its/their environment and/or pH and/or temperature and/or composition and/or redox potential is/are smaller than 10⁻⁵, 0.1, 0, 1 or 5 °C, and/or less than 10⁻⁵, 0.1, 0, 1 or 5 pH unit(s), and/or less than 10⁻⁵, 0.1, 0, 1 or 5 atom(s), and/or less than 10⁻⁵, 0.1, 0, 1 or 5 mV or V, preferentially of the nanoparticle(s) and/or entity and/or cell or TAM or of its/their environment.

The invention also relates to the method according to the invention, wherein at least one step of the method has at least one property selected in the group consisting of:
i) it is repeated more than once,
ii) it occurs in any order or in at least one order, preferentially step 2, 3, 4, 5, or 6 after step 1, step 1, 3, 4, 5, or 6 after step 2, step 1, 2, 4, 5, or 6 after step 3, step 1, 2, 3, 5, or 6 after step 4, step 1, 2, 3, 4, or 6 after step 5, and/or step 1, 2, 3, 4, or 5 after step 6,
iii) it lasts more than 0, 10⁻²⁰, 10⁻⁵, 1, 5, 10 or 10³ second(s).
iv) it occurs in at least one region comprised in or comprised outside or being the body part to be treated, tumor, and/or pathological site.

The invention also relates to at least one entity, preferentially entity of first type, or cell, preferentially cell of first type, or TAM polarized in a M1 TAM, as preferentially obtained by at least one step of the method according to the invention, to preferentially destroy or inactivate a pathological site such as a tumor or bacterial site or stimulate at least one immune response or immune entity preferentially of first category, or to defend a host or individual or healthy site preferentially surrounding a healthy site. The invention also relates to at least one entity, preferentially entity of second type, or cell, preferentially cell of second type, or TAM polarized in a M2 TAM, as preferentially obtained by at least one step of the method according to the invention, to heal or protect a wound or injury or body part or stimulate at least one hypersensitive or helminths response or immune entity preferentially of second category or to favor or trigger angiogenesis, scavenging, matrix/and/or tissue remodeling, and/or immune suppression. The invention also relates to a mixture or assembly of entities or cells or TAM preferentially comprising a majority of entities or cells of first type or M1 TAM, and preferentially a minority of entities or cells of second type or M2 and/or un-polarized TAM, as preferentially obtained by at least one step of the method according to the invention.

In some cases, at least one step of the method or the method lasts less than 10²⁰, 10¹⁰, 10⁵, 10³, 100, 50, 20, 10, 5, 2 1, 0, 10⁻¹, 10⁻⁵ or 10⁻²⁰ second(s).

The invention also relates to a therapeutic or diagnostic compound, a drug or drug component, a medical device or medical device component, a preparation or composition or adjuvant or active principle or vaccine component or vaccine adjuvant, preferentially comprising at least one entity or cell or first type or M1 or a mixture of TAM comprising a majority of entities or cells of first type or M1 TAM, and preferentially a minority of entities or cells of second type or M2 and/or un-polarized TAM, as preferentially obtained by at least one step of the method according to the invention.

In some cases, a minority of a compound or entity is or represents less than 100, 75, 50, 20, 10, 5, 2 or 1% preferentially in mass or volume or number of this compound or entity, preferentially within an assembly of different compounds or entities preferentially in types, sizes, compositions, or at least one property, where this compound or entity can be the nanoparticle and/or cell or at least one entity activating, polarizing, differentiating, activated by, dissociated from, produced by, or originating from the cell or nanoparticle.

In some cases, a majority of a compound or entity is or represents more than 0, 1, 10, 25, 50, 75, 90, 99 or 100% preferentially in mass or volume or number of this compound or entity, preferentially within an assembly of different compounds or entities preferentially in types, sizes, compositions, or at least one property, where this compound or entity can be the nanoparticle and/or cell or at least one entity activating, polarizing, differentiating, activated by, dissociated from, produced by, or originating from the cell or nanoparticle.

The invention also relates to a mixture or assembly of entity(ies), cell(s) or TAM preferentially comprising a majority of entities or cells of second type or M2 TAM, and preferentially a minority of entities or cells of first type or M1 and/or un-polarized TAM, as preferentially obtained by at least one step of the method according to the invention.

The invention also relates to a therapeutic or diagnostic compound, a drug or drug component, a medical device or medical device component, a preparation or composition or adjuvant or active principle or vaccine component or vaccine adjuvant, preferentially comprising at least one entities or cells of second type or M2 or a mixture of TAM comprising a majority of entities or cells of second type or M2 TAM, and preferentially a minority of M1 and/or un-polarized TAM, as preferentially obtained by at least one step of the method according to the invention.

In one embodiment of the invention, the at least one entity or cell, preferentially polarizable cell, comprises at least one un-polarized state & such as M0, and/or at least two polarized states α and β such as M1 and M2.

In another embodiment of the invention, the un-polarized state &, preferentially M0, is a state in which an un-polarizable cell or entity is generating or associated with or comprises or activates no or less anti-inflammatory or anti-tumor anti-pathological site or anti-angiogenesis or anti-neovasculature of inflammatory or pro-tumor or pro-angiogenesis or pro-neovasculature or immune entity, preferentially of first and/or second type(s), preferentially polarization or response or gene or cytokine or receptor, preferentially cell or cell receptor, preferentially cell or cell component or organelle, preferentially compared with polarization state(s), preferentially α and/or β;

In one embodiment of the invention, the un-polarized state, preferentially &, is the un-polarized state M0 of a macrophage or TAM.

In some case, anti-tumor and/or anti-pathological site and/or anti-angiogenesis designates at least one action or entity or immune entity that deactivates or destroys or decreases the size of at least one tumor or pathological site or angiogenesis preferentially blood vessel, partly or fully.

In another embodiment of the invention, the polarized state, preferentially α, preferentially M2, is preferentially a state in which a cell, preferentially polarizable cell, or entity, preferentially polarizable entity, is generating or associated with or comprises or activates an anti-inflammatory anti-tumor, anti-pathological site, or anti-neo-vasculature or anti-angiogenesis polarization or response or gene, cytokine, pathway, receptor or cell receptor, cell or cell component or organelle, preferentially IL-10. In another embodiment of the invention, the polarized state, preferentially α, is preferentially the polarized state M2 of a macrophage or TAM.

In another embodiment of the invention, the polarized state, preferentially β, is a state in which the cell, preferentially the polarizable cell, the entity, preferentially the polarizable entity, is generating or associated with or comprises or activates a pro-inflammatory or pro-tumor or pro-pathological site or pro-neo-vasculature or pro-angiogenesis polarization or response or gene, or cytokine, or pathway, or receptor or cell receptor, or cell or cell component or organelle, preferentially IRF, preferentially NF-kB, preferentially iNOS, and/or preferentially IL-1β.

In an embodiment of the invention, the polarized state, preferentially β, is preferentially the polarized state M1 of a macrophage or TAM.

In some case, pro-tumor and/or pro-pathological site and/or pro-angiogenesis designates at least one action or entity or immune entity that activates or increases the size of or heals or protects or repairs at least one tumor or pathological or healthy site or angiogenesis preferentially blood vessel, partly or fully. In one embodiment of the invention, the method comprises at least one step comprising or consisting in providing or selecting at least one or one or one type of nanoparticle(s).

In one embodiment of the invention the at least one or one type of nanoparticle, preferentially type 1, has a metallic composition preferentially predominantly comprising one metallic element or at least more than 10, 25, 50, 75, 90 or 99% in mass or volume of one metallic element, where the at least one metallic element is preferentially selected among one metal, most preferentially iron or gold.

In one embodiment of the invention the at least one or one type of nanoparticle has a bi-metallic or multi-metallic composition preferentially predominantly comprising at least two metallic elements, preferentially comprising at least 10, 25, 50, 75, 90 or 99% in mass or volume of two metallic elements, where the at least two metallic elements are preferentially selected among two metals, most preferentially iron and gold.

In some cases, the metallic, bimetallic and/or multi-metallic composition(s) can be preferentially predominantly that of the core of the nanoparticle and/or not that of the coating of the nanoparticle.

In one embodiment of the invention, the method or assembly of at least one nanoparticle and/or TAM and/or cell or polarizable cell or entity or polarizable entity, according to the invention, comprises at least one step preferentially of formation or one property preferentially associated with its formation consisting in or comprising: associating or association, administering or administration, moving or movement, diffusing or diffusion, internalizing or internalization, bringing or polling together, externalizing or externalization, interacting or interaction or letting or making associate, administer, bring together, externalize, interact least one nanoparticle with, together, of, in, from, and/or through at least one TAM or cell or polarizable cell or entity or polarizable entity.

In some cases, the entity can derive from or originate from or be produced by or be comprised in or be or be part of the cell or TAM.

In another embodiment of the invention, at least one step of the method results in an assembly comprising at least one cell or polarizable cell or entity or polarizable entity or TAM and at least one nanoparticle.

In another embodiment of the invention, at least one step of the method results or comprises or consists in in a change of polarization preferentially of a cell or polarizable cell or entity or polarizable entity, preferentially from an un-polarized state to a polarized state, preferentially a change from state & to state(s) α and/or β, preferentially a change from state M0 to state(s) M1 and/or M2, preferentially predominantly.

In another embodiment of the invention, at least one step of the method results or comprises or consists in a change of polarization, preferentially of a cell or polarizable cell or entity or polarizable entity, preferentially from state α to state β or from state β to state α, preferentially from state M1 to state M2 or from state M2 to state M1, preferentially predominantly.

In one embodiment of the invention, at least one step of the method consists in or comprises exposing the assembly comprising both of or either of at least one nanoparticle and at least one polarizable TAM or cell or polarizable cell or entity or polarizable entity to at least one radiation or source of radiation.

In another embodiment of the invention, the assembly comprising or both of or either of at least one nanoparticle and at least one polarizable TAM or cell or polarizable cell or entity or polarizable entity is/are exposed to at least one radiation or source of radiation or at least one radiation or source of radiation is applied on the assembly comprising or both of or either of at least one nanoparticle and at least one polarizable TAM or entity or polarizable entity.

In one embodiment of the invention, the polarization of a cell or polarizable cell or entity or polarizable entity, preferentially into M1, activates or controls or changes or alters or redistribute at least one polarizable cell or entity or at least one of its parameter(s), preferentially against a pathological site or to destroy a pathological site, partly or fully.

In one embodiment of the invention, the polarization of a cell or polarizable cell or entity or polarizable entity, preferentially into M2, activates or controls or changes or alters or redistributes at least one polarizable cell or entity or at least one of its parameter(s), preferentially to defend or protect or reconstruct a healthy site, partly or fully.

In one embodiment, the at least one or two metal(s), preferentially comprised in the nanoparticle and/or cell and/or entity, preferentially being associated with the metallic composition or property of the nanoparticle and/or cell and/or entity, is/are selected in the group of metal: in the list of metals: Actinium, Aluminium, Americium, Barium, Berkelium , Beryllium, Bismuth, Bohrium, Cadmium, Calcium, Californium, Cerium, Cesium, Chromium, Cobalt, Copper, Curium, Darmstadtium, Dubnium, Dysprosium, Einsteinium , Erbium, Europium, Fermium, Francium, Gadolinium, Gallium, Gold, Hafnium, Hassium, Holmium, Indium, Iridium, Iron, Lanthanum, Lawrencium, Lead, Lithium, Lutetium, Magnesium, Manganese, Meitnerium, Mendelevium, Mercury, Molybdenum, Neodymium, Neptunium, Nickel, Niobium, Nobelium, Osmium, Palladium, Platinum, Plutonium, Polonium, Potassium , Praseodymium, Promethium, Protactinium , Radium , Rhenium, Rhodium, Roentgenium, Rubidium , Ruthenium, Rutherfordium, Samarium , Scandium, Seaborgium , Silver, Sodium, Strontium, Tantalum, Technetium, Terbium, Thallium, Thorium, Thulium, Tin, Titanium, Tungsten, Ununbium, Ununhexium, Ununpentium, Ununquadium, Ununtrium, Uranium, Vanadium, Ytterbium, Yttrium, Zinc, Zirconium, at least one Alkali Metal, at least one Alkaline Earth Metal, at least one Transition Metal, at least one Rare Earth Element, at least one alloy, at least one mixture of at least two metals, at least one single metal, Gunmetal, Steel, Bronze, and Brass.

In some cases, the change of at least one state or state of polarization of a change from an undifferentiated to a differentiated state is triggered by activation, preferentially photo, electromagnetic, magnetic, radiation activation, preferentially of the nanoparticle and/or cell and/or entity, preferentially polarizable cell or entity and/or TAM.

Preferentially, the at least one polarization state, preferentially the at least one first polarization state, of a cell or entity, preferentially a polarizable cell or entity, is a state of a cell or element, or organelle, or compartment or entity comprised in this cell, or body part or part of a body part, preferably comprising at least one of the cell, where this state has at least one property selected in the group consisting of: i) a motion, preferentially associated with a movement, physical movement, gene migration and/or attraction, of the cell, entity, and/or cell component in the direction of at least one nanoparticle or at least one body part or at least one other cell or entity that is preferentially unpolarized or in another state or state of polarization than the cell or entity, ii) the production or expression or activation or association or interaction with (of) entities or certain immune entities such as interleukins or cytokines or MHC-I or II or CD or helper cells, preferentially generating or being pro or anti-inflammatory or pro or anti tumoral or pro or anti pathogens or pathological cell or site, and iii) a physiological state that is characterized by a given cell or organelle or cell component size or activity or morphology or activation or inactivation.

Preferentially, the at least one second polarizable state of a cell or entity, preferentially a polarizable cell or entity, is a different state or state of polarization than the at least one first polarization state. This second state or state of polarization has preferentially at least one property selected in the group consisting of: : i) a different motion of the cell or entity than the motion of the first sate of polarization, characterized preferentially by a different, opposite, lower or larger direction, or speed of motion than for the first state or state of polarization, ii) the production or expression or activation or association or interaction of the cell or entity with different or less or more immune entities or immune entities having an opposite or different activity.

Preferentially, a cell or entity polarizable cell or entity is a cell or entity capable of occupying at least one state selected from the list consisting of: a non-polarized state of a cell or entity, a polarized state of a cell or entity, M0, M1 and M2.

Preferentially, an unpolarized state of a cell or entity or M2, preferentially of a cell or entity or polarizable cell, or entity is a state in which a cell or entity does not have or trigger, preferentially partly or predominantly or fully, a reaction or activity such as an immune reaction or activity, preferentially against A or inactivates A, where A is preferentially a pathological site or a pathological cell or entity or an environment of a pathological site or cell or entity or tumor micro-environment.

Preferentially, a un-polarized state of a cell or entity or M2, preferentially of a cell or entity or polarizable cell or entity, is a state in which a cell has or triggers, preferentially partly or predominantly or fully, a reaction or activity such as an immune reaction or activity, preferentially to protect B or favor the development or survival of B, where B is a healthy site or a healthy cell or entity or a healthy portion of a body part or a healthy full body part.

Preferentially, a first type of polarized state of a cell or entity or M1, preferentially of a cell or entity or polarizable cell, is a state in which a cell has or triggers, preferentially partly or predominantly or fully, a reaction or activity such as an immune reaction or activity, preferentially against A or inactivates A, where A is a body part or individual or part of an individual or body part a pathological site or a pathological cell or entity or an environment of a pathological site or tumor micro-environment.

Preferentially, a second type of polarized state of a cell or entity or M2, preferentially of a cell or polarizable cell or entity, is a state in which a cell has or triggers, preferentially partly or predominantly or fully, a reaction or activity such as an immune reaction or activity, preferentially to protect A, where A is a body part or individual or part of an individual or body part or a pathological site or a pathological cell or entity or an environment of a pathological site or cell or entity or tumor micro-environment.

Preferentially, a cell or entity, preferentially polarizable, is a cell or entity that can occupy at least one of the three states among the unpolarized state, the polarized state of first type and the polarized state of second state, and/or M0, M1 and/or M2.

Preferentially, an immune or polarizable cell or entity, most preferentially a macrophage or TAM, can occupy an un-polarized state, most preferentially M0, and/or at least a polarized state, preferentially a polarized state of first type, most preferentially M1, and/or a polarized state of second type, most preferentially M2.

The invention also relates to nanoparticle(s) and/or cell and/or entity and/or polarizable cell and/or polarizable entity and/or TAM preferentially for use in a treatment, preferentially medical or immune treatment or diagnosis or cosmetic treatment or treatment of a tumor or pathological site or infection or cancer, preferentially of a body part preferentially to be treated or of an individual or whole individual, In one embodiment of the invention, the nanoparticle(s) and/or cell and/or entity and/or polarizable cell(s) and/or polarizable entity(ies) and/or TAM(s) and/or the treatment or method or method step in which it/they is/are involved comprise:
Administering nanoparticles and/or cell or polarizable cell and/or entity and/or polarizable entity and/or TAM to a body part preferentially to be treated of an individual, preferentially either directly in some cases, i.e. preferentially in these cases at a distance from the body part that is smaller than 10²⁰, 10¹⁰, 10⁵, 10³, 10, 5, 2, 1 or 0 nm or m, or in some other cases indirectly, i.e. preferentially in these other cases at a distance from the body part that is larger than 10⁻²⁰, 10⁻¹⁰, 10⁻⁵, 10⁻³, 10⁻¹, 0, 1, 5, 10 or 10³ nm or m, Waiting or giving a lapse of time to the organism or body part so that the cell or entity, prereferntially unpolarizable, cell can switch from an unpolarized stat to a polarized state or from a first polarized state to a second polarized state;
   and/or
Applying a radiation, preferentially sequentially or several times, on the nanoparticles and/or cell and/or polarizable cell and/or entity and/or polarizable entity and/or TAM and/or body part and/or whole organism preferentially to change the polarization state of the cell or polarizable cell or entity or polarizable entity either from the first to second polarized state or inversely from the second to the first polarized state.

In some cases, at least one property of the nanoparticle and/or radiation and/or cell and/or entity can be adjusted to trigger at least one step of the method, wherein this property is preferentially: i) the nanoparticle or cell or entity concentration, composition, surface charge, crystallinity or amorphous state, organization, for example in chain, size, magnetic property, and/or ii) the radiation type, intensity, frequency, wavelength, penetration in the body part, duration of application, number of times that it is applied on the nanoparticle or body part or cell or entity.

In some cases, it can be equivalent to say that the radiation is applied on the nanoparticles or body part and/or polarizable or polarized cell or entity than to say that the nanoparticle or body part or polarizable or polarized cell or entity is exposed to radiation.

In one embodiment of the invention, the nanoparticle(s) or cell(s) or entity(ies) or radiation(s) or body part(s) or assembly or number or concentration of nanoparticle(s) or cell(s) or radiation(s) or body part(s) designate(s) more than 1, 10, 10², 10³, 10⁵, 10¹⁰ or 10⁵⁰ nanoparticle(s) or cell(s) or entity(ies) or radiation(s) or body part(s) or assembly of nanoparticle(s) or cell(s) or radiation(s) or entity(ies) or body part(s).

In another other embodiment of the invention, the nanoparticle(s) or cell(s) or radiation(s) or body part(s) or or entity(ies) or assembly or number or concentration of nanoparticle(s) or cell(s) or radiation(s) or body part(s) designate(s) less than 10¹⁰⁰, 10⁵⁰, 10²⁰, 10¹⁰, 10⁵, 10³, 10², 50, 10, 5, 2 or 1 nanoparticle(s) or cell(s) or radiation(s) or body part(s) or entity or assembly of nanoparticle(s) or cell(s) or radiation(s) or body part(s) or entity.

In one embodiment of the invention, the nanoparticle or cell or entity or radiation region is either: i) the portion or volume or length or surface of or in the body part comprising the nanoparticles or cells or entities, or receiving or absorbing the radiation, or ii) the portion or volume or length or surface outside the body part or cell or entity comprising the nanoparticles or cells or entities, or receiving or absorbing the radiation.

In an embodiment of the invention, the body part is or comprises more than or at least 1, 2, 5, 10, or 100 similar or different organism(s), apparatus, organ(s), tissue(s), cell(s), entity(ies), nanoparticle(s) or biomolecule(s).

In some cases, the body part can be all or part of the head, neck, shoulder, arm, leg, knee, foot, hand, ankle, elbow, trunk, inferior members, or superior members. In some other cases, the body part can be or belong to an organ, the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, or integumentary system, reproductive organ (internal or external), sensory organ, endocrine glands. The organ or body part can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or skin. The body part or organ can belong to the blood circulation or circulatory system.

In some cases, the body part can be or comprise at least one tumor, cancer, virus, bacterium, or pathological cell.

In one embodiment of the invention, the body part is or comprises partly or fully water, an excipient, a solution, a suspension, at least one chemical element, organic material, cell, entity, or gel, preferentially in part or in full, which can be synthetic or produced by a living organism.

In some cases, the body part can be the body part of a living organism.

In some other cases, the body part can be the part of a non-living organism or material or container preferentially of nanoparticle(s) and/or cell or polarizable cell(s).

Preferably, the body part of an individual, also designated as the body part, represents or is part of an individual or a whole individual, where the individual is preferentially a human, an animal, or an organism, preferentially a living or inactivated or dead organism, comprising at least one prokaryotic or eukaryotic cell.

In one embodiment of the invention, the body part is alive (or not), is any tissue, water, medium, substance, cell, organelle, organ protein, lipid, DNA, RNA, biological material, preferentially localized in a specific region of an individual, preferentially originating or extracted from such region.

In an embodiment of the invention, the body part comprises a pathological site, a healthy site, and/or a nanoparticle region.

In one embodiment of the invention, the body part is or comprises a pathological site or pathological cells.

In some cases, the pathological site can be defined as an unhealthy site, or a site that is in a different condition from a site of a healthy individual, or the site of an unhealthy individual. It can comprise pathological cells, such as tumor cells, bacteria, eukaryotic or prokaryotic cells, as well as viruses or other pathological material. Pathological cells can be cells that are: i) not arranged or working as they usual do in a healthy individual, ii) dividing more quickly than healthy cells, iii) healthy cells having undergone a transformation or modification, iv) dead, sometimes due to the presence of a virus or to other organisms, or v), in contact, in interaction, with foreign material not belonging to the individual, such as viruses, where viruses can possibly penetrate, colonize, or replicate in these cells.

In some cases, pathological cells can be assimilated to viruses or to other organisms or entities that colonize cells or target cells or destroy cells or use cells or enter in interaction with cells, preferentially to enable their own reproduction, multiplication, survival, or death. In some cases, a pathological site can comprise healthy cells, preferentially with a lower number, activity or proliferation, than that of pathological cells.

In one embodiment of the invention, the body part is or comprises a healthy site or healthy cells.

In some cases, the healthy site can be defined as a site or region that comprises healthy cell(s), where a healthy cell can be defined as a cell that belongs to a healthy individual or to the body part of a healthy individual.

In some cases, the healthy site can surround the pathological site preferentially when it is located at a distance of less than 1, 10⁻¹, 10⁻³, 10⁻⁶ or 10⁻⁹ m from the pathological site.

In some cases, a cell or entity or polarizable cell or entity can be a cell that has the capacity of being polarized, preferentially observed or used or considered before, during or after its transformation or change of state from an unpolarized state to at least one state or state of polarization.

In some cases, a polarized cell or entity can be a cell or entity that is polarized, preferentially observed or used or considered before, during or after its transformation or change of state from an unpolarized state to at least one state or state of polarization.

In some cases, the transformation or change of state from an unpolarized state to at least one state or state of polarization of a polarizable or polarized state takes place or occurs when a polarizable or polarized cell or entity is brought into presence of at least one nanoparticle and/or is exposed to radiation. In some cases, a polarizable or polarized cell or entity can be a healthy cell or entity.

In some other cases, a polarizable or polarized cell or entity can be an unhealthy cell or entity.

In some cases, the number of pathological or healthy or polarized or polarizable cell(s) or entity(ies), preferentially comprised in the body part or volume exposed to the radiation, can be lower than 10¹⁰⁰, 10⁵⁰, 10²⁰, 10¹⁰, 10⁵, 10, 5, 2 or 1 cell(s) or entity(ies), preferentially per unit volume, surface, length, cm, cm2 or cm3 of body part or cell or entity.

In some other cases, the number of pathological or healthy or polarizable or polarized cell(s) or entity(ies), preferentially comprised in the body part or volume exposed to the acoustic wave or radiation, can be larger than 0, 1, 10, 30 10³, 10⁵, 10⁷, 10⁹, 10²⁰, 10⁵⁰ or 10¹⁰⁰ cell(s) or entity(ies), preferentially per unit volume, surface, length, cm, cm² or cm³ of body part or cell or entity.

In still some other cases, the ratio between the number of at least a first type of cells or entities and the number of second type of cells or entities, preferentially comprised in the body part or volume exposed to the radiation, can be lower than 10¹⁰⁰, 10⁵⁰, 10²⁰, 10¹⁰, 10⁵, 10³, 10², 10, 5, 2 or 1, where the first and second types of cells or entities are different types of cells or entities.

In some cases, the cells or entities, preferentially the first and second types of cells or entities are selected in the group consisting of: healthy, pathological, immune, polarizable, polarized cells or entities.

In still some other cases, the ratio between the number of at least a first type of cells or entities and the number of second type of cells or entities, preferentially comprised in the body part or volume exposed to the radiation, can be larger than 1, 2, 5, 10, 103, 105, 1020 or 10100.

In one embodiment, the cell or entity, preferentially immune and/or polarizable cell or entity, and/or part of it, is selected in the group consisting of: Absorptive, adipocyte, Adrenergic neural, adult, endothelial progenitor, adventitial, fibroblast, airway, deuterosomal, airway, submucosal, gland, ciliated, duct, airway, submucosal, gland, collecting, duct, epithelial, airway, submucosal, gland, duct, basal, airway, submucosal, gland, duct, ciliated, Alpha, alveolar, macrophage, alveolar, macrophage, CCL3+, alveolar, macrophage, MT-positive, alveolar, macrophage, proliferating, alveolar, type 1, fibroblast, cell, alveolar type I or II, Ameloblast, Amex_153 to 419, Anterior, lens, epithelial, apocrine, Apocrine, sweat gland, arteriole endothelial, arteriole endothelium, Astro L1 FGFR3 SERPINI2, Astro L1-6 FGFR3 AQP1, Astro L1-6 FGFR3 PLCG1, Astro_52 to 64, Astrocyte, Auditory, inner, hair, cells of organ, of Corti, B cell, B Cell Zone Reticular, Cell, B cell(/lymphocyte), Bartholin's gland, basal cell preferentially of bronchus or epithelium of lobular bronchiole or respiratory bronchiole or terminal bronchiole or trachea or urethra or olfactory epithelium or tracheobronchial tree, Basket, basophil, Basophil granulocyte and precursors, Bcell_0, BCHE (butyrylcholinesterase), Beta, Betz, Bgl_51, blood endothelial, Blood Vessel Endothelial Cell or smooth muscle cell, Bone marrow reticular tissue fibroblasts, Border cells of organ of Corti, Boundary, Bowman's gland, bronchial smooth muscle, bronchiolar smooth muscle, Brown fat, Brunner's gland, Bulbourethral gland, Bushy, CA4_190 to 198, CA13_119 to 189, Cajal-Retzius, CAP1 general capillary gCap, CAP2 aerocyte capillary, aCap, capillary endothelial, capillary endothelium, capsule mesenchymal stromal, Cardiac muscle cell, Cartwheel, CBI_298 to 307, CD4 naïve, CD4 T cell, effector memory, CD45RA, CD4 T cell memory, CD4+ T preferentially cell resident memory or memory T or single positive thymocyte, CD8 naïve or T cell effector memory or cell effector memory CD45R or cell memory, CD8+ T or resident memory or memory or single positive thymocyte, CD8aa(I) thymocyte, CD14 monocyte, CD56 bright Natural killer, cDC1 CD141-positive myeloid dendritic, cDC2 CD1c-positive myeloid dendritic, Cells of the Zona fasciculata, Cells of the Zona glomerulosa, Cells of the zona reticularis, Cementoblast, Centroacinar, Centroblast, Centrocyte, Ceruminous gland, CGE_276 to 296, Chandelier, Chemoreceptor glomus cells of carotid body, Chief, Cholinergic neurons (various types), Chromaffin cells (adrenal gland), Chrp_76 to 52, ciliated cell of terminal ciliated ducts of submucosal glands, ciliated cell of the bronchus, ciliated columnar cell of tracheobronchial tree ciliated epithelial, classical inflammatory monocyte, club, Cold-sensitive primary sensory neurons, columnar, common lymphoid progenitor, common myeloid progenitor, Connective tissue macrophage (various types), connective tissue type mast, conventional dendritic cell 1, conventional dendritic cell 2, Conventional Dendritic Cell cDC1, Conventional Dendritic Cell cDC2, COP_37 to 75, Corneal fibroblasts, Corpus luteum cell, Cortical hair shaft, cortical thymic epithelial, corticomedullary thymic epithelial, Corticotropes, Crystallin-containing lens fiber, Cuticular hair shaft cell, Cycling B, cytotoxic CD4 T, Cytotoxic T, D, Delta cell, dendritic cell, dendritic cell type 1 or DC1 or 2 or DC2 or 3 or DC3, DG_199 to 205, DLCT6b_84, DLCT6b_97 to 112, DLIT_136 to 152, DLNP_83 to 96, Dorsal Excitatory Neuron 1 to 12, Dorsal Inhibitory Neuron 1 to 9, double negative thymocyte preferentially 2 to 4, Double-bouquet, Duct, early erythroid, early Megakaryocyte, early thymic progenitor, eccrine, Eccrine sweat gland clear or dark, Effector Chondrocyte, effector memory CD4-positive, alpha-beta T cell, effector memory CD8-positive, alpha-beta T cell, Efferent duct, Elastic cartilage chondrocyte, EMSN_222 to 426, Endo L2-5 NOSTRIN SRGN, endocrine lineage, endothelial, endothelial cell of artery, Endothelial Cell of Lymphatic Vessel, endothelial cell of respiratory system lymphatic vessel, endovascular extravillous trophoblast, Enteric glial, Enterochromaffin, Enterochromaffin-like, enterocyte, Eomes low Liver NK, eosinophil, Epen_65 to 74, Ependymal, Epidermal basal cell (stem cell), Epidermal Langerhans, Epididymal basal, Epididymal principal, Epithelial reticular cell, epithelial stem, Epsilon, Erythrocyte (red blood cell), erythroid, Exc L2 LAMP5 KCNG3, Exc L2 LINC00507 ATP7B, Exc L2 to Exc L6 of at least one type, Fb1_24 to 31, fibroblast, fibroblast of pulmonary artery, Fibrocartilage chondrocyte preferentially 1 or 2, Follicular Dendritic, Fork neurons, Foveolar, G, Gall bladder epithelial, gammadelta T, ganglion, Gland of Littré, Gland of Moll cell in eyelid, glandular epithelialll, glomus, goblet cell of epithelium of lobar bronchus Golgi, Gonadotropes, Granule, granulocyte-macrophage progenitor, Granulosa, Granulosa lutein, Grid, group 3 innate lymphoid, Head direction, Heat-sensitive primary sensory neurons, Helper T, Stem cell preferentially Hematopoietic preferentially committed progenitors for the blood and immune system, Henle's layer hair root sheath cell, Hensen's cells of organ of Corti, Hepatic stellate cell (Ito cell), Homeostatic Chodrocyte, Huxley's layer hair root sheath, Hyaline cartilage chondrocyte, Hyalocyte, Hypertrophic Chondrocyte, I cell, ILF lymphatic endothelium, ILV high endothelial venule endothelium, immature preferentially immune, preferentially B, NKT, Inh L1 LAMP5 or PAX6 PVALB or SST or VIP or TH of at least one type, innate lymphoid, Inner hair cells of vestibular system of ear, Inner phalangeal cells of organ of Corti, Inner pillar cells of organ of Corti, Insulated preferentially goblet, Intermediate skeletal muscle cell, interstitial cell of Cajal, interstitial extravillous trophoblast, Interstitial kidney cells, Interstitial macrophage, Intestinal brush border cell (with microvilli), ionocyte epithelia, Juxtaglomerular, K, Keratinocyte, Kidney distal tubule cell, L, L5ET_113 to 118, Lacrimal gland or duct cell, Lactotropes, Langerhans, late erythroid, Leydig cell, Liver lipocyte, LLC_264 to 275, Loop of Henle thin segment cell, Loose connective tissue fibroblasts, LRL_315 to 322, Lugaro cells, luminal epithelial cell, lung ciliated cell, lung megakaryocyte, lung neuroendocrine, lung parenchyma resident eosinophil, lung perichondrial fibroblast, lung pericyte, lymph node lymphatic vessel endothelial, lymphatic endothelial, Lymphatic Endothelial Cell - Subcapsular Sinus Ceiling, Lymphatic Endothelial Cell of Medulla Ceiling, Lymphatic Endothelial Cell-Subcapsular Sinus Floor, Lymphatic Endothelial Cell-Trabeculae, lymphatic endothelium, lympho-myeloid progenitor cell, lymphocyte, Macrophage, M0, M1, M2, Macula densa, Magnocellular neurosecretory, Mammary gland, Marginal Reticular, Martinotti, mast, mature CD8 T, mature Natural killer, mature NK T, Medial Excitatory Neuron 1 to 4, Medial Inhibitory Neuron 1, Medium spiny neurons, Medullary hair shaft, Medullary Reticular, medullary thymic epithelial cell preferentially type 1 to 4, Megakaryocyte, megakaryocyte-erythroid-mast cell progenitor, melanocyte, Melanotropes, memory B, Memory CD4+ T, Memory CD8+ T, Merkel, Merkel cells of epidermis, Mesangial, mesothelial cell, mesothelial cell of visceral pleura, MGE_236 to 263, Mgl_4 to 12, Micro L1-6 TYROBP CD74, Microglia, mid erythroid, Midi_433 to 444, migratory dendritic cell, Misc_116 to 404, , Mmb_323 to 333, Mo cell (or M cell), Mono_3, monocyte, Monocyte (white blood cell), Monocyte Derived Cell, Monocytoid B Cell, motor neuron, MSN_206 to 240, mucosal invariant T cell, mucus secreting cell of bronchus submucosal gland or of trachea gland multipotent progenitor cell, muscle, myelocyte, Myeloid progenitor, myo-medullary thymic epithelial, myoepithelial, Myoepithelial, myoepithelial cell of bronchus submucosal or of trachea gland, Myofibroblast, Myosatellite cell (stem cell), N, naive B, Naive CD4+ T, Naive CD8+ T, naive thymus-derived CD4-positive, alpha-beta T, naive thymus-derived CD8-positive, alpha-beta T, natural killer, nerve, nerve/schwann, neuro-medullary thymic epithelial, neuroendocrine, neuroendocrine cell of epithelium of lobar bronchus, Neurogliaform, neutrophil, Neutrophil granulocyte NK CD56bright, NK cell, Nkcell_2, non-classical or patrolling monocyte, Nuclear bag cell, Nucleus pulposus cell, Odontoblast, Olfactory ensheathing, Olfactory epithelium supporting, Olfactory receptor neurons, Oligo L2-6 OPALIN FTH1P3, Oligo L2-6 OPALIN MAP6D1, Oligo L3-6 OPALIN ENPP6, Oligo L5-6 OPALIN LDLRAP1, Oligo_40 to 50, Oligodendrocyte, Oligodendrocyte Precursor Cell, Oogonium/oocyte, OPC L1-6 PDGFRA COL20A1, OPC_32 to 36, Organ of Corti interdental epithelial, Osteoblast/osteocyte, Osteoclast, Osteoprogenitor, Other nonepithelial fibroblasts, Outer hair cells of vestibular system of ear, Outer phalangeal cells of organ of Corti, Outer pillar cells of the organ of Corti, Outer root sheath hair, Oxyphil, Pain-sensitive primary sensory neurons, Pancreatic acinar, Pancreatic stellate, Paneth, Paneth, Paneth, Parafollicular, Parathyroid chief, Parietal, Parietal epithelial, Parvocellular neurosecretory cells, Peptidergic neural (various types), Per_21 to 23, perichondrial fibroblast, pericryptal fibroblastic sheath, pericyte, perineurial, Peripolar, perivascular smooth muscle, perivascular support, Photoreceptor blue-sensitive cone cells of eye, Photoreceptor green-sensitive cone cells of eye, Photoreceptor red-sensitive cone cells of eye, Photoreceptor rod cells, Pituicytes, Place, Planum semilunar epithelial cell of vestibular system of ear, plasma, Plasmablast, Plasmacytoid Dendritic, Podocyte, gamma, precursor B, Prefibrocartilage Chondrocyte, Principal, pro B progenitor, promonocyte, Proprioceptive primary sensory neurons, Prostate gland, Proximal tubule brush border, pulmonary artery endothelial, pulmonary ionocyte, Purkinje, Purkinje fiber, Red skeletal muscle (slow twitch), regulatory CD4+ T, Regulatory Chondrocyte 1 or 2, regulatory T, Renshaw, Reparative Chondrocyte, respiratory suprabasal , Retina horizontal, S, SA node, Salivary gland serous, Satellite glial, Schwann, Sebaceous gland, sebocyte, secondary crest myofibroblast, Seminal vesicle, serous cell of epithelium of bronchus, serous cell of epithelium of trachea, serous secreting cell of bronchus submucosal gland, Sertoli, skeletal muscle, smooth muscle, Smooth Muscle preferentially of the pulmonary artery, Somatotropes, SOX9+ epithelial cell, Speed cells, Spermatid, Spermatocyte, Spermatogonium, Spermatozoon, Spindle neurons, Splat_235 to 432, squamous, Starburst amacrine, Stellate, stem, Striated duct, stromal, subepithelial membrane, Surface epithelial, surface intraepithelial, CD4-CD8- lymphocyte alpha/beta, surface intraepithelial, CD4+ lymphocyte, surface intraepithelial, CD8+, alpha, alpha positive, alpha/beta lymphocyte, surface intraepithelial, CD8+, alpha beta positive, alpha/beta lymphocyte, surface intraepithelial CD8+, alpha, alpha positive, alpha/beta lymphocyte, surface intraepithelial, CD8+, lymphocyte gamma/delta, sympathetic neuron, Sympathetic Neuron, T, T Cell, Zone Reticular, T Cytotoxic, T follicular helper, T Follicular Regulatory, T helper, T killer, T reg, Tanycytes, Taste bud supporting, Taste receptor cells of taste bud, TB low Liver NK, Tcell_1, Tendon fibroblasts, Th1, Th2, Th17, Theca Interna, Theca lutein, Thex_372 to 460, thymic capsular fibroblast, thymic interlobular fibroblast, Thyroid epithelial, Thyrotropes, Tingible Body Macrophage, tissue resident mucosal type mast, Touch-sensitive primary sensory neurons, tracheal goblet, tracheobronchial chondrocyte, tracheobronchial goblet, tracheobronchial smooth muscle, transient amplifying, transitional, transitional B, transitional Dendritic, Transitional epithelium, Transitional principal-intercalated, Trichocyte, tuft, Type I pneumocyte, Type II pneumocyte, ULIT_120 to 138, Unipolar brush cells, URL_297 to 312, uterine dendritic cells, uterine macrophage, uterine natural killer cell, uterine smooth muscle type II cell, Uterus endometrium cell, vein endothelial cell, vein endothelial cell of respiratory system, VendA_1 8, VendAC_14, VendC_15, VendPLVAP_13, VendV_17, VendVC_16, venous endothelium, Ventral Excitatory Neuron 1 to 3, Ventral Inhibitory Neuron 3, venule endothelium, Vestibular apparatus supporting, VLMC L1-5 PDGFRA COLEC12, Von Ebner's gland, Vsmc_19 or 20, White fat cell, and/or White skeletal muscle cell(s).

In another embodiment, the cell, preferentially immune and/or cell or polarizable cell, is not at least one of the cells of the previous embodiment.

In another embodiment, the cell can switch from one type to another type or from being un-differentiated to being differentiated or from being differentiated to being un-differentiated or from one state or state of polarization to another state or state of polarization, preferentially following at least one step of the method according to the invention.

Preferentially, the cell or entity is of one first type or has a first polarization or has a first condition of being differentiated or undifferentiated preferentially when it contains or comprises nanoparticles of number N1 and/or size S1 and/or composition S1 and/or magnetic property M1 and/or metallic composition MC1 and/or composition comprising a core and a coating and/or assemble within a first geometric assembly, preferentially in one of its organelles or components, and/or when it is exposed to radiation.

Preferentially, the cell or entity is of one second type different from the first one or has a second polarization different from the first one or has a second condition of being differentiated or undifferentiated different from the first one preferentially when it contains or comprises nanoparticles of number N2 different from N1 and/or size S2 different from S1 and/or composition C2 different from C1 and/or magnetic property M2 different from M1 and/or metallic composition MC2 different from MC1 and/or composition not comprising a core and a coating and/or assemble within a second geometric assembly different from the first one, preferentially in one of its organelles or components, and/or when it is exposed to radiation.

Preferentially, at least one cell or entity organelle or part or component of a cell or entity is selected in the group consisting of: i), Cell or entity membrane, preferentially plasma membrane, or preferentially an internal or external cell or entity component, that is preferentially an outer or inner cell or entity layer that preferentially controls what enters and exits the cell or entity, ii) Nucleus, or preferentially an internal or external cell or entity component, that preferentially controls or comprises at least one center or component that comprises DNA that preferentially regulates gene expression, iii) Mitochondria, or preferentially an internal or external cell or entity component, that is preferentially the powerhouses that preferentially produces energy (ATP) preferentially from glucose and/or oxygen, iv) Endoplasmic reticulum, or preferentially an internal or external cell or entity component, that is preferentially a network of membranes that preferentially synthesize and/or transport proteins and/or lipids, v) Golgi apparatus, or preferentially an internal or external cell or entity component, that is preferentially a stack of preferentially flattened sacs that preferentially modify, sort, and/or package proteins and/or lipids, vi) Lysosome, or preferentially an internal or external cell or entity component, that is preferentially the digestive cell unit that preferentially breaks down waste and/or foreign materials, vii) Peroxisomes, or preferentially an internal or external cell or entity component, that preferentially is a detoxifying unit that preferentially breaks down harmful substances, viii) Centrosome and/or centrioles, or preferentially an internal or external cell or entity component, that is preferentially an organizing center, that preferentially helps in cell division and/or movement.

Preferentially, at least one cell or entity component is selected in the group consisting of: at least one organelle, macromolecule, protein, enzyme, lipid, nucleic acid, biomolecular complex, ribosome, membranes, exosome, vesicle, endosome, intracellular cell or entity component, and extracellular cell or entity component.

In another embodiment of the invention, the body part, healthy or pathological site, or nanoparticle region, has a length, surface area, or volume, which is larger than 0, 10³, 0, 1, 10⁻¹, 10⁻², 10⁻³, 10⁻⁴, 10⁻⁵, 10⁻⁶, 10⁻⁷, 10⁻⁸, or 10⁻⁹ or 10⁻²⁰ preferentially measured in m, m², or m³, respectively.

In another embodiment of the invention, the body part, healthy or pathological site, or nanoparticle region, has a length, surface area, or volume, which is lower than 10³, 0, 1, 10⁻¹, 10⁻², 10⁻³, 10⁻⁴, 10⁻⁵, 10⁻⁶, 10⁻⁷, 10⁻⁸, or 10⁻⁹ preferentially measured in m, m², or m³, respectively.

In one embodiment of the invention, the nanoparticles or cell or entity are administered to or in the body part or cell or entity, when they are directly administered to the body part or cell or entity or when they are administered close to the body part, preferentially less than 1, 10⁻¹, 10⁻², 10⁻³, 10⁻⁴, 10⁻⁵, 10⁻⁶ or 10⁻⁹ m away from the body part or cell or entity. In this case, the nanoparticles may not need to be transported or diffuse from the region where they are administered to the body part or cell or entity.

In another embodiment of the invention, the nanoparticles or cell or entity are administered to or in the body part or cell or entity, when they are administered far from the body part or cell or entity, preferentially more than 1, 10⁻¹, 10⁻², 10³, 10⁻⁴, 10⁻⁵, 10⁻⁶ or 10⁻⁹ m away from the body part or cell or entity. In this case, the nanoparticles or cell or entity may be transported or diffuse from the region where they are administered to the body part or cell or entity.

In one embodiment, the cell or entity, preferentially the cell or polarizable cell or entity, is a pathological or a healthy cell or entity or a cell or entity that evolves or moves or diffuses from being comprised in or having at least one property associated with a healthy site to being comprised in or having at least one property associated with a pathological site, or inversely from being comprised in or having at least one property associated with a pathological site to being comprised in or having at least one property associated with a healthy site .

In another embodiment, the cell or entity, preferentially the polarizable cell or entity, belongs to or is comprised in a pathological site such as a tumor microenvironment or viral focus or a healthy site such as the site or portion of a healthy organ or blood or body part or individual or body part being healed or wound.

In one embodiment of the invention, a first change or control of the polarization state of at least one cell or entity or polarizable cell or entity from at least one unpolarized state to at least one first state or state of polarization or from at least one first state or state of polarization to at least one second state or state of polarization occurs or is triggered by associating or bringing together or making interact at least one cell or entity or polarizable cell or entity with at least one nanoparticle.

In another embodiment of the invention, a second change or control of the polarization state of at least one cell or entity or polarizable cell or entity from at least one unpolarized state to at least one first state or state of polarization or from a at least one first state or state of polarization to at least one second state or state of polarization occurs or is triggered by exposing to radiation at least one cell or entity or polarizable cell or entity.

In one embodiment of the invention, a third change or control of the polarization state of at least one cell or entity polarizable cell or entity from at least one unpolarized state to at least one first state or state of polarization or from at least one first state or state of polarization to at least one second state or state of polarization occurs or is triggered by exposing to radiation at least one cell or entity or polarizable cell or entity associated or in interaction or brought together with at least one nanoparticle.

In some cases, the first or second or third change or control of the polarization state of at least one cell or entity or polarizable cell or entity occurs during or in or activates or ends or starts or facilitates or is the result of at least one step of the method according to invention.

Preferentially, the cell or entity is polarizable, polarized, undifferentiated and/or differentiated.

In some cases, the cell or entity comprises at least one nanoparticle.

In some other cases, the cell or entity does not comprise at least one nanoparticle.

In still some other cases, the cell or entity comprising or not the nanoparticle is not exposed to radiation. In still some other cases, the cell or entity comprising or not the nanoparticle is exposed to radiation.

In one embodiment, the method is used in or for a treatment, preferentially a medical treatment or diagnosis or therapy or detection or activation or change of state, preferentially polarization state, preferentially of a type or state of cell, preferentially cell or entity or polarizable cell or entity.

In some cases, a medical treatment can be the treatment of an illness such as an infectious disease, a cancer, or a therapeutic treatment. It can be the treatment or activation or detection or change of state of a disease or polarizable or polarized cell, preferentially due to or following the malfunction or undesired function of an organ or body part or polarizable or polarized cell. It can be due to the malfunction or undesired function of a body part or polarizable or polarized or polarized cell. In some cases, it can be a diagnostic of a disease or the detection of polarized or polarizable cell or a cosmetic treatment. In some cases, it can induce the death, destruction, denaturation, inactivation, disappearance, change of state, preferentially polarization state, of at least 1, 10, 103, 106, or 109 biological material(s), such as cell(s), preferentially pathological or polarizable or polarized cell(s), RNA, DNA, protein(s), lipid(s), or enzyme(s), where the change of state of cell(s) can occur through its association with nanoparticle(s) under the application (or not) of radiation.

The invention also relates to nanoparticles for use according to the invention, wherein the medical treatment is the treatment of a disease or disorder selected from the group consisting of: a disease associated with a proliferation of cells that is different from the cellular proliferation in a healthy individual, a disease associated with the presence of pathological cells in the body part, a disease associated with the presence of a pathological site in an individual or body part, a disease or disorder or malfunction of the body part, a disease associated with the presence of radio-resistant or acoustic-resistant cells, an infectious disease, an auto-immune disease, a neuropathology, a cancer, a tumor, a disease comprising or due to at least one cancer or tumor cell, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, anemia, preferentially iron anemia, and a personality disorder.

In some cases, the disease or disorder can be the disease or disorder of or belonging to the individual or body part, or the disease or disorder from which the individual is suffering.

In one embodiment of the invention, the cancer or tumor selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, waldenstrom macroglobulinemia wilms tumor, castleman disease ewing family of tumor, gastrointestinal carcinoid tumor, gastrointestinal stromal tumor, myelodysplastic syndrome pituitary tumor, and a cancerous disease such as gestational trophoblastic disease, Hodgkin disease, Kaposi sarcoma, malignant mesothelioma, and multiple myeloma.

In one embodiment, the disorder or malfunction of the body part or cell, preferentially polarizable cell, is associated with the malfunction of cells, which divide more rapidly or enter in an apoptotic or necrotic state for example, or with the malfunction of the immune system or immune cell(s).

In one embodiment of the invention, the medical treatment is the treatment of an anemia.

In one embodiment of the invention, the nanoparticle can be of at least one type.

In one embodiment of the invention, the nanoparticle and/or cell and/or entity has at least one property selected among: i) Nanoparticle and/or cell and/or entity having a metallic composition preferentially predominantly comprising one metallic element, most preferentially iron or gold, ii) Nanoparticle and/or cell and/or entity having a bi-metallic or multi-metallic composition preferentially predominantly comprising at least two metallic elements, most preferentially iron and gold, iii) Nanoparticle and/or cell and/or entity having at least one magnetic property, iv) nanoparticle and/or cell and/or entity being plasmonic or having the ability to generate or be the source of plasmonic wave, preferentially in majority at the surface of the nanoparticle, preferentially under the application of a radiation, preferentially light or a laser, v) nanoparticle and/or cell and/or entity displaying a specific assembly, preferentially an assembly in a geometric figure, preferentially following their interaction or formation in or with at least one cell or cell component, preferentially a chain, preferentially leading to uniform nanoparticle distribution, vi) nanoparticle and/or cell and/or entity being a nanoparticle of a size smaller than 1000, 100, 10 or 1 nm, vii) nanoparticle(s) and/or cell and/or entity forming an assembly of nanoparticles and/or cell and/or entity, preferentially uniformly distributed, preferentially with less than 100%, 50% or 1% of nanoparticle and/or cell and/or entity cores or interior touching each other, preferentially with more than 0, 1, 5, 10 or 50% of nanoparticle and/or cell and/or entity coatings or exterior preferentially surface touching each other, viii) nanoparticles and/or cell and/or entity acting as or being sono-sensitizer, radio enhancer or enhancer, preferentially local enhancer, of radiation, where the local enhancement of the radiation can be an increase, preferentially locally or around or near or within less than 1000 nm away from the nanoparticle(s) and/or cell and/or entity, of the radiation preferentially applied on the nanoparticle and/or cell and/or entity or of heat or of radical oxygen species or of the number of atom(s) preferentially released from the nanoparticle and/or cell and/or entity, viii) nanoparticles and/or cells and/or entities acting as or being local absorber of radiation, where the local absorption of the radiation can be a decrease, preferentially locally or around or near or within less than 1000 nm away from the nanoparticle(s) and/or cells and/or entities, of the radiation preferentially applied on the nanoparticle and/or cells and/or entities or of heat or of radical oxygen species or of the number of atom(s) preferentially released from the nanoparticle, ix) nanoparticles and/or cells and/or entities being activated when a cell or polarizable cell or entities has changed its state or state of polarization, preferentially under or after application of at least one radiation on at least one nanoparticle or on at least one nanoparticle and at least one cell or polarizable cell or entity, ix) nanoparticle being de-activated when a cell or polarizable cell or entity has not changed its state or state of polarization, preferentially before or without the application of at least one radiation on at least one nanoparticle or on at least one nanoparticle and at least one cell or polarizable cell or entity, x) nanoparticle and/or cells and/or entities absorbing at least one radiation, wherein the absorbing nanoparticle preferentially emits, diffuses, diffracts, a radiation that is different or has at least one different parameter from the radiation that is initially absorbs by the nanoparticle and/or cells and/or entities, and xi) a photo-sensible nanoparticle and/or cell and/or entity, wherein the photo-sensible nanoparticle and/or cell and/or entity is preferentially a nanoparticle and/or cell and/or entity that generates at least one radical specie, preferentially following its exposure to light.

In one embodiment, the at least one nanoparticle and/or/with/without the at least one cell or polarizable cell or entity or the at least one cell or polarizable cell or entity comprising the at least one nanoparticle has/have at least one magnetic property, preferentially selected in the group consisting of: diamagnetic, paramagnetic, superparamagnetic, ferromagnetic, and ferrimagnetic behavior.

In one embodiment, the geometric figure and/or type of assembly of the at least one nanoparticle and/or cell and/or entity, preferentially polarizable and/or polarized cell and/or entity, is/are selected in the group consisting of: a Balbis, Concave polygon, Constructible polygon, Convex polygon, Cyclic polygon, Equiangular polygon, Equilateral polygon, Penrose tile, Polyform, Regular polygon, Simple polygon, Tangential polygon, Polygons with specific numbers of sides, Henagon, Digon, Triangle, Acute triangle, Equilateral triangle, Heptagonal triangle, Isosceles triangle, Obtuse triangle, Rational triangle, Right triangle, Kepler triangle, Scalene triangle, Quadrilateral, Cyclic quadrilateral, Kite, Parallelogram, Rhombus, Lozenge, Rhomboid, Rectangle, Square, Tangential quadrilateral, Trapezoid, Isosceles trapezoid, Pentagon, Hexagon, Lemoine hexagon, Heptagon, Octagon, Nonagon, Decagon, Hendecagon, Dodecagon, Tridecagon, Tetradecagon, Pentadecagon, Hexadecagon, Heptadecagon, Octadecagon, Enneadecagon, Icosagon, Swastika, Star polygon, Pentagram - star polygon, Hexagram, Star of David, Heptagram, Octagram, Star of Lakshmi, Decagram - star polygon, Annulus, Arbelos, Circle, Archimedes' twin circles, Bankoff circle, Circumcircle, Disc, Incircle and excircles of a triangle, Nine-point circle, Circular sector, Circular segment, Crescent, Indalo, Lens, Lune, Reuleaux polygon, Reuleaux triangle, Salinon, Semicircle, Tomahawk, Triquetra, Heart, Archimedean spiral, Astroid, Cardioid, Deltoid, Ellipse, Heart, Heartagon, Various lemniscates, Oval, Cartesian oval, Cassini oval, Oval of Booth, Ovoid, Superellipse, Taijitu, Tomoe, and a Magatama, preferentially of the at least one nanoparticle and/or cell, preferentially polarizable and/or polarized cell.

In some cases, the geometric figure and/or type of assembly of the at least one nanoparticle and/or cell, has at least one property selected in the group consisting of: i) it prevents aggregation, ii) it enables uniform distribution, iii) it enables or favors communication or exchanges between the at least one nanoparticle and/or cell.

In some cases, the nanoparticle and/or cell and/or entity can comprise a core or inner part and/or coating or external part.

In some cases, the distance between the at least two nanoparticles and/or the at least two cells or entities can be smaller than or equal to or to 10²⁰, 10¹⁰, 10⁵, 10³, 10, 5, 2, 1 or 0 nm.

In some other cases, the distance between the at least two nanoparticles and/or the at least two cells or entities can be larger than or equal to 10⁻²⁰, 10⁻¹⁰, 10⁻⁵, 10⁻³, 10⁻¹, 0, 1, 2, 5 10 or 100 nm.

In some cases, the distance and/or type of assembly and/or geometric figure can in some cases favor the emission or production or triggering or relay or transmission preferentially by or associated with at least one first type of cell or entity of preferentially at least one immune entity or message or pathway such as an interleukin or cytokine or biological pathway preferentially in full or in part and/or preferentially the reception or absorption or reading or decoding preferentially by at least a second type of cell of preferentially at least one immune entity or message or pathway such as an interleukin or cytokine or biological pathway preferentially in full or in part.

In some cases, the at least one entity, preferentially immune entity, or message or pathway preferentially associated with the first type of cells or entities is preferentially the same as the at least one entity, preferentially immune entity, or message or pathway preferentially associated with the second type of cells or entities, i.e. that preferentially it is not modified or preserves its identity, nature, structure and/or function preferentially between or during its transmission, diffusion, preferentially between the at least two different cells or entities or types of cells or entities.

In some cases, a first type of cell or entity has a first polarization.

In some other cases, a second type of cell or entity has a second polarization different from the first polarization.

In some other cases, the at least one entity, preferentially immune entity, or message or pathway preferentially associated with the first type of cells or entities is preferentially different from the at least one entity, preferentially immune entity, or message or pathway preferentially associated with the second type of cells or entities, i.e. that preferentially it is modified or changes its identity, nature, structure and/or function preferentially between or during its transmission, diffusion, preferentially between the at least two different cells or entities or types of cells or entities.

In some cases, the at least one immune entity or message or pathway, preferentially its diffusion or activation or inactivation or triggering or reception or emission, preferentially in part or fully, preferentially between the at least two types of cells or entities, is/are preferentially triggered or started or ended or activated or inactivated by associating or bringing together the at least one type of cell or entity with at least one nanoparticle, preferentially under the application of radiation.

In some cases, the diffusion or activation or inactivation or triggering or reception or emission of at least one immune entity or message or pathway, preferentially in part or fully, preferentially between or by the at least one or two type(s) of cells or entities, preferentially polarized or polarizable, triggers or starts or ends or activates or inactivates a change of polarization of the at least one or two cells or entities, or a change from an undifferentiated to a differentiated state of at least one cell or entity.

In some cases, an unpolarized or polarizable cell or entity can be an undifferentiated cell or entity.

In some cases, a polarized or polarizable cell or entity can be a differentiated cell or entity.

In some cases, a cell or entity or polarizable cell or entity can be undifferentiated, differentiated, unpolarized, polarized, preferentially being in this case in at least one polarized state, wherein each or at least one of these states can preferentially be associated with a phenotype or size or activity, concentration, morphology, size, stiffness, strength, movement, existence, oxygen or oxygen concentration or metallic or nanoparticle or salt concentration, preferentially of the cell(s) and/or entity(ies), wherein at least one or two of this/these phenotype(s), and/or size(s), an/or activity(ies), concentration(s), morphology(ies), size(s), stiffness(es), strength(s), movement(s), existence(s), oxygen or oxygen concentration(s), metallic or nanoparticle or salt concentration(s), and/or state(s) can be associated with a given cell geometry, size, volume, morphology, stiffness, oxygen, gaz, metallic concentration, movement and/or activity, preferentially immune activity.

In one embodiment of the invention, the biological pathway is the pathway of the entity or cell or the pathway that leads to a change of polarization of the at least one cell or entity

In some cases, the biological pathway is selected in the group consisting of: i) metabolic pathway, ii) gene-regulated pathway, and iii) signal preferentially signal transduction preferentially triggered pathway.

In one embodiment of the invention, the biological pathway is or consists in or triggers or activates or deactivates or changes preferentially the nature or function of preferentially at least one gene or gene regulation, chemical reaction, change of state or polarization of a cell, transformation or storage or degradation or elimination of food or molecule into energy or other molecules, preferentially in the body part.

In another embodiment, the biological pathway is or consists in or triggers or activates or deactivates or changes preferentially the nature or function of a signal, preferentially from a cell's exterior to its interior, preferentially its reception, preferentially through or by or with structures, preferentially through or by or with receptors, preferentially through or by or with cell surface receptors, preferentially through or by or with interaction(s) or association(s) with or through or by these structures or receptors, preferentially through or by or with or involving signal traveling preferentially into or form or outside or inside the at least one cell or cell component or organelle. In some cases, the signal can be a message that is preferentially transmitted or diffused or activated or deactivated or turned off or on or absorbed or internalize or externalized preferentially from or by at least one cell or body part preferentially by specialized or specific or certain immune entity(ies) or biological material or protein(s) or lipid(s) or enzyme(s) that trigger(s) at least one preferentially specific reaction preferentially in or from the cell or body part.

In some cases, the biological pathway can be or be associated with at least one chemical signal preferentially from inside or outside the cell, preferentially triggering preferentially directly or indirectly the cell to produce or active or deactivate a particular protein or biological material preferentially inside or outside the at least one cell or body part.

In some cases, the biological pathway can be associated with or triggers the activation or deactivation or change of polarization or differentiation state of at least one protein or biological material or cell preferentially polarizable cell or entity that preferentially triggers a cell movement, cell differentiation, and/or cell change of state or state of polarization.

In one embodiment, the cell or entity or nanoparticle or body part or pathway or reaction, preferentially biological and/or metabolic, the state or polarization or differentiation or transformation or change of polarization preferentially of the cell or entity, preferentially in part or in full, involves or comprises or is comprised in or is selected in the group consisting of at least one of: molecule, basic, oxidized, reduced acid molecule, alcohol, ethanol, inositol phosphate, Carbohydrate, Sugar, Ascorbate, aldarate, Chondroitin sulfate, dermatan sulfate, Citric Acid Cycle, Eicosanoid, Fructose, Galactose, Glucose, Energy, ATP, ADP, Glycogen, Glycolysis, Gluconeogenesis, Glycosaminoglycan, molecule, Heparan sulfate, Keratan sulfate, keratin, Mannose, Naphthalene, Pentose phosphate, Pyruvate, Respiratory Electron Transport, Sorbitol, Starch, sucrose, Cell Cycle, Mitosis, meiosis, at least one phase of mitosis or meiosis, Mitosis,: G1-G1/S phases, Mitosis: G2-G2/M phases, Mitosis: M-M/G1 phases, Mitotic Metaphase and/or Anaphase, Drug, 4-hydroxybenzoate, Abacavir, Acetaminophen, Butirosin, neomycin, Caffeine, Codeine, morphine, Nicotine, Tamoxifen, Lipid, enzyme, DNA, RNA, protein, amino acid, nucleic acid, Fatty Acid, Arachidonic acid, Bile acid, bile salt, unsaturated fatty acids or molecule, Cholesterol, Ether lipid, Fatty acid beta or molecule, Fatty Acid, Fatty acid, Fatty acid omega, Fatty acid, triacylglycerol, ketone, Glycerolipid, Glycerophospholipid, Glycosphingolipid, Glyoxylate, dicarboxylate, HETE and HPETE, Leukotriene Linoleic acid, Lipoic acid, lipids, lipoproteins, Oleate, matrix, preferentially extracellular matrix, Palmitate, Peroxisomal lipid, Phospholipid, Propanoate, Prostaglandin, Sphingolipid, Stearate, Neurotransmitter, Acetylcholine, Anandamide, Dopamine, Serotonin, Nucleotide, Nucleoside, Amino sugar, nucleotide sugar, Purine, Pyrimidine, Wybutosine, Peptide, Hormone, Catecholamine, Melatonin, Noradrenaline, adrenaline, Peptide, hormone, Regulation of insulin or molecule, secretion of molecule, Renin-angiotensin system, Protein, Amino Acid, Alanine, aspartate, glutamate, Arginine, proline Arylamine Asparagine, nbeta-Alanine Butanoate, Citrulline, Collagen, Creatine, D-arginine, D-ornithine, D-Glutamine, D-glutamate, Glutamine, Glutathione, Glycine, serine, threonine, Histamine, Histidine, Lysine, Methionine, cysteine One-carbon Phenylalanine, Putrescine, Spermine, spermidine, Taurine, hypotaurine, tRNA, Tryptophan, Tyrosine, Urea, CycleValine, Leucine, Isoleucine, Steroid, Androgen Estradiol Estrogen, fibronectin, elastin, protein, preferentially structural protein, inhibitor or activator preferentially of pathway or biological material, growth factor, epithelial growth factor, vascular endothelial growth factor, environment or micro-environment preferentially of a healthy or pathological site, macrophage, barrier, reactome, preferentially immune-reactome, Estrone, Glucocorticoid, Mineralocorticoid steroid, hormone, Vitamin, DSteroid, hormone, thyroid hormone, Vitamin, Coenzyme, Nicotinate, nicotinamide, Biotin, Folate, NAD, Pantothenate, CoA, Propionyl-CoA, Retinoate, Retinol, Riboflavin, Thiamine, Ubiquinol, Vitamin A, carotenoid, Vitamin B6, Vitamin, Iron, metal, organic material, immune entity, cell, polarizable or polarized cell, nitric oxide, Nitrogen, carbon dioxide, Selenium, Sulfur, Benzo(a)pyrene, Porphyrin, salt, metabolism, metabolic, digestion, absorption, elimination, hydration, catabolism, synthesis, synthetism, reduction, oxidation, breakdown, appearance, disappearance, change of pH, oxidation, reduction, state, charge, composition, size, type of assembly or interaction, preferentially biologic, degradation, formation, elongation, enlargement, size reduction, and/or aminoacylation, in some cases preferentially reversible or irreversible.

In some cases, the molecule can be at least one biological material.

In one embodiment, the pathway, preferentially signaling and/or transducing and/or biological pathway, is selected in the group consisting of: Akt/PKB, AMPK, cAMP, Eph/ephrin, Hedgehog, Hippo, Insulin signal, JAK-STAT, MAPK/ERK, mTOR, Nodal, Notch, PI3K/AKT/mTOR, TGF beta, TLR, VEGF, Wnt pathway(s), preferentially signaling and/or transducing pathway(s).

In one embodiment, the pathway, preferentially biological or genomic or genetic pathway comprises at least one or two molecular regulator(s) or gene(s) or cell(s) or polarizable or polarized cell(s) that preferentially interact(s) preferentially with each other and/or preferentially with other substances or immune entity(ies) in or outside the cell or cell component to preferentially govern or trigger or activate or deactivate the expression or activation or production or reproduction of at least one gene or biological material or mRNA or protein which preferentially determine(s) the function or type or activation or deactivation of the cell preferentially polarization state of the cell.

In one embodiment, the pathway or state or state of polarization is responsible or associated with cell morphogenesis, the creation or activation or deactivation or appearance or disappearance or movement or diffusion or immobile of body structure(s) or body part.

In some cases, the nanoparticle or cell or entity can be magnetic or preferentially have at least one magnetic property or be metallic or comprise at least one metal.

Preferably, at least one entity, preferentially immune entity, is an immune-attractant, wherein an immune attractant is an immune entity that attracts at least one immune cell or entity, preferentially in the body part, preferentially to trigger the change of state of a cell or polarizable cell.

In some cases, the entity, preferentially immune entity, can be bound or associated to the at least nanoparticles or cell, preferentially polarizable or polarized cell, preferentially releasably, and is activated or released upon excitation of the nanoparticles by radiations.

Preferentially, an immune-attractant relates to a substance which attracts immune cells, optionally upon excitation of the nanoparticles by radiations.

Preferentially, the entities, immune cells or entities or polarizable or polarized cells, optionally attracted by the immune-attractant according to the invention, are preferentially selected from those belonging to the innate or adaptive immune system, or from the group consisting of: an antigen presenting cell (APC), a basophil, a dendritic cell, an eosinophil, a granulocyte, a killing cell, a leukocyte, a lymphocyte, a macrophage, a mast cell, a natural killer, a neutrophil, a phagocyte, a B or T cell, such as a CD8+T lymphocyte, a helper cell (Thi or Th2), or a gamma delta T cell.

More preferably, the entity, immune entity, polarizable or polarized cell, immune-attractant is a pathological cell, an immune cell or part of an immune cell, an immune substance or part of an immune substance.

In some cases, the entity, preferentially the immune substance or entity, can be selected in the group consisting of: amino acids, an acid such as uric acid, an antigen, an antibody, a base such as NaOH, a cluster such as a cluster of differentiation, CpG, a complex such as a major histocompatibility complex, MHC, MHC-1, MHC-2, MHC-3, a cytokine, a cytoplasmic molecule such as HMGB 1, DNA, preferentially bacterial DNA, an endotoxin, an enzyme, flagellin, glycan, glycoconjugate, a ligand such as a ligand expressed at the surface of stressed cells, an interleukin, a lipid, a lipopolysaccharide (LPS), a lipoteichoic acid, a protein, a stress protein, a heat shock protein, a formylated protein, RNA, a pathogen-associated molecular pattern (PAMP), peptidoglucan, a receptor, such as molecular pattern recognition receptor (PRR), a specific Toll-like receptor (TLR), a NOD-like receptor (NLR), a RIG-I-like receptor (RGR), or a C-type lectin receptor (CLR), a substance not belonging to the individual to be treated with the composition, an inactivated or attenuated microorganism, an inactivated toxic compound that leads to the appearance of the pathological cell, a biological substance, a subunit of protein, lipid, DNA, RNA, a substance produced by a plant, an animal, a bacterium, a fungus, a eukaryotic or prokaryotic cell, a polysaccharide, or a recombinant vector, a vaccine component or vaccine adjuvant, or an equivalently active substance to those listed above that is non-toxic such as MPLA, which is a non-toxic equivalent of LPS.

Preferably, the entity, preferentially the immune-attractant or immune entity or cell, preferentially polarizable or polarized, is linked to the nanoparticle or cell by bonds, preferentially weak bonds, which can be hydrogen bonds or van der Waals interactions.

Alternatively, the entity, preferentially the immune-attractant or immune entity or cell or cell component can be linked to or in interaction with or associated with the nanoparticle or metal complex or salts or cell by strong bonds, which can be ionic or covalent bonds.

In some cases, the metal complexes or salts differ from the nanoparticles, preferentially by at least one property in the group consisting of:
They are or act as precursor(s) of nanoparticle(s);
They are not crystallized or don't comprise a crystallized structure or a structure with well-ordered atoms, preferentially of size larger than 0.1, 1, 10 or 50 nm;
They comprise an assembly of atoms that interact through weak interactions or through at least two different types of interaction or with a distribution of distances separating at least two different atoms within the assembly that is large or that is larger than 0.1, 1, 10 or 50 nm;
In some cases, or for a first part of them, they have a size smaller than 0.1, 1, 10 or 50 nm;
In some other cases, or for a second part of them, they have a size larger than 100, 104 or 105 nm. Preferentially, the entity, preferentially the immune-attractant or immune entity or cell, preferentially polarizable or polarized, is associated or in interaction with the nanoparticle or cell.

In one embodiment of the invention, the nanoparticle is of at least one type preferentially before, during or after its use in the method or one step of the method, preferentially before, during or after its administration in the body part or before, during or after its interaction with at least one cell.

In one embodiment, the nanoparticle and/or cell and/or entity is/are in powder, liquid, and/or gaseous form(s).

In one embodiment of the invention, the nanoparticle(s) and/or cell and/or TAM(s) and/or entity is/are selected and/or identified and/or chosen, preferentially through at least one step of the method, preferentially by:
Carrying out at least one biocompatibility or toxicity test, preferentially selected in the group consisting of: cytotoxic and/or not irritant and/or not genotoxic and/or without leading to acute and/or chronic toxicity, pyrogenic, preferentially to select the nanoparticle, cell and/or entity that is biocompatible and non-toxic, preferentially in the presence of or towards the cell or polarizable cell or entity, where this sub-step is preferentially carried out by following the guidelines on toxicity assessment of drugs or medical devices such as ISO 10993;
Carrying out at least one test of physico-chemical characterization, preferentially selected in the group consisting of composition, surface charge, endotoxin, concentration, and size measurement, preferentially to show that the nanoparticle and/or cell and/or entity is non aggregating, and/or stable,
Carrying out at least one efficacy test, preferentially selected in the group consisting of: preferentially to select a nanoparticle and/or cell and/or entity that is efficient, preferentially in at least one of the following manners: i) the nanoparticle and/or cell and/or entity destroys or inactivates at least one pathological cell or protects at least healthy cell, preferentially either directly or without involving another entity or biological mechanism partly or fully, ii) the nanoparticle and/or cell and/or entity changes at least one state or state of polarization of at least one type of cell or entity or polarizable cell or entity, preferentially either directly or without involving another entity or biological mechanism partly or fully, preferentially in the presence of or towards the cell or entity or polarizable cell or entity, preferentially to show that the nanoparticle and/or cell and/or entity is/are efficient,
wherein at least one of i) to iii) is preferentially associated with or preferentially corresponds to a screening step, preferentially used to determine the type of nanoparticles and/or cells or entity(ies) or polarizable cells or entity(ies) that is/are the best suited for the method.

In one embodiment of the invention, the nanoparticle(s) or cell(s) or entity(ies) or polarizable cell(s) or entity(ies) or TAM is/are separated by less than 100, 100, 10, 5, 2 or 1 nm.

In another embodiment of the invention, the nanoparticle(s) and cell(s) or entity(ies) or polarizable cell(s) or entity(ies) or TAM is/are separated by more than 100, 100, 10, 5, 2 or 1 nm.

In another embodiment of the invention, at least one nanoparticle is internalized in or comprised in at least one type of cell or entity or polarizable cell or entity, where in this case the at least of nanoparticle is preferentially internalized or located or comprised in at least one type of cell or entity or polarizable cell or entity or at least one component or organelle or cytoplasm of such cell or entity, preferentially following or during at least one step of the method according to the invention.

In another embodiment of the invention, at least one nanoparticle is externalized or expelled from at least one cell or entity or polarizable cell or entity, where in this case the at least of nanoparticle is preferentially externalized or expelled or located outside at least one type of cell or entity or polarizable cell or entity or at least one component or organelle or cytoplasm of such cell or entity, preferentially following at least one step of the method according to the invention.

In another embodiment of the invention, at least one entity, preferentially immune entity, is producing or the production of at least one immune entity is induced, preferentially a cytokine or interleukin, preferentially of a first or second type, preferentially from a cell or entity or polarizable cell or entity, preferentially triggering or being concomitant with a change of state of the cell or entity or polarizable cell or entity from an unpolarized to a polarized state or from a first to second polarization state.

In one embodiment, the cell or polarizable cell or entity and/or nanoparticle and/or at least one step of the method comprise diffusion, preferentially active or passive diffusion, of at least one nanoparticle towards or in direction of at least one cell or entity or body part, preferentially following or with the application of radiation.

In one embodiment of the invention, the cell or entity or polarizable cell or entity changes its polarization or at least one step of the method results in a change of polarization state of a polarizable state from an un-polarized state such as M0 to a polarized state such as M1 or M2, or from a first polarized state to a second polarized state such as M1 to M2 or M2 to M1.

In some cases, passive diffusion can be the diffusion of at least one cell or entity and/or nanoparticle preferentially by itself / themselves and/or without or in the absence of application of a radiation preferentially on the nanoparticle, cell or entity and/or boy part.

In some cases, active diffusion can be the diffusion of at least one cell and/or nanoparticle preferentially with or in the presence of application of a radiation preferentially on the nanoparticle, cell or entity and/or boy part.

In one embodiment of the invention, at least one step of the method consists in or comprises exposing or exposure of the assembly comprising at least one nanoparticle and/or at least one cell or entity or polarizable cell or entity or TAM to as least one radiation,

In some cases, the radiation can be a perturbative radiation or a radiation triggering or creating a perturbation or a heating radiation or a radiation that heats or ionizes the at least one nanoparticle and/or body part and/or cell or entity or polarizable cell or entity and/or part of it/them,
In some cases, the temperature or temperature increase or perturbation or radiation parameter produced or applied by the radiation or physico-chemical disturbance preferentially on the at least one nanoparticle and/or at least one cell or entity or polarizable cell or entity has at least one property selected in the group consisting of:
it is sufficiently moderate not to destroy or inactivate at least one nanoparticle and/or cell or entity or polarizable cell or entity and/or to prevent the change of polarization state of at least one cell or entity or polarizable cell or entity,
it is a temperature or temperature increase or gradient smaller than 1000, 100, 75, 60, 55, 40 or 37°C, and
it is a radiation dose or parameter or intensity or wavelength or frequency or depth of penetration or duration of application or number of times that it is applied or type smaller than 1000, 100, 75, 60, 55, 10, 5, 2, 1, 10⁻¹ or 10⁻³ preferentially Gy, seconds, nm, cm, Hz, MHz, W preferentially per cm, cm² or cm³, or mm, nm² or nm³, or m, m² or m³ of body part, cell and/or nanoparticle and/or of piece of equipment emitting at least one radiation or surface, volume or length of it.

In some other cases, the temperature or temperature increase or perturbation or radiation parameter produced or applied by the radiation or physico-chemical disturbance preferentially on the at least one nanoparticle and/or at least one cell or entity or polarizable cell or entity has at least one property selected in the group consisting of:
it is sufficient or sufficiently large or sufficiently intense to destroy or activate at least one nanoparticle and/or cell or polarizable cell or entity and/or to change the polarization state of at least one cell or polarizable cell or entity,
it is a temperature or temperature increase or gradient larger than 0, 10-3, 1, 5, 37, 45, 50, 75 or 100 °C, and
it is a radiation dose or parameter or intensity or wavelength or frequency or depth of penetration or duration of application or number of times that it is applied or type larger than 0, 10⁻²⁰, 10⁻⁵, 10⁻¹, 1, 2, 5, 10, 100, 10³ or 10⁵ preferentially Gy, seconds, nm, cm, Hz, MHz, W preferentially per cm, cm² or cm³, or mm, nm² or nm³, or m, m² or m³ of body part, cell and/or nanoparticle and/or of piece of equipment emitting at least one radiation or surface, volume or length of it.

In one embodiment of the invention, the state or state of polarization of the cell or entity or polarizable cell or entity changes from the polarization state α or Mi to the polarization state Mj or β, or inversely from the polarization state Mj or β to the polarization state α or Mi,

In one embodiment of the invention, the at least one cell or entity or polarizable cell or entity or change of polarization of the cell or entity or polarizable cell or entity or at least one step of the method originates from, or is triggered or activated by, or results in or is associated with or comprises or triggers or activates the production or induction or existence of at least one entity or immune entity, preferentially a cytokine or interleukin or a component or complex of a immune cell such as a cell receptor or MHC or part or whole of a biological pathway.

In one embodiment of the invention, wherein at least one step of the method and/or the polarizable or polarized cell or entity results in or comprises the activation of at least one polarizable or polarized cell or entity against or to destroy partly or fully a pathological site.

In one embodiment of the invention, the change of polarization of a cell or entity or polarizable cell or entity is triggered by, corresponds to, is associated with, is characterized or triggered or activated or deactivated by or is a change, control, increase, or decrease preferentially by a factor of preferentially more in some cases or of preferentially less in some other cases than 0, 0.001, 0.1, 1, 5, 2 or 10 of: i) at least one category or number of entity(ies), immune entity(ies), cytokine(s), interleukin(s), preferentially produced or emitted or activated by the at least one cell or entity or polarizable cell or entity, ii) at least one polarization state of at least one cell or entity or polarizable cell or entity, iii) at least one property, type or number of radiation(s) preferentially applied on the body part, nanoparticle(s) and/or cell or entity or polarizable cell(s) or entity, and/or iv) at least one property, type or number of nanoparticle(s) and/or cell or entity or polarizable cell or entity, preferentially comprised in or associated with at least one cell or entity or polarizable cell or entity and/or body part.

In some cases, the at least one property of the radiation can be the intensity, power, frequency, energy, wavelength, depth of penetration, number of times that it is applied and/or duration of application of the radiation, preferentially per unit volume, surface and/or length of body part, nanoparticle and/or cell and/or entity.

In some cases, the at least one property of the nanoparticle and/or cell or entity or polarizable cell or entity can be the type, number, concentration, charge, surface charge, composition, type of assembly, coating or core or inner part or external part type or composition, magnetic property, metallic and/or carbonaceous composition, apyrogenicity, sterility, stability of the nanoparticle and/or cell or polarizable cell, type of interaction or binding of the nanoparticle and/or polarizable cell or entity preferentially with the nanoparticle and/or cell or entity or polarizable cell or entity, location of the at least one nanoparticle and/or cell or entity or polarizable cell or entity, preferentially inside or outside the least one nanoparticle and/or cell or entity or polarizable cell or entity or cell or entity component. In some cases, the charge of surface charge of the nanoparticle and/or cell or entity or cell or entity component can be smaller than 105, 103, 100, 50, 40, 10, 5, 2, 0, -5, -20, -50, or -100 mV, preferentially predominantly or partly or fully.

In some cases, the charge of surface charge of the nanoparticle and/or cell or entity or cell or entity component can be larger -10 000, -1000, -100, -50, -20, -10, -5, -2, -1, 0, 1, 2, 5, 10 or 50 mV preferentially predominantly or partly or fully.

In some cases, the type of nanoparticle or cell or entity is selected in the group consisting of: metallic, organic, magnetic, sterile, stable, coated, organic, inorganic, crystallized, and amorphous nanoparticle or cell, preferentially predominantly or partly or fully.

In some cases, the nanoparticle and/or cell or entity or carbonaceous composition of the nanoparticle(s) and/or cell(s) or entity is characterized by the presence of or comprises more than 10⁻²⁰, 10⁻¹⁰, 10⁻⁵, 10⁻¹, 0, 1, 5, 10, 50 % in mass or volume of carbon preferentially per nanoparticle(s) or cell(s) or entity.

In some cases, the nanoparticle and/or cell or entity or carbonaceous composition of the nanoparticle(s) and/or cell(s) or entity is characterized by the presence or comprises of less than 10-5, 100, 10, 5, 0% in mass or volume of carbon preferentially per nanoparticle(s) or cell(s) or entity.

In some cases, the nanoparticle and/or cell or entity or apyrogenicity or sterility of the nanoparticle(s) or cell(s) or entity is characterized by the presence of or comprises less than 10¹⁰, 10⁵, 10³, 100, 50, 5, 1 or 0 EU or endotoxin or EU or endotoxin per nanoparticle or cell or entity or cm³ of body part.

In some cases, the nanoparticle and/or cell or the apyrogenicity or sterility of the nanoparticle(s) or cell(s) or entity is characterized by the presence of or comprises more than 10⁻¹⁰, 10⁻⁵, 10⁻³, 10⁻¹, 0, 1, 5 or 10 EU or EU per nanoparticle or cell or entity or cm³ of body part.

In some cases, the composition of the nanoparticle(s) and/or cell(s) or entity is made of or comprises at least one or more than 0, 5, 10, 25, 50, 99 or 100% preferentially in mass of volume of metallic and/or carbon and/or atom and/or ion.

In some other cases, the composition of the nanoparticle(s) and/or cell(s) or entity is made of or comprises less than 1 or less than 10³, 100, 75, 50 or 10% preferentially in mass of volume of metallic and/or carbon and/or atom or ion.

In one embodiment, the atom or ion preferentially comprised in the nanoparticle and/or cell or entity, preferentially before, during or after its administration in the body part or assembly in the nanoparticle and/or cell or entity or dissociation from the nanoparticle and/or cell or entity, is selected in the group consisting of: H, Hydrogen, He, Helium, Li, Lithium, Be, Beryllium, B, Boron, C, Carbon, N, Nitrogen, O, Oxygen, F, Fluorine, Ne, Neon, Na, Sodium, Mg, Magnesium, Al, Aluminum, Si, Silicon, P, Phosphorus, S, Sulfur, Cl, Chlorine, Ar, Argon, K, Potassium, Ca, Calcium, Sc, Scandium, Ti, Titanium, V, Vanadium, Cr, Chromium, Mn, Manganese, Fe, Iron, Co, Cobalt, Ni, Nickel, Cu, Copper, Zn, Zinc, Ga, Gallium, Ge, Germanium, As, Arsenic, Se, Selenium, Br, Bromine, Kr, Krypton, Rb, Rubidium Sr, Strontium, Y, Yttrium, Zr, Zirconium, Nb, Niobium, Mo, Molybdenum, Tc, Technetium, Ru, Ruthenium, Rh, Rhodium, Pd, Palladium, Ag, Silver, Cd, Cadmium, In, Indium, Sn, Tin, Sb, Antimony, Te, Tellurium, I, Iodine, Xe, Xenon, Cs, Cesium, Ba, Barium, La, Lanthanum, Ce, Cerium, Pr, Praseodymium, Nd, Neodymium, Pm, Promethium, Sm, Samarium, Eu, Europium, Gd , Gadolinium, Tb , Terbium, Dy, Dysprosium, Ho, Holmium, Er, Erbium, Tm, Thulium, Yb , Ytterbium, Lu, Lutetium, Hf, Hafnium, Ta, Tantalum, W, Tungsten, Re, Rhenium, Os, Osmium, Ir, Iridium, Pt, Platinum, Au, Gold, Hg, Mercury, Tl, Thallium, Pb, Lead, Bi, Bismuth, Po, Polonium, At, Astatine, Rn, Radon, Fr, Francium, Ra, Radium, Ac, Actinium, Th, Thorium, Pa, Protactinium, U, Uranium, Np, Neptunium, Pu, Plutonium, Am, Americium, Cm, Curium, Bk, Berkelium, Cf, Californium, Es, Einsteinium, Fm, Fermium, Md, Mendelevium, No, Nobelium, Lr, Lawrencium, Rf, Rutherfordium, Db, Dubnium, Sg, Seaborgium, Bh, Bohrium, Hs, Hassium, Mt, Meitnerium, Ds, Darmstadtium, Rg, Roentgenium, Cn, Copernicium, Nh, Nihonium, Fl, Flerovium, Mc, Moscovium, Lv, Livermorium, Ts, Tennessine, Og, and Oganesson.

In some cases, the stability of the at least one nanoparticle and/or cell or entity is characterized by the fact or such that the nanoparticle and/or cell or entity remain(s) suspended or homogenously distributed, does not aggregate, does not lose at least o1, 5, 10, 10³, 10⁵, or 10¹⁰ atom(s) or property(ies) or more than 1, 5, 10, 50 or 10% of its atom(s) or molecule(s) or property(ies), preferentially during more than 10⁻¹⁰, 10⁻⁵, 10⁻¹, 0, 1, 5, 10, 10³, 10⁵, or 10¹⁰ second(s) or minute(s) or day or month or year.

In some cases, the type of interaction or binding between the at least one nanoparticle and the at least one cell or entity is weak or is such that the nanoparticle can diffuse away or leave or be expelled from or externalized from the cell or entity or is such that a cell or entity component or compartment or vesicle or organelle can be associated with or interact with or engulf at least one nanoparticle preferentially to expel or externalize or diffuse away the nanoparticle preferentially from or outside the cell or entity, preferentially with or without having to apply a radiation, preferentially with or without a decomposition of the nanoparticle and/or cell or dissociation of at least one compound or entity from the nanoparticle and/or cell or entity, preferentially in part or fully.

In some other cases, the type of interaction or binding between the at least one nanoparticle and the at least one cell or entity is strong or is such that the nanoparticle remains in or associated with or internalized in the cell or entity or is such that a cell or entity component or compartment or vesicle or organelle can be associated with or interact with or engulf at least one nanoparticle preferentially to maintain or internalize or diffuse the nanoparticle preferentially in or inside the cell or entity, preferentially with or without having to apply a radiation, preferentially with or without a decomposition or dissolution of the nanoparticle and/or cell or entity, preferentially in part or fully.

The invention relates to the method according to the invention, and/or the nanoparticle(s) and/or cell(s) or entity comprising:
not exposing the nanoparticle(s) and/or cell(s) or entity to at least one radiation, and/or the nanoparticle(s) and/or cell(s) or entity not exposed to at least one radiation, preferentially associated with or comprising no change of polarization of the polarizable or polarized cell or entity and/or no differentiation of an undifferenced cell or entity;
exposing the nanoparticle(s) and/or cell(s) or entity to at least one or two radiation(s), and/or the nanoparticle(s) and/or cell(s) or entity not exposed to at least one or two radiation(s), preferentially associated with or comprising at least one change of polarization of the polarizable or polarized cell or entity, preferentially from M0 to M1 or M0 to M2, or M1 to M2 or M2 to M1, and/or a differentiation of an undifferenced cell or entity preferentially from an undifferentiated to a differentiated state;
   and/or
assembling the cell(s) and nanoparticle(s), with or without the application of radiation, preferentially associated with or comprising at least one change of polarization of the polarizable or polarized cell or entity, preferentially from M0 to M1 or M0 to M2, or M1 to M2 or M2 to M1, and/or a differentiation of an undifferenced cell or entity preferentially from an undifferentiated to a differentiated state.

In one embodiment of the invention, the assembly comprising at least one nanoparticle and at least one un-differentiated, differentiated, un-polarized and/or polarized cell or entity is exposed to radiation or a radiation is applied on the at least one un-differentiated, differentiated, un-polarized and/or polarized cell, preferentially before, during or after at least one step of the method according to the invention.

The invention also relates to the nanoparticle(s) and/or cell(s) and/or method or at least one step of the method according to the invention, wherein the nanoparticle(s) and/or cell(s) or entity is/are detected, imaged, identified, has/have its/their polarized state(s) measured, has/have at least one state of its/their polarization state detected or identified, or has/have at least one of its/their property or change of property or change of state or state of polarization detected or measured, preferentially by determining if the cell or entity or polarizable cell or entity has changed its state or state of polarization during or following at least one step of the method;

In one embodiment of the invention, the at least one property or change of property of cell or polarizable or polarized cell or entity, preferentially its change of polarization or its differentiation, that is preferentially measured during at least one step of the method, is preferentially the morphology, size, movement, production of type or number or category of immune entity(ies), of the cell or cell component or at least one of its variation preferentially during at least one step of the method.

In another embodiment of the invention, the nanoparticle and/or cell or entity preferentially polarizable, un-polarizable, polarized, differentiable, un-differentiable and/or differentiated is/are detected, imaged, identified, assembled, interact, preferentially during at least one step of the method.

In another embodiment, at least one change of at least one property or state or state of polarization of cell or entity preferentially polarizable, un-polarizable, polarized, differentiable, un-differentiable and/or differentiated or polar is repeated more than once, occurs mor than once, and/or is carried out at more than two different time points, preferentially before, during or after at least one step of the method according to the invention.

In one embodiment, the state of the cell or entity is selected in the group consisting of state of: un-polarization, polarization, undifferentiation and differentiation.

In another embodiment of the invention, the cell or entity is selected in the group consisting of: polarizable, un-polarizable, polarized, un-polarized, differentiable, un-differentiable, differentiated and un-differentiated cell.

In another embodiment, at least one state of the cell is detected, imaged, identified, measured, triggered, activated, and/or de-activated, preferentially by determining if the cell or entity has changed its state and/or by triggering, activating, and/or de-activating the change of state of the cell, preferentially before, during or following at least one step of the method and/or by determining, activating, de-activating and/or triggering the change (or not) of at least one state of the cell or entity.

In one embodiment of the invention, at least one step of the method is stopped or interrupted, preferentially if or after or when the outcome of at least one step of the method is that at least one state of the cell has changed.

In another embodiment of the invention, at least one step of the method is followed by or results in the continuation of at least one step of the method, preferentially if or when the outcome or after at least one state of the method has not changed.

The invention also relates to the method according to the invention, wherein the radiation is selected in the group consisting of: i) an electromagnetic radiation, ii) a magnetic field, iii) an electric field, iv) an alternating electric or magnetic field, v) a laser or laser light, vi) a lamp or light produced by a lamp, vii) light emitted at a single wavelength, viii) light emitted at multiple wavelengths, ix) a ionizing radiation, x) microwave, xi) radiofrequency, xii) a photon, and xiii) alpha, beta, gamma, X-ray, neutron, proton, electron, ion, neutrino, muon, meson, photon particles or radiation, xiv) radiation of particles, substances, or photons originating from an atom or a molecule, and xv) an acoustic wave, an ultrasound, a hyper sound, or an infrasound.

The invention also relates to the method according to the invention, wherein the radiation has at least one property selected in the group consisting of: i) the intensity or power or power density, ii) the frequency or wavelength, and iii) the duration of application of the radiation.

In some cases, the radiation can be produced or emitted by an equipment that comprises a power supply, an actuator, a transistor, a transductor, an ultrasound or acoustic wave system, a laser, a light, a magnet, an induction system, a cryo-system, a radiotherapy equipment, a scanner, an MRI, a system that converts one type of radiation such as an electric signal into another type of radiation such as an acoustic wave, light, magnetic field, a system that transports or focalizes the radiation such as an optical fiber, a segment, a spire, a conductor, a microscope, an objective, preferentially in the body part.

In some cases, the radiation can have an intensity, power, or power density that is/are preferentially lower than:
10¹⁰⁰, 10⁵⁰, 10²⁰, 10¹⁰, 10⁹, 10⁶, 10⁵, 10³, 10², 10², 100, 50, 10, 5, 1, 10⁻¹ 10⁻², 10⁻³, 10⁻⁵ or 10⁻⁷ W (Watt), 10¹⁰⁰, 10⁵⁰, 10²⁰, 10¹⁰, 10⁹, 10⁶, 10⁵, 10³, 10², 100, 50, 10, 5, 3, 1, 10⁻¹ 10⁻², 10⁻³, 10⁻⁵ or 10⁻⁷ W per cm, W per cm², or W per cm³,
10¹⁰⁰, 10⁵⁰, 10²⁰, 10¹⁰, 10⁹, 10⁶, 10⁵, 10³, 10², 100, 50, 10, 5, 3, 1, 10⁻¹, 10⁻², 10⁻³, 10⁻⁵ or 10⁻⁷ W per cm of body part, W per cm² of body part, or W per cm³ of body part,
10¹⁰⁰, 10⁵⁰, 10²⁰, 10¹⁰, 10⁹, 10⁶, 10⁵, 10³, 10², 100, 50, 10, 5, 3, 1, 10⁻¹, 10⁻², 10⁻³, 10⁻⁵ or 10⁻⁷ W per cm of piece of equipment generating or emitting the radiation, W par cm² of piece of equipment generating or emitting the radiation, or W per cm³ of piece of equipment generating or emitting the radiation, and/or 10¹⁰⁰, 10⁵⁰, 10²⁰, 10¹⁰, 10⁹, 10⁶, 10⁵, 10³, 10², 100, 50, 10, 5, 3, 1, 10⁻¹, 10⁻², 10⁻³, 10⁻⁵ or 10⁻⁷ W per gram of nanoparticle or cell or entity.

In some cases, 1 cm³, 1 cm², and 1cm can be 1 cm³ of body part, 1 cm² of body part, and 1 cm of body part, respectively.

In some other cases 1 cm³, 1 cm², and 1 cm can be 1 cm³ of piece of equipment generating or emitting the radiation, 1 cm² of piece of equipment generating or emitting the radiation, and 1 cm of piece of equipment generating or emitting the radiation, respectively.

In some other cases, 1 gram of nanoparticles can be 1 gram of nanoparticles exposed to the radiation or 1 gram of nanoparticles onto which the radiation is applied.

In some other cases, the radiation can have an intensity, power, or power density that is/are larger than:
i) 10⁻¹⁰⁰, 10⁻⁵⁰, 10⁻²⁰, 10⁻¹⁰, 10⁻⁷, 10⁻⁵, 10⁻³, 10⁻², 10⁻¹, 1, 5, 10, 10², 10³, 10⁵, 10⁷ or 10⁹ W (Watt),
ii) 10⁻¹⁰⁰, 10⁻⁵⁰, 10⁻²⁰, 10⁻¹⁰, 10⁻⁷, 10⁻⁵, 10⁻³, 10⁻², 10⁻¹, 1, 5, 10, 10², 10³, 10⁵, 10⁷ or 10⁹ W per cm, W per cm², or W per cm³,
iii) 10⁻¹⁰⁰, 10⁻⁵⁰ 10⁻²⁰, 10⁻¹⁰, 10⁻⁷, 10⁻⁵, 10⁻³, 10⁻², 10⁻¹, 1, 5, 10, 10², 10³, 10⁵, 10⁷ or 10⁹ W per cm of body part, W per cm² of body part, or W per cm³ of body part,
iv) 10⁻¹⁰⁰, 10⁻⁵⁰, 10⁻²⁰, 10⁻¹⁰, 10⁻⁷, 10⁻⁵, 10⁻³, 10⁻², 10⁻¹, 1, 5, 10, 10², 10³, 10⁵, 10⁷ or 10⁹ W per cm of piece of equipment generating or emitting the radiation, W per cm² of piece of equipment generating or emitting the radiation, or W per cm³ of piece of equipment generating or emitting the radiation, and/or
v) 10⁻¹⁰⁰, 10⁻⁵⁰, 10⁻²⁰, 10⁻¹⁰, 10⁻⁷, 10⁵, 10³, 10⁻², 10⁻¹, 1, 5, 10, 10², 10³, 10⁵, 10⁷ or 10⁹ W per gram of nanoparticle or cell or entity.

In some other cases, the radiation can have an intensity, power, or power density that is between:
0 and 10¹⁰⁰, 10⁻¹⁰⁰ and 10¹⁰⁰, 10⁻¹⁰ and 10¹⁰, 10⁻⁵ and 10⁵, 10⁻³ and 10³, or between 10⁻¹ and 10 W (Watt), 0 and 10¹⁰⁰, 10⁻¹⁰⁰ and 10¹⁰⁰, 10⁻¹⁰ and 10¹⁰, 10⁻⁵ and 10⁵, 10⁻³ and 10³, or between 10⁻¹ and 10 W per cm, W per cm², or W per cm³,
0 and 10¹⁰⁰, 10⁻¹⁰⁰ and 10¹⁰⁰, 10⁻¹⁰ and 10¹⁰, 10⁻⁵ and 10⁵, 10⁻³ and 10³, or between 10⁻¹ and 10 W per cm of body part, W per cm² of body part, or W per cm³ of body part,
0 and 10¹⁰⁰, 10⁻¹⁰⁰ and 10¹⁰⁰, 10⁻¹⁰ and 10¹⁰, 10⁻⁵ and 10⁵, 10⁻³ and 10³, or between 10⁻¹ and 10 W per cm of piece of equipment generating or emitting the radiation, W per cm2 of piece of equipment generating or emitting the radiation, or W per cm³ of piece of equipment generating or emitting the radiation, and/or
0 and 10¹⁰⁰, 10⁻¹⁰⁰ and 10¹⁰⁰, 10⁻¹⁰ and 10¹⁰, 10⁻⁵ and 10⁵, 10⁻³ and 10³, or between 10⁻¹ and 10 W per gram of nanoparticle or cell or entity.

The invention also relates to nanoparticles for use according to the invention, wherein the energy or energy density of the radiation is lower than:
i) 10⁹, 10⁶, 10⁵, 10³, 100, 10, 1, 10⁻¹, 10⁻², or 10⁻⁵ W.sec,
ii) 10⁹, 10⁶, 10⁵, 10³, 100, 10, 1, 10⁻¹, 10⁻², or 10⁻⁵ W.sec/cm, W.sec/cm², or W.sec/cm³,
iii) 10⁹, 10⁶, 10⁵, 10³, 100, 10, 1, 10⁻¹, 10⁻², or 10⁻⁵ W.sec per cm of body part, W.sec per cm² of body part, or W.sec per cm³ of body part,
iv) 10⁹, 10⁶, 10⁵, 10³, 100, 10, 1, 10⁻¹, 10⁻², or 10⁻⁵ W.sec per cm of piece of equipment generating or emitting the radiation, W.sec per cm² of cell or polarizable or polarized cell, or W.sec per cm³ of cell or entity or polarizable or polarized cell or entity,
v) 10⁹, 10⁶, 10⁵, 10³, 100, 10, 1, 10⁻¹, 10⁻², or 10⁻⁵ J (Joule)
vi) 10⁹, 10⁶, 10⁵, 10³, 100, 10, 1, 10⁻¹, 10⁻², or 10⁻⁵ J per cm, J per cm², or J per cm³,
vii) 10⁹, 10⁶, 10⁵, 10³, 100, 10, 1, 10⁻¹, 10⁻², or 10⁻⁵ J per cm of body part, J per cm² of body part, or J per cm³ of body part,
viii) 10⁹, 10⁶, 10⁵, 10³, 100, 10, 1, 10⁻¹, 10⁻², or 10⁻⁵ J per cm of piece of equipment generating or emitting the radiation, J per cm² of piece of equipment generating or emitting the radiation, or J per cm³ ₒf piece of equipment generating or emitting the radiation, and/or
ix) 10⁹, 10⁶, 10⁵, 10³, 100, 10, 1, 10⁻¹, 10⁻², or 10⁻⁵ J per gram of nanoparticle or cell or entity, In some cases, the radiation can be applied for a shorter time than 24, 12, 6, 3, 2, or 1 hour, 50, 30, 15, 10, 5, 2, or 1 minute(s), or 50, 30, 20, 10, 5, 2, 1, 10⁻¹, 10⁻³, 10⁻⁶ or 10⁻⁹ second(s).

In some cases, the radiation can be applied for a longer time than 24, 12, 6, 3, 2, or 1 hour, 50, 30, 15, 10, 5, 2, or 1 minute(s), or 50, 30, 20, 10, 5, 2, 1, 10⁻¹, 10⁻³, 10⁻⁶ or 10⁻⁹ second(s).

The invention also relates to nanoparticles for use according to the invention, wherein the energy or energy density of the radiation is larger than:
i) 10⁻⁵⁰, 10⁻¹⁰, 10⁻⁵, 10⁻³, 10⁻¹, 1, 10, 10², or 10⁵ W.sec,
ii) 10⁻⁵⁰, 10⁻¹⁰, 10⁻⁵, 10⁻³, 10⁻¹, 1, 10, 10², or 10⁵ W.sec/cm, W.sec/cm², W.sec/cm³,
iii) 10⁻⁵⁰, 10⁻¹⁰, 10⁻⁵, 10⁻³, 10⁻¹, 1, 10, 10², or 10⁵ W.sec per cm of body part, W.sec per cm² of body part, or W.sec per cm³ of body part,
iv) 10⁹, 10⁻¹⁰, 10⁻⁵, 10⁻³, 10⁻¹, 1, 10, 10², or 10⁵ W.sec per cm of piece of equipment generating or emitting the radiation, W.sec per cm² of piece of equipment generating or emitting the radiation, or W.sec per cm³ of piece of equipment generating or emitting the radiation,
v) 10⁹, 10⁻¹⁰, 10⁻⁵, 10⁻³, 10⁻¹, 1, 10, 10², or 10⁵ J (Joule),
vi) 10⁻⁵⁰, 10⁻¹⁰, 10⁻⁵, 10⁻³, 10⁻¹, 1, 10, 10², or 10⁵ J/cm, J/cm2 or J/cm3.
vii) 10⁻⁵⁰, 10⁻¹⁰, 10⁻⁵, 10⁻³, 10⁻¹, 1, 10, 10², or 10⁵ J per cm of body part, J per cm² of body part, or J per cm³ of body part,
viii) 10⁻⁵⁰, 10⁻¹⁰, 10⁻⁵, 10⁻³, 10⁻¹, 1, 10, 10², or 10⁵ J per cm of piece of equipment generating or emitting the radiation, J per cm² of piece of equipment generating or emitting the radiation, or J per cm³ of piece of equipment generating or emitting the radiation, or
ix) 10⁻⁵⁰, 10⁻¹⁰, 10⁻⁵, 10⁻³, 10⁻¹, 1, 10, 10², or 10⁵ J per gram of nanoparticle or cell or entity.

In some cases, the energy or energy density of the radiation is between:
i) 10-50 and 1050, 10-10 and 1010, 10-5 and 105, 10-1 and 105, or between 1 and 104 W.sec,
ii) 10-50 and 1050, 10-10 and 1010, 10-5 and 105, 10-1 and 105, or between 1 and 104 W.sec/cm, W.sec/cm2, W.sec/cm3,
iii) 10-50 and 1050, 10-10 and 1010, 10-5 and 105, 10-1 and 105, or between 1 and 104 W.sec per cm of body part, W.sec per cm2 of body part, or W.sec per cm3 of body part,
iv) 10⁻⁵⁰ and 10⁵⁰, 10⁻¹⁰ and 10¹⁰, 10⁻⁵ and 10⁵, 10⁻¹ and 10⁵, or between 1 and 10⁴ W.sec per cm of piece of equipment generating or emitting the radiation, W.sec per cm² of piece of equipment generating or emitting the radiation, or W.sec per cm³ of piece of equipment generating or emitting the radiation,
v) 10⁻⁵⁰ and 10⁵⁰, 10⁻¹⁰ and 10¹⁰, 10⁻⁵ and 10⁵, 10⁻¹ and 10⁵, or between 1 and 10⁴ J (Joule),
vi) 10⁻⁵⁰ and 10⁵⁰, 10⁻¹⁰ and 10¹⁰, 10⁻⁵ and 10⁵, 10⁻¹ and 10⁵, or between 1 and 10⁴ J/cm, J/cm² or J/cm³,
vii) 10⁻⁵⁰ and 10⁵⁰, 10⁻¹⁰ and 10¹⁰, 10⁻⁵ and 10⁵, 10⁻¹ and 10⁵, or between 1 and 10⁴ J per cm of body part, J per cm² of body part, or J per cm³ of body part,
viii) 10⁻⁵⁰ and 10⁵⁰, 10⁻¹⁰ and 10¹⁰, 10⁻⁵ and 10⁵, 10⁻¹ and 10⁵, or between 1 and 10⁴ J per cm of piece of equipment generating or emitting the radiation, J per cm² of piece of equipment generating or emitting the radiation, or J per cm³ of piece of equipment generating or emitting the radiation, and/or
ix) 10⁻⁵⁰ and 10⁵⁰, 10⁻¹⁰ and 10¹⁰, 10⁻⁵ and 10⁵, 10⁻¹ and 10⁵, or between 1 and 10⁴ J per gram of nanoparticle or cell or entity.

Preferentially, the radiation has a frequency or wavelength that is preferentially lower than 10⁵ MHz or nm. In some cases, the frequency of the acoustic wave or radiation can be lower than 10¹⁰⁰, 10⁵⁰, 10³⁰, 10²⁰, 10¹⁰, 10⁵, 10³, 10², 10, 1, 10⁻¹, 10⁻², 10⁻³, 10⁻⁴, 10⁻⁵, 10⁻⁶, 10⁻⁷, 10⁻⁸, 10⁻¹⁰ or 10⁻¹⁵ MHz or kHz or nm. In some other cases, the radiation has a frequency or wavelength that is larger than 10⁻¹⁰⁰, 10⁻⁵⁰, 10⁻²⁰, 10⁻¹⁰, 10⁻⁸, 10⁻⁷, 10⁻⁶, 10⁻⁵, 10⁻⁴, 10⁻³, 10⁻², 10⁻¹, 1, 10, 10³, 10⁵ or 10¹⁰ kHz or MHz or nm.

In still some other cases, the radiation can have a frequency or wavelength between 10⁻¹⁵ and 10¹⁵ MHz or nm, 10⁻¹³ and 10¹³ MHz or nm, 10⁻¹¹ and 10¹¹ MHz or nm, 10⁻⁹ and 10⁹ MHz or nm, 10⁻⁷ and 10⁷ MHz or nm, 10⁻⁵ and 10⁵ MHz or nm, 10⁻³ and 10³ MHz or nm, or between 10⁻¹ and 10 MHz or nm.

In some cases, the frequency or wavelength of the radiation can be the frequency or wavelength of oscillation of the radiation.

The invention also relates to nanoparticles and/or cell(s) or entity, preferentially polarizable and/or polarized, preferentially for use in the method according to the invention.

In some cases, the radiation or nanoparticle(s) or cell(s) or entity can have a penetration depth, preferentially in the body part or individual, which is preferentially larger than 10⁻¹⁰, 10⁻⁵, 10⁻³, 10⁻¹, 1, 5, 10, 20, 50 or 500 cm.

In some other cases, the radiation or nanoparticle(s) or cell(s) or entity can have a penetration depth, preferentially in the body part or individual, which is lower than 10¹⁰, 10⁵, 10³, 50, 50, 20, 10, 5, 1, 0.1 or 0.001 cm.

In still some other cases, the radiation or nanoparticle(s) or cell(s) or entity can have a penetration depth, preferentially in the body part or individual, which is between 10⁻¹⁰ and 10¹⁰, 10⁻⁵ and 10³, 10⁻³ and 10³, 10⁻³ and 100, 10⁻² and 10, or between 10⁻² and 10 cm.

Preferentially, the penetration depth of the radiation or nanoparticle(s) or cell(s) or entity is the depth of penetration of the radiation through or in the body part or is the depth at which: i) the radiation, nanoparticle(s) or cell(s) or entity is located, or ii) the region comprising the radiation or nanoparticle(s) or cell(s) or entity is located, or iii) 99, 75, 50, 25, 1, 0.1, 10⁻³, 10⁻⁹ or 10⁻²⁰ % of the body part or region comprising the radiation or nanoparticle(s) or cell(s) or entity is located.

Preferentially, the penetration depth of the radiation is the depth of the region separating the equipment generating the radiation and the body part, cell(s) or entity and/or nanoparticle(s) preferentially exposed or receiving radiation.

The invention also relates to the method according to the invention, wherein the cell or entity or polarizable cell or entity has at least one of its polarization states that is changed by changing, varying, increasing or decreasing at least one property of:
the radiation, preferentially selected in the group consisting of: i) the intensity or power or power density, ii) the frequency or wavelength, iii) the penetration depth, and iv) the duration of application of the radiation, v) the number of times that the radiation is applied;
the nanoparticle, preferentially selected in the group consisting of: the type, number, concentration, charge, surface charge, composition, type of assembly, coating or core type or composition, magnetic property, metallic and/or carbonaceous composition, apyrogenicity, sterility, stability, type of interaction or binding with the at least one cell or polarizable cell, location of the at least one nanoparticle, preferentially inside or outside the least one cell or entity or polarizable cell or entity or cell or entity component.

The invention also relates to the method according to the invention, wherein the cell, preferentially cell, consists of or comprises the whole or part of such cell or entity or a cell or entity component or organelle, preferentially polarized or preferentially triggering the polarization of the cell.

The invention also relates to the method according to the invention, wherein the radiation is selected among:
A heating radiation, preferentially a radiation that produces heat, preferentially above the physiological temperature, preferentially at the site or in the environment of the nanoparticle location or body part,
A cooling radiation, preferentially a radiation that produces cold, preferentially below the physiological temperature, preferentially at the site or in the environment of the nanoparticle location or body part,
An ionizing radiation, preferentially a radiation that produces ionization or radical species, preferentially below the physiological temperature, preferentially at the site or in the environment of the nanoparticle location or body part,
wherein preferentially the radiation is sufficiently intense or induces a sufficiently large amount of heat, cold or radical species to trigger at least one perturbation or to activate or change the state of at least one cell or entity or polarizable cell or entity;
wherein preferentially the radiation is sufficiently mild or induces a sufficiently moderate amount of heat, cold or radical species to avoid the destruction or inactivation or change of state of at least one cell or entity or polarizable cell or entity;
In some cases, the radiation preferentially applied on the nanoparticle and/or cell or entity or polarizable cell or entity is sufficiently intense or induces a sufficiently large amount of heat, cold or radical species to trigger at least one perturbation or to activate at least one cell or entity or polarizable cell or entity or to change at least one state preferentially of polarization of the at least one cell or entity or polarizable cell or entity, preferentially when its intensity or power or power density is larger than 0, 0.001, 0.1, 1, 10 or 100 W or Gy or the amount of heat or cold or radical species or gradient of it/them that it produce is larger than 0, 0001, 0.1, 10 or 100 °C preferentially in absolute values or 0, 1, 10, 10⁵ radical species preferentially per m³, m², mm, mm³, mm², or mm, preferentially of cell(s), body part, nanoparticle(s) and/or cell(s) or entity, piece(s) of equipment generating the radiation.

In some other cases, the radiation preferentially applied on the nanoparticle and/or cell or entity or polarizable cell or entity is sufficiently mild or induces a sufficiently moderate amount of heat, cold or radical species or gradient of it/them preferentially to avoid the destruction or inactivation of at least one cell or entity or polarizable cell or entity or to prevent the absence of change at least one state or state of polarization of the at least one cell or entity or polarizable cell or entity, preferentially when its intensity or power or power density is smaller than 10¹⁰, 10⁵, 100, 10, 1 or 10⁻³ W or Gy or the amount of heat or cold or radical species or gradient of it/them that it produce is smaller than 10⁵, 10³, 100, 50, 20, 10, 5, 1, 0 or 0001 °C preferentially in absolute values or 10¹⁰, 10⁵, 10³, 10, 5, 2, 1 or 0 radical species preferentially per m³, m², mm, mm³, mm², or mm, preferentially of cell(s) or entity, body part, nanoparticle(s), piece(s) of equipment generating the radiation.

In some cases, the physico-chemical disturbance preferentially applied on the nanoparticle and/or cell or entity or polarizable cell or entity is sufficiently intense or induces a sufficiently large amount of disturbance preferentially of the nanoparticle and/or cell or entity or polarizable/polarized cell or entity and/or its/their environment to trigger at least one perturbation or to activate at least one cell or entity or polarizable cell or entity or to change at least one state or state of polarization of the at least one cell or entity or polarizable cell or entity, preferentially when the pH, oxydo-reduction potential, substance concentration, or its/their variation(s) is smaller than 10¹⁰, 14, 5, 2 or 0 pH unit, mV, substance(s) preferentially per cm³ or mm³ or nm³ preferentially of preferentially nanoparticle(s), body part, cell(s), or entity, and/or its/their environment.

In some other cases, the radiation preferentially applied on the nanoparticle and/or cell or entity or polarizable cell or entity is sufficiently mild or induces a sufficiently moderate amount of disturbance, preferentially to avoid the destruction or inaction of at least one cell or entity or polarizable cell or entity or to prevent the absence of change at least one state or state of polarization of the at least one cell or polarizable cell or entity, preferentially when the pH, oxydo-reduction potential, substance concentration, or its/their variation(s) is larger than 10⁻¹⁰, 0, 1 or 10 pH unit, mV, substance(s) preferentially cm³ or mm³ or nm³ preferentially of preferentially nanoparticle(s), body part, cell(s), and/or its/their environment.

In some cases, the physico-chemical disturbance and/or radiation is/are applied at the site or in the environment of the nanoparticle, body part, and/or cell or entity.

The invention also relates to the method according to the invention, wherein the cell or entity or polarizable cell or entity occupies at least one state preferentially of polarization, preferentially selected from the group consisting of: i) an undifferentiated state, ii) a differentiated state, iii) au unpolarized state, iv) a first state or state of polarization, and v) and a second state or state of polarization, wherein the cell or polarizable cell changes its state preferentially of polarization by undergoing a perturbation or physico-chemical disturbance, which is preferentially due to its interaction with at least one nanoparticle preferentially under the application of a radiation.

The invention also relates to the method according to the invention, wherein the cell or entity or polarizable cell or entity occupies or is in or comprises at least one state preferentially of polarization, preferentially selected from the group consisting of: i) an undifferentiated state, ii) a differentiated state, iii) au unpolarized state, iv) a first state or state of polarization, and v) and a second state or state of polarization,
wherein preferentially the polarizable cell or entity changes its state or state of polarization preferentially following the application of or resulting from the application of a physico-chemical disturbance and/or radiation on at least one nanoparticle and/or cell or entity or polarizable cell or entity.

The invention also relates to the method according to the invention, wherein the cell or polarizable cell or entity changes its state or state of polarization, preferentially by undergoing at least one or two perturbation(s) preferentially type(s) of perturbation(s), preferentially selected in the group consisting of:
A first perturbation consisting in at least one association of at least one cell or entity or polarizable cell or entity with at least one nanoparticle without or before the application of a physico-chemical disturbance and/or radiation;
A second perturbation consisting in at least one association of at least one cell or entity or polarizable cell or entity with at least one nanoparticle under or followed by the application of a physico-chemical disturbance and/or radiation;
A third perturbation consisting in the application of a radiation and/or physico-chemical disturbance on at least one cell or entity or polarizable cell or entity without at least one association of the at least one nanoparticle with at least one cell or entity or polarizable cell or entity; and
A fourth perturbation consisting in the application of a radiation and/or physico-chemical disturbance on at least one cell or entity or polarizable cell or entity under or followed by the association of the at least one nanoparticle with at least one cell or entity or polarizable cell or entity;
In some cases, the at least one perturbation or radiation or physico-chemical disturbance is applied sequentially in time, i.e. that preferentially the at least one nanoparticle is either first associated with or interacts first with the at least one cell or entity or polarizable cell or entity, and the radiation and/or physico-chemical disturbance is then applied on the at least one nanoparticle and/or at least one cell or entity or polarizable cell or entity or the radiation and/or physico-chemical disturbance is first applied on the at least one cell or entity or polarizable cell or entity and the at least one cell or entity or polarizable cell or entity is then associated with or interacting with the at least one nanoparticle;
wherein preferentially the at least one perturbation is applied sequentially in space, i.e. that preferentially the at least one nanoparticle is associated with the at least one cell or entity or polarizable cell or entity within or in a first region or location and the radiation and/or physico-chemical disturbance is applied on the at least one nanoparticle and/or the at least one cell or entity or polarizable cell or entity within or in a second region or location;
wherein preferentially the association of the nanoparticle with the cell or entity or polarizable cell or entity preferentially means that the cell or polarizable cell or at least one of its components comprises or is associated with or embeds or internalizes or expels or produces or engulfs or degrades or interacts with or diffuses at least one nanoparticle, where the at least one nanoparticle is preferentially located or in interaction with at least one component or organelle of the at least one cell or entity, preferentially before, during or after the radiation and/or physico-chemical disturbance has/have been applied on the cell or entity and/or nanoparticle;
In some cases, the at least one perturbation results in : i) the change of polarization from at least one first polarization state to at least one second state or state of polarization of the cell or entity or polarizable cell or entity or ii) two different states of polarization.

In some other cases, the at least two different perturbations result in at least three states of polarization of the at least one cell or cell or entity or polarizable cell or entity;
In still some other cases, the at least one perturbation results in or is associated with: i) the activation or production of the immune system or at least one or one type or category of immune entity, and/or ii) the de-activation or absence of production of the immune system or at least one or one type or category of immune entity.

In some cases, the at least one, two, three state(s) of polarization is/are reached or obtained concomitantly, simultaneously, or separately in time and/or space.

In some cases, the method involves and/or the nanoparticle and/or cell or entity and/or body part and/or at least one of its/their component(s) is exposed to or undergoes a radiation and/or physico-chemical disturbance, preferentially changing the state or state or state of polarization of the cell or cell or cell or and/or at least one property of the nanoparticle and/or cell or entity.

In some other cases, a radiation and/or physico-chemical disturbance is applied on the nanoparticle and/or cell or entity and/or body part and/or at least one of its/their component(s), preferentially changing the state or state of polarization of the cell or polarizable cell and/or at least one property of the nanoparticle and/or cell or entity.

In one embodiment of the invention, the physico-chemical disturbance applied on the nanoparticle and/or cell or entity and/or body part and/or at least one of its/their component(s) according to the invention is due to or associated with a variation of the environment of the nanoparticle and/or cell and/or body part.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a pH variation preferentially of less than 14, 13, 12, 11, 10, 9, 8, 7, 6, 5, 4, 3, 2, 1, 0.5, 0.2, 0.1, 0.01, 0.005, 0.001, 10⁻⁴, 10⁻⁵, 10⁻⁶, 10⁻⁷, 10⁻⁸, 10⁻⁹, 10⁻¹¹, 10⁻¹¹, 10⁻¹², or 10⁻¹³ pH units preferentially of that environment or at least one substance comprised in that environment.

In one embodiment of the invention, the physico-chemical disturbance applied on the nanoparticle and/or cell or entity and/or body part and/or at least one of its/their component(s) or the variation of the environment of the nanoparticle and/or cell or entity and/or body part and/or at least one of its/their component(s) according to the invention is a pH variation preferentially of more than 14, 13, 12, 11, 10, 9, 8, 7, 6, 5, 4, 3, 2, 1, 0.5, 0.2, 0.1, 0.01, 0.005, 0.001, 10⁻⁴, 10⁻⁵, 10⁻⁶, 10⁻⁷, 10⁻⁸, 10⁻⁹, 10⁻¹⁰, 10⁻¹¹, 10⁻¹², or 10⁻¹³ pH units preferentially of that environment or at least one substance comprised in that environment.

In one embodiment of the invention, the physico-chemical disturbance applied on the particle or the variation of the environment of the nanoparticle and/or cell or entity and/or body part and/or at least one of its/their component(s) according to the invention is a temperature variation preferentially of less than 10¹⁰, 10⁵, 100, 100, 1, 0, 0,1, 0,01 or 10⁻⁵ °C of this environment or at least one substance comprised in this environment.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a temperature variation of more than 10¹⁰, 10⁵, 100, 10, 5, 1, 0.1, 0.01, 0.001, or 10⁻²⁰ °C of this environment or at least one substance comprised in this environment.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a standard potential variation of less than 10³, 100, 10, 5, 2, 1, 0, 0.5, 0.1, 0.01, 10⁻⁵ or 10⁻²⁰ Volt of this environment or at least one substance comprised in this environment.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a standard potential variation of less than 10²⁰, 10³, 100, 10, 5, 1, 0, 0.5, 0.001, or 10⁻¹³ Volt of this environment or at least one substance comprised in this environment.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a variation of the concentration of at least one substance of this environment of more of 10¹⁰, 10⁵, 100, 5, 1, 0, 0.1, 10⁻⁵ or 10⁻²⁰ mole per liter, micromole per liter, nano-mole per liter, mole per milliliter, micromole per milliliter, nano-mole per milliliter, mole per cubic meter, mole per cubic decimeter, mole per cubic centimeter, or mole per cubic millimeter.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a variation in the chemical composition of at least one substance of this environment of less than of 10¹⁰, 10⁵, 100, 10, 5, 1, 0, 0.1, 0.01, 10⁻⁵ or 10⁻²⁰ mole per liter, micromole per liter, nano-mole per liter, mole per milliliter, micromole per milliliter, nano-mole per milliliter, mole per cubic meter, mole per cubic decimeter, mole per cubic centimeter, or mole per cubic millimeter.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is a modification of at least 1, 2, 5, 100, 500 or 10¹⁰ substance(s) in this environment where this modification may be a chemical or structural modification and/or the appearance or disappearance of substance(s) from that environment.

In one embodiment of the invention, the physico-chemical disturbance applied on the nanoparticle and/or cell or entity and/or body part and/or at least one of its/their component(s) or the variation of the environment of the nanoparticle and/or cell and/or body part and/or at least one of its/their component(s) according to the invention is the variation of chemical composition of less than 1, 2, 5, 10, 100 or 10¹⁰ substances in this environment, where this variation may be a chemical or structural modification and/or the appearance or disappearance of substance(s) in that environment.

In one embodiment of the invention, the physico-chemical disturbance applied on the nanoparticle and/or cell or entity and/or body part and/or at least one of its/their component(s) induces a modification of the condition of the nanoparticle and/or cell and/or body part and/or at least one of its/their component(s) that is preferentially selected in the group consisting of: (i) an increase or decrease in pH of the nanoparticle and/or cell or entity and/or body part and/or at least one of its/their component(s) of more or less than 10⁵, 14, 5, 1, 0.001 or 10⁻¹³ pH units, (ii), a temperature increase or decrease of the nanoparticle and/or cell or entity and/or body part and/or at least one of its/their component(s) of more or less than 10¹⁰, 100, 10, 5, 1, 0, 0.1, 0.001 or 10⁻¹³ °C, (iii), an increase or decrease in charge of the particle of more or less than 10³, 100, 10, 5, 2, 1, 0, 0.001 or 10⁻¹⁰ Volt, and (iv), an increase or decrease in the number of atoms or components or ions comprised in the nanoparticle and/or cell or entity and/or body part and/or at least one of its/their component(s) of more or less than 10¹⁰⁰, 10¹⁰, 10³, 10, 5, 1 or 0 atom(s) or component(s) or ion(s).

In some cases, at least one substance in or of the environment of the nanoparticle and/or cell or entity and/or body part is the nanoparticle and/or cell or entity and/or body part.

In some other cases, at least one substance in or of the environment of the nanoparticle and/or cell or entity and/or body part is a substance that is different from, released from, expelled from, activated or inactivated by, diffusing from or between and/or produced by the at least one or two nanoparticle(s) and/or cell(s) or entity and/or body part(s) and/or at least one or two of its/their component(s).

In still some other cases, the environment of the nanoparticle and/or cell or entity and/or body part is/are the region, location, volume, surface, length embedding, surrounding, and/or comprising the nanoparticle and/or cell or entity and/or body part, preferentially with a diameter or size that is in some cases smaller than 0 or 1 km, m, cm, mm, µm or nm or than the size of body part or individual, preferentially with a diameter or size that is in some other cases larger than 0 or 1 km, m, cm, mm, µm or nm or than the size of body part or individual.

In some cases, the cell or entity can be a cell selected in the group consisting of: a cell or entity or polarizable cell or entity, a polarized cell or entity preferentially in at least one state or state of polarization, a healthy cell or entity, a pathological cell or entity, an immune cell or entity, a pro-inflammatory cell or entity, an anti-inflammatory cell or entity, a macrophage, M0, M1, M2, an un-differentiated cell or entity, a differentiated cell or entity, a stem cell or entity, a mast cell, a cell or entity acting against or inactivating or eliminating a pathological site or body part or tumor, a cell or entity acting against or inactivating or eliminating a healthy site or body part, a cell or entity protecting or activating or maintaining alive or active a pathological site or body part or tumor, a cell or entity protecting or activating or maintaining alive or active a healthy site or body part, and at least one component of at least one of this or these cell(s).

The invention also relates to the method according to the invention, wherein the cell or entity or polarizable cell or entity is a cell or entity, preferentially an immune cell or entity, most preferentially a macrophage, that can preferentially be in at least 1, 2, 3 or 4 different state(s) of non-polarization(s) or polarization(s);

The invention also relates to the method according to the invention, wherein the cell or entity or polarizable cell or entity comprises or is associated with or embeds or internalizes or expels or produces or engulfs or degrades at least one nanoparticle, where the at least one nanoparticle is preferentially located or in interaction with the cell or entity or polarizable cell or entity or at least one component or organelle of this cell or entity, wherein the polarized cell or entity is preferentially unpolarized when the cell or entity or polarizable cell or entity does not comprise or is not associated with or does not embed or does not internalize or expel or produce or engulf or degrade at least one nanoparticle, wherein the polarized cell or entity is preferentially polarized when the polarizable cell or entity does comprise or is associated with or embeds or internalizes or expels or produces or engulfs or degrades at least one nanoparticle, wherein the polarized cell or entity is preferentially in a different state or state of polarization when it is exposed to radiation than when it is not exposed to radiation or when it is exposed to a radiation with a first set of parameters than when it is exposed to a radiation with a second set of parameters.

The invention also relates to the method according to the invention, wherein the cell or entity or polarizable cell or entity comprises or is associated with or embeds or internalizes or expels or produces or engulfs or degrades at least one nanoparticle, where the at least one nanoparticle is preferentially located is or is in interaction with at least one component or organelle of the at least one cell or entity. The invention relates to the method according to the invention, wherein the change from at least one polarization state to at least one other polarization state of the at least one cell or entity or polarizable cell or entity results from the application of at least one radiation on at least one nanoparticle, preferentially without inactivating or destroying at least one cell or entity or polarizable cell or entity. In one embodiment of the invention, the cell or polarizable cell or entity is in at least one state or state of polarization, wherein a cell or entity in this state has at least one property selected in the group consisting of:
it produces, comprises, is associated with, originates from, is differentiated or polarized or un-differentiated or un-polarized by, and/or activates at least one immune entity of first or second category, cytokine, interleukin, part of a biological pathway,
it is not undergoing, preferentially predominantly or partly, at least one type of death or destruction or inactivation preferentially selected in the group selected in the group consisting of:
   a necrotic death,
   an apoptotic death,
   a ferroptosis death,
   a direct death or destruction or inactivation, where a direct death or destruction or inactivation preferentially corresponds to a death occurring immediately or less than 100, 10, 5, 2, 1 year, 1 day, 1 hour, 1 minute or 1 second following the association of the cell or polarizable cell with at least one nanoparticle and/or the application of radiation and/or without the mediation or the intervention of another entity, preferentially carrying a death triggering message, an indirect death or destruction or inactivation, where an indirect death or destruction or inactivation preferentially corresponds to a death occurring with a time delay or more than 10⁻⁹, 10⁻³, 0, 1, 10, or 10³ second(s) following the association of the cell or polarizable cell with at least one nanoparticle and/or the application of radiation,
   it has a movement having at least one first property selected in the group consisting of:
      it is orientated in the direction of at least one cell or pathological or healthy cell;
      It is triggered by the application of a radiation or physico-chemical disturbance or perturbation, preferentially an external radiation, preferentially a magnetic field, on the nanoparticle(s) and/or cell(s);
      It is larger than 0, or 1 nm or cm per sec, min, hour, day or blood flow preferentially in the body part to be treated;
      it is orientated in the direction of at least one cell or entity or pathological or healthy cell;
      it has a movement having at least one second property selected in the group consisting of:
         it is not orientated in the direction of at least one cell or entity or pathological or healthy cell;
         It is not triggered by the application of a radiation or physico-chemical disturbance or perturbation, preferentially an external radiation, preferentially a magnetic field, on the nanoparticle(s) and/or cell(s) or entity;
         It is smaller than 10⁵, 10³, 10, 1 or 0 nm or cm per sec, min, hour, day or blood flow preferentially in the body part to be treated;
         it is not orientated in the direction of at least one cell or entity or pathological or healthy cell;
         it has at least one dimension or comprises at least one of its components or organelles, which has at least one dimension, preferentially selected among its width, diameter, thickness, height, length, that is smaller than 1000, 100, 10, 5, 2, 1, 10⁻¹, 10⁻² or 10⁻³ µm;
         it has at least one dimension or comprises at least one of its components or organelles, has at least one dimension, preferentially selected among its width, diameter, thickness, height, length, that is larger than 1000, 100, 10, 5, 2, 1, 10⁻¹, 10⁻² or 10⁻³ µm;
         it is in or comprises at least one phase selected in the group consisting of: prophase, metaphase, anaphase and telophase;
         it is a meiosis or mitosis state,
         it is the state of a stem cell or non-differentiate cell or entity or a cell or entity that does not have or does not belong to a specific organ or body part,
         it is the state of a cell or entity that is in a differentiated cell or entity or a cell or entity that has or belongs to a specific organ or body part or tissue or blood or plasma,
         it triggers or produces an immune response or entity(ies) that attract(s) or activate(s) at least one immune cell, preferentially acting against or inactivating at least one pathological cell or site or protecting or repairing or healing at least one healthy cell or site or entity,
         it triggers or activates an immune response or entity(ies) that attract(s) or activate(s) at least one immune cell, preferentially a helper cell or a dendritic cell or a B or T cell, that activates or produces another immune entity, preferentially an antibody, a major or minor histocompatibility complex or cell receptor, which either protects or activates at least one healthy cell or site or destroys or de-activates at least one pathological cell or site or entity, partly or fully.

The invention also relates to a drug or drug component or medical device or medical device component or preparation or composition or adjuvant or active principle or vaccine component or vaccine adjuvant comprising at least one cell or entity or polarized cell or entity, with or without at least one nanoparticle, which preferentially is or was or has been exposed (or not) to radiation, which preferentially has changed or changes its state or state of polarization, preferentially originating from the method according to the invention.

The invention also relates to the method for use or use of the nanoparticle(s) and/or cell(s) or entity preferentially for the treatment or detection of body part.

The invention also relates to a method to fabricate at least one cell or entity or polarizable cell or entity preferentially by following at least one step of the method according to the invention.

In one embodiment of the invention, the nanoparticle or cell or entity comprises or is selected from the group consisting of: a nanosphere, a nano-capsule, a dendrimer, a carbon nanotube, a lipid/solid nanoparticle, a lipid or protein or DNA or RNA based nanoparticle, a nanoparticle with an inner aqueous environment surrounded by a layer, preferentially a stabilizing layer, most preferentially a phospholipid layer, a multilayer nanoparticle, a polymeric nanoparticle, a quantum dot, a metallic nanoparticle, a polymeric micelle or nanoparticle, a carbon based nano-structure, a nanobubble, a nanosome, a pharmacyte, a niosome, a nanopore, a microbivore, a liposome, a virus, preferentially recombinant, a herbal nanoparticle, a magnetosome, a nanoparticle comprising at least one metallic or magnetic atom, a metallic nanoparticle, a magnetic nanoparticle, predominantly or not, a crystallized or amorphous nanoparticle, an antibody, and/or a vesicle.

In one embodiment of the invention, the nanoparticles and/or cell and/or entity has/ have or are characterized by at least one of the following properties: i) the presence of a core, preferentially magnetic, preferentially mineral, preferentially composed of a metal or metallic oxide such as iron oxide, most preferentially maghemite or magnetite, or an intermediate composition between maghemite and magnetite, ii) the presence of a coating that surrounds the core and preferentially prevents nanoparticle aggregation, preferentially enabling nanoparticle administration in an organism or in the body part or stabilizing the nanoparticle core, where coating thickness may preferably lie between 0.1 nm and 10 µm, between 0.1 nm and 1 µm, between 0.1 nm and 100 nm, between 0.1 nm and 10 nm, or between 1 nm and 5 nm, iii) magnetic properties leading to diamagnetic, paramagnetic, superparamagnetic, ferromagnetic, or ferrimagnetic behavior, iv) a coercivity larger than 0.01, 0.1, 1, 10, 100, 10³, 10⁴, 10⁵, 10⁹ or 10²⁰ Oe, v) a ratio between remanent and saturating magnetization larger than 0.01, 0.1, 0.2, 0.3, 0.4, 0.5, 0.75, 0.9 or 0.99, vi) a saturating magnetization larger than 0.1, 1, 5, 10 or 50 emu/g, vii) magnetic properties such as coercivity, remanent and saturating magnetization, preferentially measured or observed at a temperature larger than 0.1 K, 1 K, 10 K, 20 K, 50 K, 100 K, 200 K, 300 K, 350 K or 3000 K, viii) a crystallinity, i.e. nanoparticles preferentially possessing at least 1, 2, 5, 10 or 100 crystalline plane(s), preferentially observable or measured by electron microscopy, ix) the presence of a single domain, x) a size that is larger than 0.1, 0.5, 1.5, 10, 15, 20, 25, 30, 50, 60, 70, 80, 100, 120, 150 or 200 nm, xi) a size lying between 0.1 nm and 10 µm, between 0.1 nm 20 and 1 µm, between 0.1 nm and 100 nm, between 1 nm and 100 nm, or between 5 nm and 80 nm, xii) a non-pyrogenicity or apyrogenicity, which preferentially means that nanoparticles possess an endotoxin concentration lower than 1020, 10000, 1000, 100, 50, 10, 5, 2 or 1 EU (endotoxin unit) per mg of nanoparticle or per mg of iron comprised in nanoparticles, or which means that nanoparticles do not trigger fever or an increase in whole body temperature larger than 100, 50, 6.6, 5, 3, 2 or 1 °C following their administration to a living organism or body part, xiii) a synthesis by a synthetizing living organism, preferentially by bacteria, xiv) a chemical synthesis, xv) the presence of less than 50, 25, 15, 10, 5, 2 or 1% of organic or carbon material originating from the synthetizing living organism, xv), the presence of more than 99, 95, 80, 70, 60, 50 or 25% of mineral material originating from the synthetizing living organism, or xvi) a specific absorption rate (SAR) that is larger than 1, 10, 1000 or 104 Watt per gram of nanoparticle, preferentially measured under the application of an alternating magnetic field of strength preferentially larger than 0.1, 1, 10 or 100 mT, and/or frequency larger than 1, 10, 100 or 1000 KHz, alternatively preferentially measured under the application of the acoustic wave, alternatively under the application of a radiation such as an electromagnetic acoustic, or light radiation.

In some cases, the nanoparticle and/or cell and/or entity, preferentially polarized and/or polarizable cell or entity, is comprised, produced, formed, extracted from, introduced in, appears in or outside the body part, in the body part without being present or existing outside the body part or prior to the treatment or method according to the invention, or with being present or existing outside the body part or prior to the treatment or method according to the invention.

Preferentially, TH1 Dual monocyte are transformed into M0 macrophage, preferentially in the presence of PMA, most preferentially 5 ng per mL of PMA.

Preferentially, M0 macrophages are polarized into M1 macrophages in the presence of IFN and/or LPS. Preferentially, M1 macrophages preferentially produce or secrete pro-inflammatory cytokines, preferentially IL, IL-1β, IL-6, IL-6, IL-12, TNF and/or TNF-α.

Preferentially, M0 macrophages are polarized into M2 macrophages in the presence of IL, IL-4 and/or IL-13.

Preferentially, M2 macrophages produce or secrete anti-inflammatory cytokines, preferentially TGF, TGF-β, CCL and/or CCL-18.

Preferentially, M1 macrophages induce pro-inflammatory, anti-tumor response or are activated against at least one pathological cell or pathological site, or activated to protect a healthy site, preferentially surrounding the pathological site, preferentially by involving or through the activation or their interaction immune entity, receptor, cell receptor, CD, preferentially CD90 or CD-96, MHC, preferentially MHC-II, iNOS.

Preferentially, M2 macrophages induce anti-inflammatory response, preferentially to protect a health site, preferentially by involving or through the activation or their interaction with CD, preferentially CD163, CD206, CD209, ARG1, or CD-96,

Preferentially, cells or entity or polarizable cells or entity such as Macrophages display plasticity, where plasticity is preferentially characterized by a capacity of these cells or entity to adapt or change or switch or control or trigger or activate or inactivate their state or phenotypes or state or state of polarization depending on context, environmental cues, response to stimuli or radiation, presence (or not) of nanoparticles, presence or detection or inactivation or preservation (or not) of a pathological or healthy site.

Preferentially, differentiation can be the transformation or change of state of a cell or polarizable cell or entity from an undifferentiated to a differentiated state.

In some cases, undifferentiated monocytes can be differentiated to produce or in naive macrophages (M0).

In some case, the method according to the invention is preceded by or comprises a step, preferentially an additional step, during or in which an undifferentiated cell is transformed into a differentiated cell, preferentially by associating or bringing tother at least one un-differentiated cell or entity with at least one nanoparticle under the application (or not) of radiation.

In some case, the cell or entity or polarizable cell or entity results from the differentiated cell, preferentially by associating or bring tother at least one un-differentiated cell with at least one nanoparticle under the application (or not) of radiation.

Preferentially, the cell or entity can occupy at least two states of polarizations with opposite or different functions or functional patterns, preferentially towards or against a pathological or healthy site.

In some case, the macrophage can be polarized into M1 and/or M2 macrophages preferentially with opposite functional patterns.

In some cases, at least one polarizable or polarized cell or entity, preferentially a M1 macrophage, exhibit at least one pro-inflammatory effect or activity, preferentially through the secretion of pro-inflammatory immune entities or cytokines, such as TNFα, IL-6, IL-12, IL-23, or IFNγ, preferentially acting against a pathological site or cell.

In some other cases, at least one polarizable or polarized cell or entity, preferentially a M2 macrophage, is involved in or triggers the regulation or activation or inactivation of at least one immune response or immune entity preferentially to restore or activate or protects or deactivate homeostasis or healthy site or cell preferentially within tissues or body part preferentially through the secretion or activation or deactivation of at least one immune entity or anti-inflammatory immune entity or cytokines such as IL-10.

Preferentially, in or within at least one pathological site or tumor or its environment or micro-environment, at least one cell or entity or polarizable or polarized cell or entity, most preferentially at least one macrophage or monocyte-derived macrophages, is/are recruited or diffuse or attracted or activated or inactivated or extracted from or by or to or towards the blood vessels or body part or healthy site or pathological site preferentially to infiltrate or diffuse into or activate or deactivate or restore or heal or destroy, partly or fully, the body part or tumor or pathological site or healthy site.

In some cases, at least one cell or entity, preferentially a polarizable or polarized cell, is transformed or changes its state or state of polarization by or in the body part, healthy site, pathological site, and/or tumor microenvironment, preferentially from an undifferentiated to a differentiated state, or from one state or state of polarization or non-polarization to another state or state of polarization, preferentially from one type of cell or entity or polarizable cell or entity, preferentially a cell or entity or polarizable cell or entity that is not associated with a pathological site or tumor to a cell or polarizable cell that is associated with a pathological site such as a tumor-associated macrophages (TAM).

In some cases, tumor-associated macrophages (TAM) exhibit a M2-like phenotype.

In some cases, at least one polarizable or polarized cell, preferentially a M2 macrophage, secretes or triggers or activates at least one immunosuppressive signal that preferentially block at least one immune activity, most preferentially the immune activity of an immune entity or cell or Th or Th1-type immune activity, preferentially losing the immune control of cancer cells, preferentially promoting tumor growth, angiogenesis and/or matrix remodeling, preferentially favoring resistance to treatments.

In one embodiment, the method according to the invention and/or the at least one nanoparticle and/or the at least one cell or entity, preferentially polarizable and/or polarized, is used to monitor or diagnose or trigger or activate or inactivate at least one pathway or at least one immune entity or activity preferentially of the body part or individual, preferentially the Nuclear Factor Kappa B (NF-κB) signal transduction pathway and/or interferon regulatory factor (IRF), and/or inflammation.

In one embodiment, the detection and/or activation of at least one pathway and/or immune entity such as NF-κB pathway, involves a gene and/or a reporter construct, preferentially expressing "secreted embryonic alkaline phosphatase" (SEAP) gene, which is preferentially activated preferentially via at least one receptor such as PRR (Pattern Recognition Receptors) or TLR (Toll Like Receptor) receptor preferentially under the control of a promoter that preferentially activates a pathway such as the transcription of NF-kapaB genetic pathway.

In some cases, a first immune entity, preferentially of first or second category, can be helped or activated to switch or change one state or state of polarization to another state or state of polarization, by a second immune entity, preferentially of first or second category, in the presence or not of nanoparticle(s) under the application (or not) of radiation, preferentially involving an immune entity such as a helper cell, a cell receptor, a cytokine, and/or an interleukin, a complex (for example MHC-I, II, or III).

In some cases, the individual can be a mammal or a human or an animal or part of it.

In some cases, the body part can be comprised in the individual or part of it.

In some cases, the cell or cell component such as an aurosome or a nanometer cell component, which is preferentially comprised in the cell or entity or polarizable cell or entity preferentially together with at least one nanoparticle, can exist or modulate or trigger or activate or inactivate the response or activity, preferentially inflammatory in some cases, preferentially anti-inflammatory in some other cases, preferentially pro-tumoral or favorable to the development of a pathological site in some cases, preferentially anti-tumoral or inactivating a pathological site in some other cases, preferentially protecting a healthy site or body part in some cases, preferentially inactivating or destroying or eliminating a healthy site in some other cases, preferentially of the cell or polarizable cell or macrophage, preferentially under the application of a radiation, preferentially by activating or following activation or vibration or excitation or heating or cooling of plasmons or surface or internal waves or at least one component, crystal plane, core, coating, ion, atom preferentially of the at least one nanoparticles, preferentially individually or collectively, preferentially locally, preferentially in some cases at nanoparticle site or locally, preferentially in some other cases at body part site or length scale or macroscopically, preferentially during or following photothermal therapy or photodynamic therapy or magnetic hyperthermia or exposure to radiation.

In some cases, at least one change of polarization of at least one cell or entity or polarizable cell or entity corresponds to or is associated with or results in: the programming or re-programming of a cell or polarizable cell, the modeling or remodeling of body part or tissue, healthy site, pathological site, Fibrosis, and/or Tissue repair.

In one embodiment, the method is or comprises at least one treatment, preferentially selected in the group consisting of: i) a medical treatment and/or diagnosis, ii) a treatment of the nanoparticle and/or cell, iii) a radiation and/or radio, and/or immuno and/or chemo therapy, iv) a surgery, v) an anesthesia, vi) a treatment and/or diagnosis preferentially of a disease preferentially comprising at least one pathological site or cell.

In some cases, the cell or polarizable cell can be part of or the entire of the adaptive and/or innate immune system or cell and/or its change of polarization can make it switch from a cell belonging to the adaptative immune system or being to a cell belonging to the innate immune system and/or inversely its change of polarization can make it switch from a cell belonging to the innate immune system to a cell belonging to the adaptative immune system.

In some cases, the radiation and/or physcio-chemical disturbance and/or perturbation can be a stimulus preferentially applied on the cell or polarizable cell, preferentially triggering its change of state or polarized state.

In one embodiment, the method according to the invention is a screening method, preferentially used to identify the type of state of polarized state of the at least one cell or polarizable cell.

In some case, the nanoparticle(s) and/or cell(s) or entity, in part or fully, according to the invention is/are or comprise(s) or is/are comprised in at least one system, device, drug, and/or therapeutic or diagnosis substance, preferentially nanometric, preferentially resulting or produced by or issued from at least one step of the method according to the invention.

In some case, the nanoparticle(s) and/or cell(s) or entity according to the invention is/are this/those before, during or after their introduction in or interaction with the body part or nanoparticle(s) and/or cell(s) or use in at least one step of the method according to the invention.

In some case, the nanoparticle(s) and/or cell(s) or entity is a nanoparticle or nanomaterial comprising a mixture of at least one cell or entity or cell or entity component and at least one nanoparticle or nanoparticle component, wherein the nanoparticle component, preferentially nanoparticle core, is preferentially surrounded by a coating or at least one cell component, preferentially part or the entire of a cell organelle.

In some case, the nanoparticle core or inner part comprises less immunogenicity than the nanoparticle coating or external part and/or the nanoparticle core or inner part is not immunogenic and/or the nanoparticle coating or external part is immunogenic.

In some cases, the nanoparticle is able to trigger or triggers or activates a change of state, preferentially polarization state, of the at least one cell or entity, preferentially polarizable cell, preferentially when it comprises an immune entity, preferentially in its coating, preferentially of first or second category.

In some cases, the state or state of polarization is or corresponds to or leads to an immune-reaction, which in some cases is different depending on the state or state of polarization.

In some cases, the cell or entity or polarizable cell or entity can have plasticity, preferentially a higher level of plasticity when it is polarized than when it is unpolarized or when it is differentiated than when it is undifferentiated.

In some cases, the plasticity of the cell or entity is or is associated with its faculty to change its phenotype or state or state of polarization.

In some other cases, the cell or entity can switch from state A to state B, preferentially following or during at least one step of the method according to the invention.

In some cases, state A can be a state of the pathological site or tumor in which the pathological site or tumor is protected or defends itself or prevents at least one mechanism or immune entity or cell to attack or inactivate or destroy at least one pathological site or tumor, partly or fully,

In some cases, state A can be immune-suppressive, preferentially when it prevents at least immune entity or cell to attack or inactivate or destroy at least one pathological site or tumor, partly or fully,

In some cases, state A can be the state of a cold pathological or tumor site or of its environment or microenvironment.

In some other cases, state B can be the state of the pathological site or tumor in which the pathological site or tumor is not protected or lets at least one mechanism or entity or immune entity or cell to attack or inactivate or destroy at least one pathological site or tumor, partly or fully,

In still some other cases, state B can be the state of a hot pathological or tumor site or of its environment or microenvironment.

In some cases, the parameter P or the number of entity or cell can be PA in state A and/or PB in state B. In some cases, PA/PB, PA/(PA+PB), PA/(PA-PB) is/are larger, preferentially in absolute value or not, in percentage or not, than 0, 1, 10⁻²⁰, 10⁻¹, 1.1, 2, 5, or 10.

In some cases, PA/PB, PA/(PA+PB), PA/(PA-PB) is/are smaller, preferentially in absolute value or not, in percentage or not, than 0, 1, 10²⁰, 10, 1.1, 2, 5, or 10.In some cases, the cell or entity or polarizable cell or entity is a Naive polarizable cell or entity, preferentially an unpolarized cel or entity 1, preferentially M0.

In some cases, the environment is the tumor environment or environment of a phytological site or an environment responsible for the polarization of or polarizing or differentiating a polarizable and/or differentiable cell or entity in at least one polarized and/or differentiated state.

In some cases, a cell or entity or polarizable cell or entity is genetically modified preferentially through transfection preferentially to specifically activate or trigger or deactivate or monitor or detect a pathway, immune entity, or transduction signal such as the Nuclear Factor Kappa B (NF-κB).

In some cases, genetic modification preferentially of the cell or entity or polarizable cell or entity can be carried out using a reporter or reporter construct preferentially expressing a gene such as "secreted embryonic alkaline phosphatase" (SEAP) gene.

In some cases, modification such as genetic modification or selection or obtention of a cell or entity or polarizable cell or entity can be used to produce or choose a cell or polarizable cell with a specific function or component or receptor such as a TLR (Toll Like Receptor) receptor.

In some cases, the expression or production of genes or receptors by a cell or entity or polarizable cell or entity occurs under the control or following of a specific pathway, such as the transcription of NF-κB pathway gene.

In some other cases, the expression or production of a first set of genes or receptors preferentially by a cell or entity or polarizable cell or entity occurs under the control or following the expression or production of a second set of genes or receptors.

In some cases, the secretion or production of at least one molecule or substance such as SEAP preferentially being a signature of the activity or change of polarization of the at least one cell or entity or polarizable cell or entity, is detected, preferentially to identity the or the presence of the cell or polarizable cell and/or at least one of its state(s) or state(s) of polarization(s).

In some cases, a specific set of genes is expressed to trigger a specific pathway preferentially by the cell or polarizable cell, for example IFN-stimulated response element (ISRE) genes may trigger the activation of interferon-regulatory factor pathway (IRF). In such situation, it may be possible to consider that the set of genes and corresponding activated pathway form a pair, where the set of genes preferentially activates its corresponding pathway.

In some cases, the cells can change their state or state of polarization, or be polarized, or be un-polarized, or be differentiated or be un-differentiated, preferentially by being exposed to at least one or one mixture or cocktail of substance(s) preferentially immune entity(ies), preferentially consisting of lipopolysaccharides (LPS), cytokine(s), interleukin(s), chemokine(s), IFN-γ, IL-4, and/or IL-13, preferentially to lead to at least one state or change of state of the cell or entity, preferentially polarized, such as M1 and/or M2 macrophages.

In some cases, the change of state achieved by the at least one or one mixture or cocktail of substance(s) of the above embodiment is different from the change of state achieved by at least one step of method or by the at least one cell or entity being assembled or brought into interaction with at least one nanoparticle and/or exposed to the radiation.

In some cases, the cells or entity can change their state or state of polarization, or be polarized, or be un-polarized, or be differentiated or be un-differentiated, preferentially by being exposed to at least one or one mixture or cocktail of substance(s) or entities preferentially immune entity(ies), and/or to nanoparticle and/or to radiation for more than 0, 1, 10, 102, 103 or 105 second(s).

In some cases, the cells or entity can change their state or state of polarization, or be polarized, or be un-polarized, or be differentiated or be un-differentiated, preferentially by being exposed to at least one or one mixture or cocktail of substance(s) preferentially immune entity(ies), and/or to nanoparticle and/or to radiation for less than 105, 103, 102, 5, 2, 1 or 0 second(s).

In some cases, the at least one cell or entity or polarizable cell or entity has at least one phenotype associated preferentially with at least one state or polarization state, which is selected in the group consisting of: a round shape or a more round shape than in another state or polarization state, ii) a compact shape or morphology or a more compact shape or morphology than in another state or polarization state, ii) a rounded shape or morphology or a more rounded shape or morphology than in another state or polarization state, iii) a tendency to branch towards neighboring cells or entity, a more pronounced tendency to branch towards neighboring cells or entity than in another state or polarization state.

In some cases, one type of cell or polarized cell displays a different appearance from another type of cell or entity or polarized cell or entity, for example M1 macrophages can display a distinct fusiform appearance compared to M0 or M2 macrophages.

In some cases, the state or state of polarization of a cell or entity or polarizable cell or entity is derived from the involvement of or activation by an immune entity such as THP-1.

In some cases, the differentiation is not or occurs at a different time or in a different location as or than the change of polarization of a cell or entity.

In some other cases, the differentiation is or occurs at the same time or in the same location as or than the change of polarization of a cell or entity.

In some cases, the state or polarization state can be detected by PCR.

In some cases, some cell or entity or polarizable cells or entity, preferentially un-differentiated cells or entity, preferentially monocytes, exhibit minimal or no pro and/or anti-inflammatory cytokines or immune entity secretion and/or activation, preferentially indicative of their non-inflamed status.

In some other cases, some cell or entity or polarizable cells or entity, preferentially M0 macrophages, preferentially unpolarized cells or entities, demonstrate high or higher levels of pro and/or anti-inflammatory cytokines or immune entity secretion or activation preferentially compared to monocytes, and/or lower levels of pro and/or anti-inflammatory cytokines or immune entity secretion or activation preferentially compared to M1 and/or M2.

In some cases, some entities, preferentially immune entities, such as helper cells or Th1 are produced or activated by some polarized cells or entities, preferentially M1.

In some cases, some polarized cells or entities, preferentially M1 macrophages, secrete high levels or significantly higher levels of entities, preferentially immune entities, such as IFN-γ and IL-1β preferentially than other polarized cells or entities or cells or entities in another state or state of polarization such as their M2 counterparts.

In some cases, the state or state of polarization is triggered or activated by specific substances:
- Preferentially in some cases inflammatory agents, preferentially LPS, preferentially to trigger the activation of a receptor such as TLR4, preferentially to activate the expression of pro-inflammatory mediators or agents;
   and/or
- Preferentially in some other cases anti-inflammatory agents, preferentially dexamethasone, preferentially to inhibit the expression of inflammatory mediators or agents;

In some cases, the nanoparticle and/or radiation and/or physico-chemical disturbance polarize the cell or entity.

In some cases, cell or entity polarization and/or differentiation can be a change of the state or state of polarization from M0 to M1 and/or M2 and/or from an un-differentiated state to M0 and/or from an un-differentiated state to M1 and/or M2.

In some cases, the nanoparticle and/or radiation and/or physico-chemical disturbance de-polarize the cell or entity.

In some cases, cell de-polarization can be a change of the state or state of polarization from M1 and/or M2 towards M0 and/or M0 towards an un-differentiated state and/or M1 or M2 towards an undifferentiated state.

In some cases, the cell or polarizable cell can occupy or be in at least one state, preferentially selected among an undifferentiated state, a differentiated unpolarized state, or at last one polarized state.

In some other cases, before at least one step of the method according to the invention, the cell or entity or polarizable cell or entity is preferentially predominantly in the undifferentiated state or differentiated unpolarized state.

In still some other cases, preferentially during or after one step of the method according to the invention, the cell or entity or polarizable cell or entity is preferentially predominantly in at least one polarized state.

In one embodiment, the cells or polarizable cells form an assembly, wherein preferentially more than 0, 1, 2, 5, 10, 50, 75, 90, 99 or 100% of these cells or entity are in the undifferentiated state or differentiated unpolarized state and less than 100%, 99, 50, 25 or 1% of these cells in at least one polarized state, preferentially before or without or with or during or after at least one step of the method according to the invention.

In one embodiment, the cells or entity or polarizable cells or entity preferentially occupy the undifferentiated state or differentiated unpolarized state more than 0, 1, 2, 5, 10, 50, 75, 90, 99 or 100% of the time or of their lifetime or of the time during which they are in the body part, and/or preferentially occupy at least one polarized state less than 100%, 99, 50, 25 or 1% of the time or of their lifetime or of the time during which they are in the body part, preferentially before or without or with or during or after at least one step of the method according to the invention.

In one embodiment, the cells or entity or polarizable cells or entity preferentially occupy the undifferentiated state or differentiated unpolarized state preferentially less than 100%, 99, 50, 25 or 1% of their lifetime or of the time during which they are in the body part, and/or preferentially occupy at least one polarized state more than 0, 1, 2, 5, 10, 50, 75, 90, 99 or 100% of the time or of their lifetime or of the time during which they are in the body part, preferentially before or without at least one step or sub-step of the method according to the invention.

In one embodiment, the cells or entity or polarizable cells or entity form an assembly, wherein preferentially more than 0, 1, 2, 5, 10, 50, 75, 90, 99 or 100% of these cells or entity are in at least one first polarized state and less than 100%, 99, 50, 25 or 1% of these cells or entity are in a second polarized state, preferentially before or without or with or during or after at least one step of the method according to the invention.

In one embodiment, the cells or polarizable cells or entity occupy at least one polarized state more than 0, 1, 2, 5, 10, 50, 75, 90, 99 or 100% of the time or of their lifetime or of the time during which they are in the body part, preferentially before or without or with or during or after at least one step of the method according to the invention.

In one embodiment, the cells or polarizable cells or entity occupy at least one differentiated state preferentially less than 100%, 99, 50, 25 or 1% of their lifetime or of the time during which they are in the body part, preferentially before or without or with or during or after at least one step or sub-step of the method according to the invention.

In some cases, the radiation is infrared light.

In some cases, the entity or immune response can be intracellular or extracellular or be comprised in the cell or outside the cell.

In some cases, the cell or entity or polarizable cell or entity produces or activates a growth or transforming growth factor.

In some cases, the cell or entity or polarizable cell or entity preferentially in at least one of its states of polarization produces a growth factor that preferentially promotes the progression of a pathological or healthy site or cell or entity.

In some other cases, the cell or entity or polarizable cell or entity preferentially in at least one of its states of polarization inhibits a growth factor that preferentially promotes the progression of a pathological or healthy site or cell or entity.

In some cases, the nanoparticle and/or cell or entity and/or method, optionally under the application of a radiation or physio-chemical disturbance, according to the invention, is used for or triggers the change of state of at least one pathological site or tumor from preferentially a state wherein the pathological site or tumor is lacking at least one immune entity or cell such as a T cell that preferentially acts against or deactivates or destroys the pathological site or tumor or pathological cell to a state wherein the pathological site or tumor comprises at least one immune entity such as a T cell that preferentially acts against or deactivates or destroys the pathological site or tumor or pathological cell, preferentially partly or fully.

The invention also relates to the cell or a distribution or assembly or population, preferentially resulting from the method according to the invention, which comprises at least one cell in a state selected in the group consisting of:
State 1): cell(s) or entity in an undifferentiated state, preferentially at a percentage P1,
State 2): cell(s) or entity in a differentiated state and unpolarized state, preferentially M0, preferentially at a percentage P2,
State 3): cell(s) or entity in a first polarized state, preferentially M1, preferentially at a percentage P3,
State 4): cell(s) or entity, in a second polarized state, preferentially M2, preferentially at a percentage P4.

In some cases, preferentially in a first configuration, the number or volume of at a least one cell or entity in at least one state is larger than the number or volume of the at least one cell in at least one other state. In some other cases, preferentially in a second configuration, the number or volume of at a least one cell or entity in at least one state is smaller than the number or volume of the at least one cell or entity in at least one other state,
wherein preferentially the assembly or at least one cell or entity is associated or brought into the presence of at least one nanoparticle, preferentially changing or resulting in or being the distribution of cells or entity within the assembly, preferentially in some cases to increase the number or percentage or resulting in a larger number or percentage of cell or polarizable cells or entity preferentially relatively to the percentage or number of unpolarized cells or entity, preferentially in some cases to increase the number of percentage of at least one polarized state relatively to at least one other polarized state; preferentially in some other cases to decrease the number or percentage of cell or entity or polarizable cells or entity relatively to the percentage of unpolarized cells or entity, preferentially in some other cases to decrease the number of percentage of at least one polarized state relatively to at least one other polarized state;

Preferentially the assembly or at least one cell or entity preferentially with at least one nanoparticle is exposed to radiation, preferentially to change the distribution of cells within the assembly, preferentially in some cases to increase the number or percentage of cell or entity or polarizable cells or entity relatively to the percentage of unpolarized cells or entity, preferentially in some cases to increase the number of percentage of at least one polarized state relatively to at least one other polarized state, preferentially in some other cases to decrease the number or percentage of cell or entity or polarizable cells or entity relatively to the percentage of unpolarized cells or entity, preferentially in some other cases to decrease the number or percentage of at least one polarized state relatively to at least one other polarized state.

In some cases, the at least one state or state of polarization of a cell or entity or polarizable cell or entity is preferentially selected in the group consisting of: 1) an undifferentiated state such as a state of a mast state, 2) a differentiated state such as the state of M0, 3) a first polarized state such as a state of M1, 4) a second polarization state such as a state of M2.

In some cases, P1+ P2 + P3+ P4 = 100%

In some other cases, P1 is larger than P2, P3, and/or P4.

In some other cases, P2 is larger than P1, P3, and/or P4.

In some other cases, P3 is larger than P1, P2, and/or P4.

In some other cases, P4 is larger than P1, P2, and/or P3.

In some other cases, P1 is smaller than P2, P3, and/or P4.

In some other cases, P2 is smaller than P1, P3, and/or P4.

In some other cases, P3 is smaller than P1, P2, and/or P4.

In some other cases, P4 is smaller than P1, P2, and/or P3.

In some cases, P1, P2, P3 and/or P4 is/are larger than 0, 1, 10, 25, 50, 75, 80, or 99 %.

In some other cases, P1, P2, P3 and/or P4 is/are smaller than 100, 75, 50, 25, 10, 5, 2, 1 or 0%.

In some cases, the above percentage(s) represent(s) the number or volume or surface or cell or entity or length or thickness of a given cell divided by the total number or volume or surface or cell or length or thickness of cells or entities, preferentially in or within the body part, healthy site, and/or pathological site;

The invention also relates to nanoparticles, preferentially involved in or activating or following at least one step of the method according to the invention, preferentially synthesized in the presence of at least one cell or entity, preferentially eukaryotic, and/or organelle(s) or cellular vesicle(s) or endosome(s) or exosome(s) or lysosome(s) or cellular compartment(s), preferentially lysosome(s), and at least one salt, preferentially a metallic salt, most preferentially gold and/or iron salt, optionally originating from a nanoparticle or magnetosome, preferentially administered to or in a body part, preferentially being dissolved or transformed or decomposed into a salt or ions or ions forming a salt, preferentially in or by the body part, partly or fully,
wherein the nanoparticle(s) or salt has/have at least one property selected in the group consisting of:
it is composed of or comprises at least 2 metals or metal atoms, which are surrounded by or mixed with or bound through or interacting through or assembled by part of or the entire of an organelle or cellular vesicle or endosome or exosome or lysosome or cellular compartment, preferentially intracellular, most preferentially inside an eukaryotic cell, preferentially belonging to or originating from a pathological cell,
   and
it forms a nanoparticle or a particle with a size smaller than 1000, 100, 50, 20, 10, 5, 2 or 1 nm, preferentially in at least one dimension or cross section of the nanoparticle or particle, through the composition of at least 1 or 2 metals or metal atoms, which is/are surrounded by or mixed with or bound through or interacting through or assembled by or transformed into a nanoparticle or particle that has at least one property that differs from that of a single atom, or made crystallized or magnetic, preferentially diamagnetic, paramagnetic, superparamagnetic, ferromagnetic or ferrimagnetic, by part of or the entire of an organelle or cellular vesicle or endosome or exosome or lysosome or cellular compartment, preferentially intracellular, most preferentially inside an eukaryotic cell, preferentially belonging to or originating from a pathological cell,

The invention also relates to an assembly of three components, preferentially involved in or activating or following at least one step of the method according to the invention, comprising:
A first component or part comprising at least one extracellular vesicle or vesicle membrane,
A second component or part comprising at least one nanoparticle and/or salt, preferentially metallic salt, And
A third component or part comprising at least one immune entity, preferentially immuno-regulating, wherein the first, second and third components preferentially interact through weak or strong bonds, preferentially the third component is weakly bound to the first and/or second component(s), preferentially to enable its release and/or dissociation from the first and/or second component(s), wherein the second component is preferentially comprised in the first component,
wherein the assembly is preferentially releasing the third component, preferentially upon the application of radiation,
wherein the assembly is preferentially polarizing and/or differentiating at least one cell, preferentially a TAM,
wherein the assembly is preferentially formed, preferentially through at least one interaction between at least two of its components, or activated, preferentially to destroy, partly or fully a pathological site or to protect, partly or fully, a healthy site.

The invention also relates to a method, preferentially involved in or activating or following at least one step of the method according to the invention, to determine by PCA the change in polarization, differentiation or immune modulation of at least one cell, preferentially a TAM, comprising:
A first step comprising collecting at least two different immune cells or entity, preferentially two macrophages or TAM with two different states of polarization;
A second step of measuring the different shapes, morphologies, plasticity, sizes, and/or productions of at least two different immune entities by the two cells;
A third step of classifying the immune cells based on the results of the second step;
The invention relates to a method comprising: i) step 1 of providing at least one salt or nanoparticle and at least one entity, ii) step 2 of assembling and/or mixing, and/or conjugating, and/or complexing at least one salt or nanoparticle with at least one entity, and/or iii) step 3 of exposing or having exposed or letting exposed the material of or resulting from step 1 or 2, partly or fully, to a radiation or physico-chemical disturbance.

### DESCRIPTION OF THE FIGURE

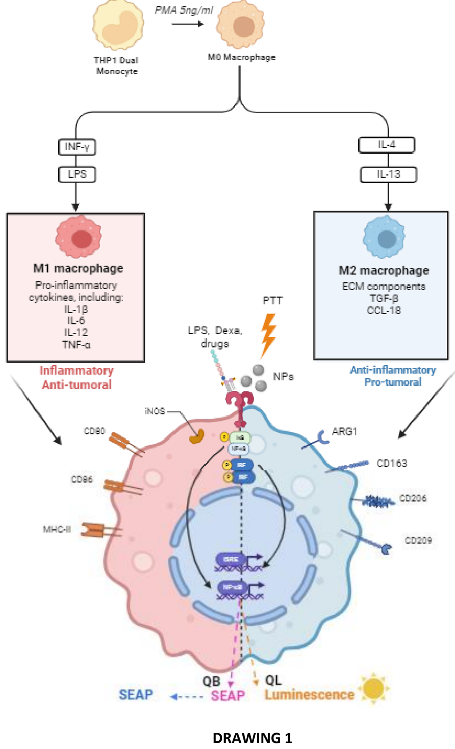

**Figure 1****:** Schematic illustration of a screening test platform to predict the effects of different treatment on macrophage polarization and inflammation *in vitro.* Dexa: Dexamethasone; IRF: Interferon Regulatory Factor pathway; NF-κB: Nuclear factor kappa B pathway; SEAP: Secreted Embryonic Alkaline Phosphatase. QB: QUANTI Blue, QL: QUANTI-Luc, TLR: Toll-Like Receptor; NPs: Nanoparticle, ISRE: Interferon Stimulated Response Element. Scheme Created with BioRender.com.

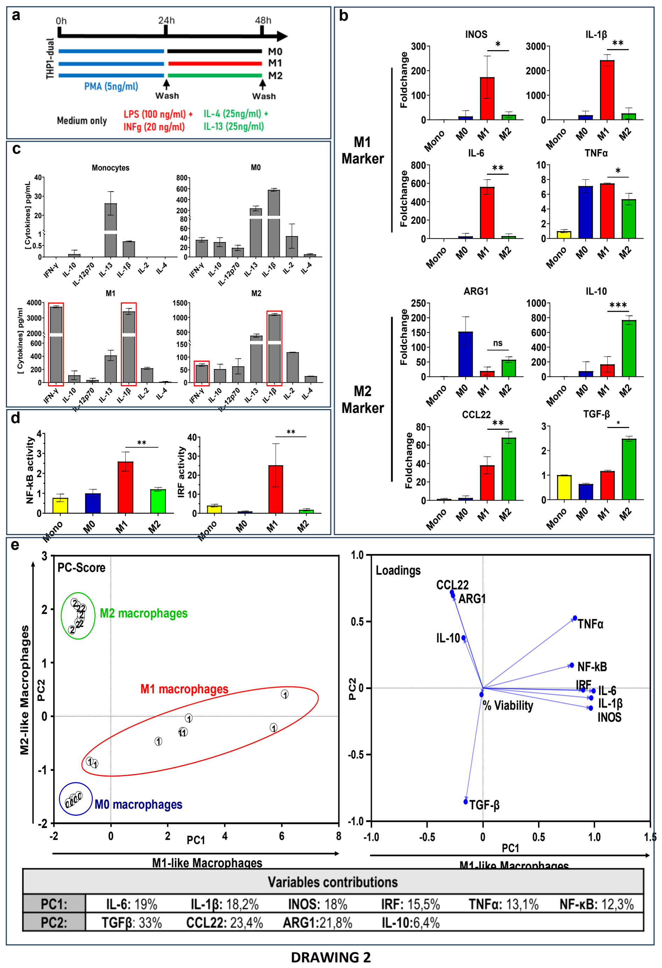

**Figure 2****:** Validation of THP-1 Dual cell line polarization into M1 and M2-macrophages. a) Protocol of polarization used. b) Relative mRNA expression of M1 and M2 macrophage markers was assessed in monocytes and M0, M1, and M2 macrophages using qPCR. Analysis was conducted on untreated samples and normalized to the RPLPO housekeeping gene. Results are presented as foldchange based on monocytes and expressed as mean ± 1 SD. N=3. c) Quantification of human pro-inflammatory cytokines using MSD multiplex ELISA. Samples were collected from supernatant of THP-1 Dual monocyte and differentiated macrophages into M0, M1 and M2 after 48h in culture; d) IRF and NF-κB activation of THP-1 Dual monocytes and macrophages. IRF and NF-κB signaling pathways were quantified using QUANTI-Luc^{™} and QUANTI-Blue^{™}, respectively. Data were normalized into foldchange based on M0 macrophages. e) The Principal Component Analysis (PCA) was carried out on differentiated macrophages, utilizing the methodology described in this study and based on data from b and d. qPCR and pathway activation data were normalized on M0 macrophages. PCA automatically identified three distinct clusters representing M0, M1, and M2 differentiated macrophages (shows as 0,1 and 2 respectively on the PCA scatter plot (PC scores, left), thereby validating the reliability of our differentiation protocol. The loading plot (right) refers to the contributions of the different features into the two first PCs. The contributions are detailed in the variable contributions table. Results are shown as the mean ± 1 SD N=3. Results of univariate statistical analysis are illustrated by *, ** or ***, respectively indicating p < 0.05, 0.01 or 0.001.

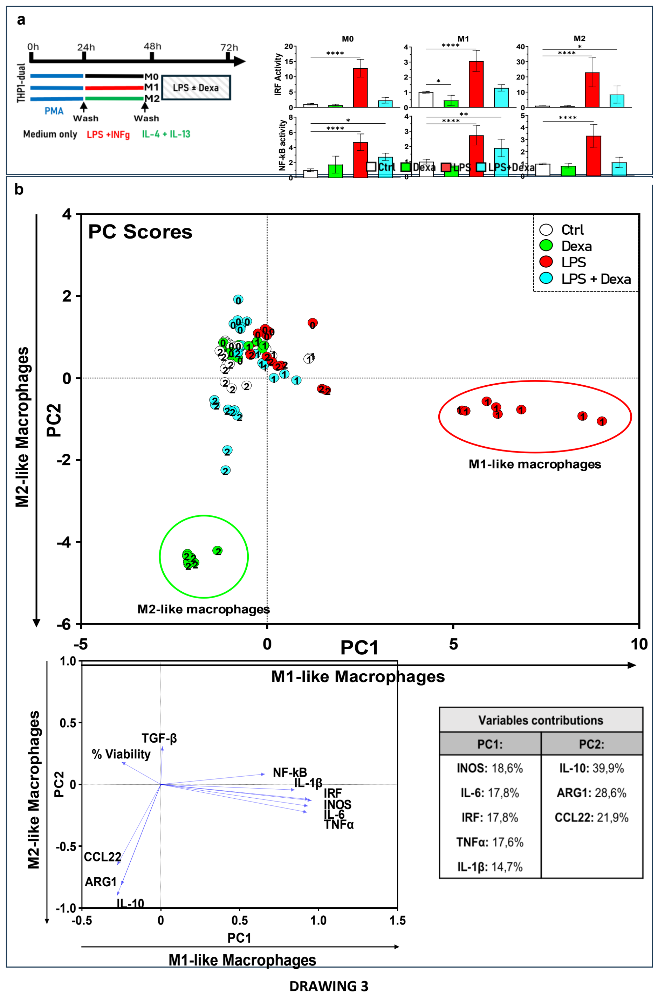

**Figure 3****:** a) Polarization protocol employed, and control treatments used to modulate macrophage polarization and NF-kB or IRF activity. The macrophages were treated with two different agents: Lipopolysaccharide (LPS), which acts as an inflammatory agent, triggering Toll-like receptors (TLRs) and prompting a proinflammatory response; Dexamethasone, which was used as an anti-inflammatory control. Combination of LPS and Dexamethasone was used to evaluate the protective effects of dexamethasone against inflammation. Specifically, LPS was administered at a concentration of 100 ng/mL to induce activation, while dexamethasone was applied at 10 µg/mL to inhibit inflammation. b) PCA was carried out to uncover the immunomodulatory effects of differentiated macrophages, with these additional control treatments considered for examination. Scatter plot (PC scores, top) exhibited three clusters, two of them corresponding to extremely polarized macrophages (i.e. M1 macrophages treated with LPS and M2 macrophages treated with Dexa). The third cluster regroup controls and macrophages treated with inflammatory agent inducing opposing effect to the polarization (M1 macrophages treated with Dexa, and M2 macrophages treated with LPS). The loading plot (bottom) refers to the contributions of the different features into the two first PCs. The contributions are detailed in the variable contributions table. Results of univariate statistical analysis are illustrated by *, ** or ***, respectively indicating p < 0.05, 0.01 or 0.001.

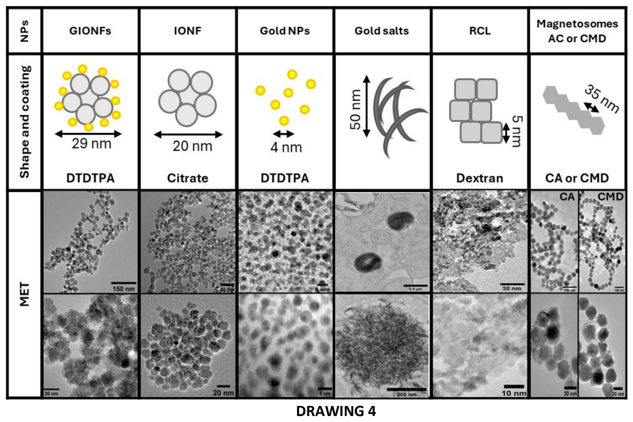

**Figure 4****:** Description of NPs used in this study. Gold-decorated Iron Oxide Nanoflowers (GIONF) consist of citrate-coated 20 nm multicore Iron Oxide Nanoflowers (IONF) decorated with 2-5 nm gold NPs coated with dithiolated diethylenetriamine pentaacetic acid (DTDTPA). The gold salt aurothiomalate was used to treat THP-1 Dual^{®} derived M0 macrophages resulting in the formation of intralysosomal nanoassemblies of tiny gold 2 nm nanoclusters, forming curved lashes, also called aurosomes. RCL NPs are multicore magnetic plates embedded in a dextran (H(C6H10O5)xOH) 40kDa matrix. Magnetosomes isolated from magnetotactic bacteria were coated with carboxymethyl dextran (CMD) or citric acid (CA). Transmission electron microscopy (TEM) was employed to obtain images of the NPs deposited on the grid. In case of Au salts, TEM pictures show 30 nm slices of THP-1 Dual^{®} macrophages (M0) treated with Au salts for 24 hours and embedded in resin.

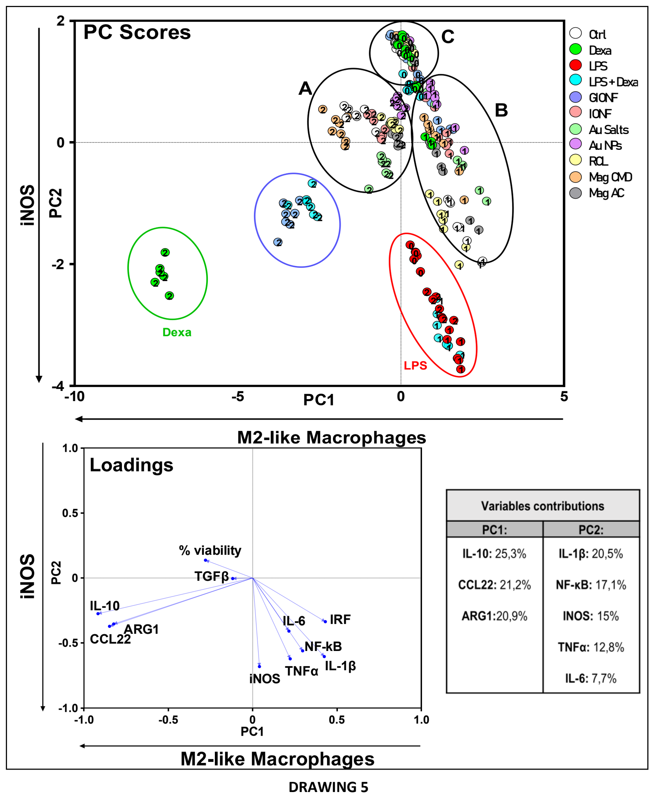

**Figure 5****:** PCA was conducted on M0, M1, and M2 macrophages and exposed to various nanoparticles (NPs). NPs used in this study encompass gold and iron oxide NPs, magnetosomes, RCL (Resonant Circuits Limited Company) NPs, and Au Salts. The PCA was performed to comprehensively evaluate the impact of different NPs on macrophages. The concentrations of NPs used were below the toxicity thresholds for macrophages to avoid confounding effects on gene expression or inflammation modulation resulting from cell death. Specifically, a concentration of 5 µg/mL of iron or gold was employed. LPS and Dexamethasone was used as pro and anti-inflammatory control respectively. The figure presents scatter plot (PC scores, top) showing 6 clusters. The loading plot (bottom) refers to the contributions of the different features into the two first PCs. The contributions are detailed in the variable contributions table.

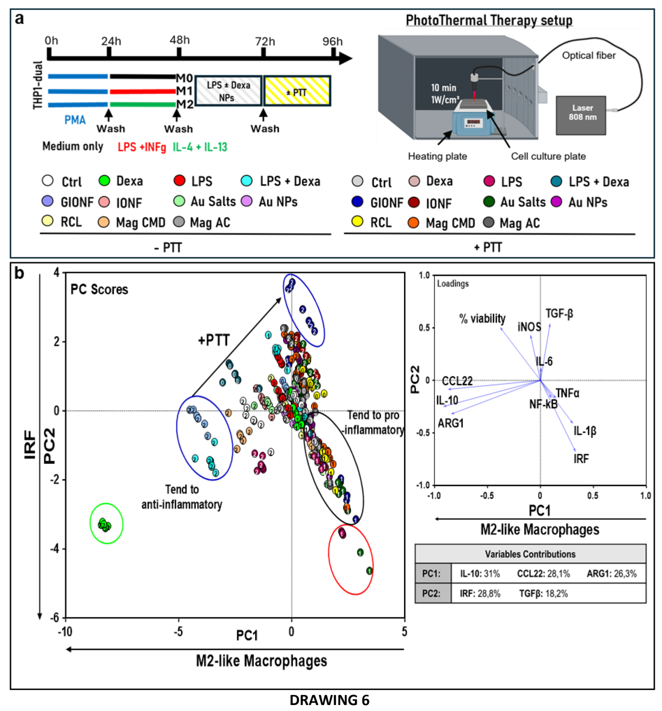

**Figure 6****:** a) Protocol for polarization was employed with and without photothermal therapy (PTT), utilizing a PTT session at 808 nm for 10 minutes at a power density of 1W/cm². The two PCA graphs illustrate the impact of various nanoparticles (NPs) on macrophage polarization and inflammation status, with and without PTT activation. b) PCA demonstrates the activation of the IRF pathway depending on M2-like macrophage polarization.

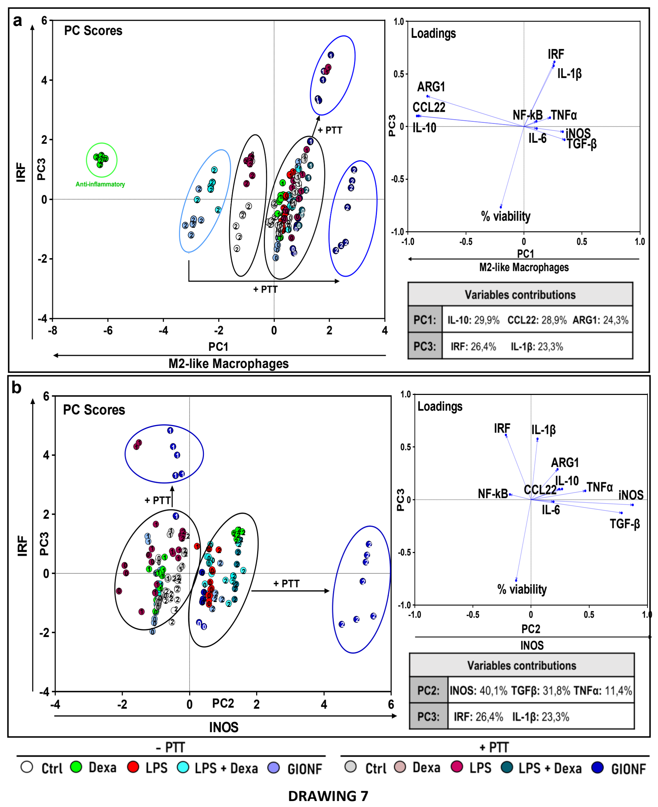

**Figure 7****:** The figure is focused on controls and GIONFs on macrophage behavior using PCA, with and without PTT. a) The figure presents scatter plot (PC scores, left) showing 6 clusters. The loading plot (right) refers to the contributions of the different features into the two first PCs. The contributions are detailed in the variable contributions table. The PCA graph illustrates the differentiation gradient of macrophages and the activation status of the IRF pathway. b) The figure presents scatter plot (PC scores, left) showing 4 clusters. The loading plot (right) refers to the contributions of the different features into the two first PCs. The contributions are detailed in the variable contributions table.

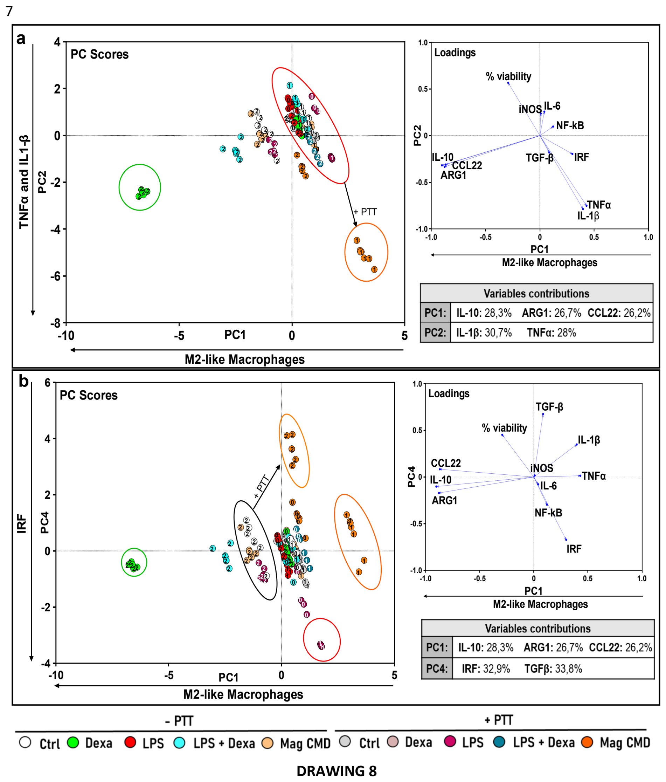

**Figure 8****:** To explore the impact of CMD magnetosomes on macrophage behavior, PCA was conducted on M0, M1, and M2 cells treated with CMD, with or without photothermal therapy (PTT), alongside control groups, including LPS, dexamethasone, and PBS. a) The figure presents scatter plot (PC scores, left) showing 4 clusters. The loading plot (right) refers to the contributions of the different features into the two first PCs. The contributions are detailed in the variable contributions table. The PCA depicts the differentiation gradient of macrophages towards M2-like states along PC1 and the activation status of TNFα and IL-1β along PC2. b) The figure presents scatter plot (PC scores, left) showing 7 clusters. The loading plot (right) refers to the contributions of the different features into the two first PCs. The contributions are detailed in the variable contributions table.

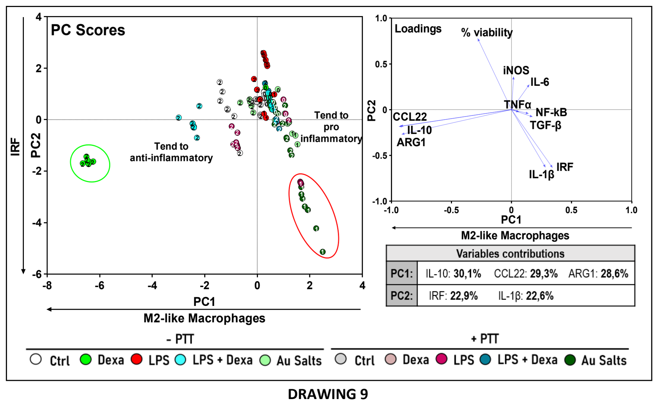

**Figure 9****:** Gene expression profiles of macrophages under Au Salts treatments. The figure presents scatter plot (PC scores, left) showing 3 clusters. The loading plot (right) refers to the contributions of the different features into the two first PCs. The contributions are detailed in the variable contributions table. PCA delineates distinct clusters: dexamethasone (green) indicating an anti-inflammatory shift toward an M2 state, and LPS + PTT (red) corresponding to a pro-inflammatory M1 state. Photoactivated Au salts prompt M2 macrophages toward an M1-like phenotype, while PTT alone accentuates M1 polarization toward M1-like phenotype and IRF pathway activation, suggesting an immunomodulatory role of gold nanostructures under PTT influence.

The description is followed by the non-limiting example below.

### EXAMPLE:

### Multivariate screening and automated clustering of macrophage immunoreactome to nanoparticles and photothermal therapy

The goal of personalized medicine is to fully and timely control the immune system in response to threats, malignancies, and various treatments. Immunotherapy harnesses the reactivity of the immune system to modulate the immune response toward a beneficial defense against diseases such as cancer or infections. Nanotechnology is part of the armamentorium to reprogram the immune response in a spatially and temporally controlled manner for enhanced immunotherapy. However, the complexity and plasticity of the immunoreactome makes it difficult to predict using simple in vitro high-throughput screening tests. Here, we present a fast machine learning-assisted predictive screening assay to monitor with reduced dimensionality the multifactorial responses and the modulation of the macrophage phenotype spectrum following any type of treatment. Human THP-1 monocyte cell lines engineered to monitor NF-kB and IRF pathway activation were differentiated into M0 macrophages and further polarized into M1- and M2-macrophages. Principal component analysis (PCA) of M0, M1, and M2 cytokine secretion, gene expression, and NF-kB and IRF pathway activation profiles demonstrated clusters of behaviors that separated the macrophage subsets and revealed their distinct multifactorial responses to lipopolysaccharide (LPS) and dexamethasone as pro- and anti-inflammatory stimuli, respectively. This screening model was used to compare the outcome of the complex phenotypic pattern of macrophages after treatment with different iron oxide and gold nanoparticles (NPs), as well as gold salt treatment, followed by NIR light irradiation to induce a photothermal (PTT) effect. Most NPs induced per se some shifts in macrophage polarization, with PTT intensifying the IRF-triggered pro-inflammatory responses of M1-like cells. Interestingly, gold-iron oxide nanoflowers (GIONFs) were the only NPs that induced M2-like macrophage polarization, whereas the addition of PTT reprogrammed the M2-like macrophages into the M1-like cell profile without IRF pathway activation. These findings shed light on the intricate immunomodulatory effects of NPs and PTT on macrophage behavior and provide a basis for an adaptable quick screening method for the predictive design of therapeutic strategies for immunotherapy in cancer and other diseases.

The capacity to predict, control, and modulate the immune system in response to threats and pathological conditions is currently one of the most challenging and promising objectives of modern medicine. Immunomodulation is a therapeutic intervention that enhances or represses the body's defense mechanisms. Immunotherapy is revolutionizing the management of cancer, helping our immune system to recognize and fight malignant cells by stimulating the so-called inflammatory "hot" tumor microenvironment (TME). In addition, acute inflammation, which normally leads to recovery from infection or tissue repair in the case of injury, can lead, when not properly phased by stimuli and checkpoints, to chronic inflammation, tissue destruction, remodelling, fibrosis and even favour cancer progression.

In order to progress in the control of immunity, immunotherapeutic approaches have rapidly expanded its armamentarium with new small molecules drugs as well as nanosystems that can activate innate and adaptive immune responses and show h3igh affinity to crosstalk with the immune system. The remote activation of nanosystems that are responsive to light, magnetic fields, or other physical stimuli, can potentially offer additional spatial and temporal control over the targeted modulation of immunity in cancer, autoimmune, or inflammatory diseases. However, given the complexity of the immune reactivity to multiple environmental stimuli, it is still difficult to predict the immunomodulatory properties of a given treatment in a specific pathological context. There is a lack of in vitro immunomodulatory assays and companion analysis protocols that can embrace the broad spectrum of immune reactivity to compare drug candidates and allow for rational AI-assisted drug design and selection. In vitro high-throughput high-content screening methods combined with machine learning approaches, could help predict multifactorial effects of therapeutic interventions and drive immunomodulatory science in vivo, accelerating the development of novel and more precise immunotherapeutic approaches.

In this paper, we present a combination of versatile high-content screening tests with a simple statistical analytic approach to appraise the immunomodulatory properties on macrophage functions of any drugs or nanosystems, activated or not with external fields. Our test is designed to embrace a broad range of macrophage phenotypes and functions, and to elucidate, by means of principal components analysis (PCA), how some specific nanoparticle treatments, coupled or not to photothermal activation, can shift or reprogram the macrophage immune-reactome in a precise way.

This screening model can identify combinatory treatments to finely and timely control macrophage reactivity in different pathological contexts, including solid tumors and inflammatory disorders. Building up a screening model to assess macrophage response patterns to different treatments.

Monocytes and macrophages are innate immune cells involved in multiple pathophysiological processes, including (i) clearance of dead cells and recognition of pathogens such as bacteria or viruses via pattern recognition receptors, (ii) increased proliferation to eliminate a large diversity of pathogens, (iii) recruitment of other immune cells by functioning as antigen-presenting cells, leading to an indirect activation of immune effectors such as T cells, and (iv) regulation of inflammation through the production of pro- or anti-inflammatory cytokine [Rostam, H. M. et al Sci Rep 7, 3521 (2017).]. Macrophages display high plasticity, allowing them to adapt their phenotypes depending on context, environmental cues, and response to stimuli, including NPs. In the past decades, intensive phenotyping of macrophage lineages in different contexts has revealed distinctive macrophage functional subsets or patterns of activity. In vitro models of monocyte-derived macrophages have been extensively used as surrogates to assess in vivo macrophage function, focusing on schematized subsets with different polarization M0, M1 and M2. Monocytes are commonly differentiated to produce naive macrophages (M0) and then polarized into M1 and/or M2 macrophages with opposite functional patterns. M1 macrophages exhibit pro-inflammatory effects through the secretion of pro-inflammatory cytokines, such as TNFα, IL-6, IL-12, IL-23, or IFNγ. The microbicidal and phagocytosis machinery of M1 macrophages allows them to participate to the clearing of acute infections and elimination of xenobiotics. However, excessive, or prolonged M1 polarization can lead to tissue injury and contribute to pathogenesis. In contrast, M2 macrophages are involved in the regulation of the immune response to restore homeostasis within tissues through the secretion of many anti-inflammatory cytokines such as IL-10. M2 subpopulation, therefore, presents regenerative properties. Nevertheless, M2 macrophages cover a continuum of phenotypic and functional properties. For example, specific M2 program induced by bacterial infection can lead to a chronic evolution of infectious diseases. In tumors, monocyte-derived macrophages are recruited from the blood vessels to infiltrate the tumor. These cells are transformed by the tumor microenvironment into tumor-associated macrophages (TAM) that mostly exhibit a M2-like phenotype. M2 macrophages secrete immunosuppressive signals that block the Th1-type immune activity, losing the immune control of cancer cells, promoting tumor growth, angiogenesis and matrix remodeling, and favoring resistance to treatments [Lewis, C. E. et al Cancer Research 66, 605-612 (2006).]. Therefore the M2 macrophage subset has emerged as a promising target for anti-cancer therapy, and the induction of a shift in polarization from M2 to M1 macrophages holds significant potential in advancing anti-tumor therapeutic strategies [Han, S. et al. Theranostics 11, 2892-2916 (2021), Cheng, Y. et al, Adv Healthcare Materials 10, 2100590 (2021), Riley, R. S. et al WIREs Nanomed Nanobiotechnol 9, e1449 (2017)]. In sum, the plasticity of the monocyte/macrophage system to dynamically adapt to a wide array of environmental cues has challenged the vision of stable tissue- and response-specific differentiation lineages and polarization patterns and has claimed for an ever-evolving progression of functional patterns within heterogeneous populations. Therefore, an in vitro screening model to assess the multifactorial effects of various treatments should encompass the entire spectrum of macrophage response patterns. For this purpose, we used monocyte-derived macrophages polarized to M0, M1, and M2 phenotypes as recipient cells for nanoparticles, drug, and light exposure, and investigated the resulting display of numerous distinct functional phenotypes through multivariate statistical analysis (figure 1).

This rapid and straightforward test is designed as a screening tool to offer a snapshot of the inflammation and polarization status of macrophages in vitro during various treatments, including metallic iron oxide and gold nanoparticles (NPs) combined or not to photoactivation. To achieve this, we used THP-1 Dual^{®}, a commercial human monocytic cell line genetically modified through transfection to specifically monitor the Nuclear Factor Kappa B (NF-κB) signal transduction pathway and interferon regulatory factor (IRF), both of which are involved in inflammation. For the detection of NF-κB pathway activation, a reporter construct expressing "secreted embryonic alkaline phosphatase" (SEAP) gene is activated via PRR (Pattern Recognition Receptors) or TLR (Toll Like Receptor) receptor under the control of a promoter activated by the transcription of NF-kapaB pathway genes. SEAP secreted into the medium is thus quantified by measuring absorbance using a detection reagent. For the detection of the activation of interferon-regulatory factor pathway (IRF), IFN-stimulated response element (ISRE) genes were expressed under the control of the Interferon-stimulated gene 54 (ISG54) promoter activated in response to external stimulation. ISRE expression was linked to a luciferase reporter construct (Figure 1). Luciferase production thus allows measurement of IRF pathway activation using a detection reagent. The first step was to validate whether previously defined differentiation protocol could induce polarisation into M1 and M2 macrophages of the THP-1 Dual^{®} cell line. The THP-1 Dual^{®} monocytes were first treated with phorbol 12-myristate 13-acetate (PMA) for 24 hours to induce M0 macrophage differentiation. Subsequently, the cells were washed and exposed to different cocktails, consisting of lipopolysaccharides (LPS) + IFN-γ or IL-4 + IL-13, to polarize towards M1 and M2 macrophages, respectively, over a 24-hour period (Figure 2a). Morphologically, THP-1-derived M0 macrophages exhibited a rounded and compact shape, while M2 macrophages displayed a rounded morphology with a tendency to branch towards neighboring cells. In contrast, M1 macrophages displayed a distinct fusiform appearance compared to M0 macrophages. These observable phenotypic variations provided initial evidence of successful differentiation. qPCR results showed that genes associated with M1 characteristics (iNOS, IL-1β, IL-6 and TNFα) were expressed at higher levels in THP-1 dual^{®}-derived M1 macrophages than in monocytes, M0, and M2 macrophage subsets, whereas the genes known to present an elevated expression in M2 macrophages (ARG1, IL-10, CCL22, TGF- β) were also more highly expressed in THP-1 derived M2 macrophages generated using the aforementioned protocol (Figure 2b). The differentiation protocol was also assessed by analyzing cytokines release from differentiated macrophages and monocytes. Monocytes exhibited minimal pro-inflammatory cytokines secretion, indicative of their non-inflamed status. In comparison, M0 macrophages demonstrated higher levels of pro-inflammatory cytokines secretion compared to monocytes, consistent with the inherently more inflammatory nature of macrophages compared to monocytes. Finally, the results showed that THP-1 derived M1 macrophages secreted significantly higher levels of IFN-γ and IL-1β than the M2 counterparts (Figure 2c). These findings are consistent with the established understanding that M1 macrophages exhibit a more inflammatory profile than M2 macrophages [Strizova, Z. et al. Clinical Science 137, 1067-1093 (2023)]. The behavior of differentiated THP-1 Dual^{®} macrophages appeared to mimic that of regular primary macrophages. To further investigate the inflammatory pathways specifically expressed in THP-1 Dual^{®} cells, namely NF-κB and IRF, the activation levels of these two transcription factor families, key components of inflammation signaling, were examined (Figure 2d). The results showed in figure 2d are the one at 48h and indicate that the activation of these two pathways increased in the M1 polarization state compared to that in both M0 and M2 cells. These findings align with the existing literature and qPCR results, confirming that our protocol could successfully differentiate THP1 Dual^{®} monocytes to M0 and then polarize them to M1 and M2 macrophages, with heightened inflammatory profile in M1 compared to their M0 and M2 counterparts, and with distinctive M1 and M2 characteristics.

Based on the multiple features that were measured to characterize each macrophage population, namely the cell metabolic activity, the gene expression levels, the cytokines concentrations and the activation level of NF-κB and IRF inflammatory pathways, we performed a multivariate analysis by Principal Component Analysis (PCA) to present the outcomes of the differentiation/polarization protocol in an integrated way with reduced dimensionality. In figure 2e, the graph displays the PC Score of the THP-1 Dual^{®} derived macrophages. The abscissa, or the horizontal axis (labelled PC1), illustrates a gradient of macrophage polarization towards an M1-like profile, as confirmed by the adjacent loading graph that encompasses a range of pro-inflammatory genes and cytokines, including IRF, NF-kB, iNOS, IL-1β, etc. The ordinate, or the vertical axis (labelled PC2), represents a polarization gradient towards an M2-like profile, characterized by genes such as CCL22, ARG1, and IL-10, as depicted in the loading graph. In all PCA graphs, THP1 dual^{®}-derived macrophages M0, M1, and M2 according to the above protocol will consistently be denoted as "0", "1", and "2", respectively. As expected, three primary clusters are visible: one at the bottom left corresponding to M0 cells, another at the top left representing M2 cells, and an expanded cluster at the bottom right characterizing M1 macrophages. These cells exhibited significant polarization plasticity and variability, with some cells remaining closer to the M0 cell state and others displaying an extreme M1 phenotype on the far right. This first PCA confirmed that the set of variables and features that were quantified was sufficient to cluster the nominal M1 and M2 polarization of macrophages, also clearly distinguished from naive THP-1 Dual^{®} derived M0.

The next step to establish a relevant multivariate analysis able to characterize the multifactorial immunomodulatory effect of different treatments to assess the outcome of well-known pro- and anti-inflammatory agents that will be further used as control treatments LPS was used as an inflammatory agent to trigger TLR4 activation, and dexamethasone was used as an anti-inflammatory control that inhibits the expression of inflammatory mediators. Finally, a combination of dexamethasone and LPS was used to evaluate the protective effects of dexamethasone on LPS-induced inflammation. These treatments were applied on M0, M1 and M2 THP-1 derived macrophages. The impact of control treatments is illustrated in Figure 2a that depict the activation of the IRF and NF-κB pathways. Treatment with lipopolysaccharide (LPS) appears to activate both pathways across different cell lines, while dexamethasone displays a modest inhibitory effect on IRF pathway activity exclusively in M1 macrophages and appears ineffective in modulating NF-κB activity within these pro-inflammatory cells (Figure 2a). This observation may be attributed to the pronounced activation state of M1 macrophages induced by the differentiation protocol. In instances where inflammation is already established within the M1 pro-inflammatory profile, Dexamethasone encounters challenges in mitigating inflammation. Moreover, to identify the optimal timing for observing the activation of both the NF-κB and IRF pathways on THP1 dual monocytes and macrophages, we performed a monitoring of NF-kB and IRF pathway over time at intervals of 3 hours, 24 hours, and 48 hours following the different treatment.

Results indicate dexamethasone treated M1 macrophages have a significant protective effect against the activation of these pathways at 24 hours, but this effect is lost at 48h time point. However, combined treatment with dexamethasone and LPS demonstrates an intermediate effect, potentially due to dexamethasone protective role in inflammatory conditions provoked by LPS.

PCA results depicted in figure 3b the impact of these opposite control treatment in the macrophage polarization. PCA show a horizontal axis (PC1) that illustrates a gradient of macrophage polarization towards an M1-like profile while the vertical axis demonstrates a gradient towards M2-like polarization extending from top to bottom. Two additional distinct populations were discernible in the PCA score plot. A red cluster to the right of PC1 corresponds to M1 cells treated with LPS, whereas a green cluster at the left of PC1 and bottom of PC2 corresponds to M2 cells treated with dexamethasone. These clusters demonstrate the capacity of LPS and dexamethasone to drastically shift the polarization status of macrophages along the gradients principally defined by PC1 and PC2. Examination of loading vectors revealed that variables associated with the red cluster included IRF, iNOS, IL-6, IL-1 β, TNFα, and NF-kB, which are markers of M1. Conversely, the variables associated with the green cluster included CCL22, ARG1, and IL-10, indicating M2-like characteristics. On the PC2 axis from top to bottom, we observe successively the clusters of M0, then M1 and M2, whether they are treated or non-treated, while on the PC1 axis, from left to right, we observe M2, M0 and then M1 subsets. Treatment with LPS tends to shift all populations to the right, but more importantly the M1. Treatment with dexamethasone has a prominent effect only on M2 subset. In contrast the combination of LPS plus dexamethasone mitigates the effect of dexamethasone on M2, and of LPS on M1, on PC2 and PC1 axis, respectively, whereas it tends to shift the M0 to the top. This clear and consistent separation of cellular profiles demonstrates the reactivity of M1 and M2 THP-1 Dual^{®} derived macrophage to LPS, Dexa or the combined treatment. These results validate our screening model based on THP-1 Dual^{®} monocytes coupled to PCA to investigate the macrophage plasticity and its regulation by external stimuli. In the following, we use this novel methodological approach leveraging PCA to appraise the immunomodulatory effects of metallic nanoparticles and salts, combined or not with photo-activation, that have been proposed as innovative treatments, primarily in the field of cancer.

Broad spectrum impact of various gold and iron oxide nanoparticles and salts on macrophage polarization and inflammatory status.

Gold and iron oxide nanoparticles have been extensively employed in clinical and preclinical contexts as imaging contrast agents, [Luo, D. et al Cancers 13, 1825 (2021)], drug carriers, photo- or magnetically activable nanosystems, bactericidal agents or metal supplements. Despite their rapid uptake by the monocyte-macrophage systems or their internalization by tumor-associated macrophages (TAMs) within the tumor microenvironment [Daldrup-Link, H. E. et al. Clinical Cancer Research 17, 5695-5704 (2011)], the specific influence of NPs on macrophage physiology remains unclear and probably tissue dependent. Given their extensive application in cancer diagnosis, therapy, and anaemia [Fütterer, S et al Journal of Pharmaceutical and Biomedical Analysis 86, 151-160 (2013)] as well as inflammatory diseases treatment, studies deciphering the broad spectrum of macrophage modulation by NPs should be of significant interest to predict in vivo response.

In this study, we take advantage of the methodological approach reported above to comprehensively assess the broad-spectrum impact of different NPs. Seven NPs were chosen based on their differences in shape, materials (gold and iron oxide), coating and size distribution (figure 4) and their promising therapeutic applications. Citrate coated Iron Oxide Nanoflowers (IONFs) are monocrystalline 20-25 nm multicore maghemite (Fe2O3) superparamagnetic NPs with unique magnetic properties that make them outstanding contrast agents for MRI and one of most efficient magnetic nanostructures known to generate hyperthermia under alternating magnetic field [Nicolas-Boluda, A. et al. Nanoscale 13, 18483-18497 (2021)]. They have been also reported as efficient photothermal agents under NIR light (808 nm) irradiation in preclinical models of solid tumors, especially when IONF were decorated with ultrasmall gold NPs measuring 2-5 nm and coated with dithiolated diethylenetriaminepentaacetinanoparticles (Au@DTDTPA), forming the so-called gold iron oxide nanoflowers (Au@DTDTPA@IONF or GIONF) [11]. The efficacy of GIONF as PTT agents for softening and shrinking cholangiocarcinoma human tumor xenografts was demonstrated and their immunomodulatory properties were investigated in a syngeneic model of triple negative breast cancer [Nicolás-Boluda, A. et al. ACS Nano, 2020 May 26;14(5): 5738-5753]. Furthermore, ultrasmall Au@DTDTPA also function as radiosensitizing nanoagents with the aim of enhancing the efficacy and targeted delivery of radiotherapy. Additionally, these nanoparticles have been envisioned to support image-guided therapy and promote renal clearance [Alric, C. et al. Nanoscale 5, 5930 (2013)]. The selection of these three types of nanoparticles (IONF, GIONF and Au@DTDTPA) was primarily driven by their multifaceted applications coupled with their controlled biodegradability in cellular environments. In addition, aurothiomalate is a gold salt that has been used for decades as bactericides and anti-inflammatory agents for the treatment of rheumatoid arthritis [Balfourier, A. et al. Proc. Natl. Acad. Sci. U.S.A. 117, 103-113 (2020)]. Importantly it has been shown that Au salts can be rapidly internalized by cells and compartmentalized within lysosomes, where they undergo bio-mineralization, forming first three-dimensional (3D) arrangement of tiny gold nanoclusters and ultimately 2-D shaped nanostructures reminiscent of curved leaves, named "aurosomes" that were observed in patients treated with Au salts for months [Balfourier, A et al Proc. Natl. Acad. Sci. U.S.A. 117, 22639-22648 (2020)]. These intracellular nanostructures exhibit long term bio-persistence, sparking interest in the study of gold-based nanomaterials for biomedical applications and potential photosensitivity. Notably, we have shown recently that the later stages of intralysosomal degradation of gold nanoparticles appear to coincide with this gold biomineralization process starting from Au salts, highlighting a common mechanism of gold metabolism. Here we have treated THP-1 Dual^{®} derived macrophages (M0) with aurothiomalate for 24 hours and observed aurosomes formation consisting of 3D assemblies of curved lashes within membrane bound endo-lysosomes as shown in figure 4. These intracellular gold nanostructures evolve after 7 days into larger and more defined 2D curved leaves, as previously observed in treated patient's cells. These aurosomes piqued attention as intriguing biogenic platform for selective targeting and therapeutic administration due to their potential optical properties resulting from the gold quantum confinement effect and light-absorbing capabilities. In the context of the tumor microenvironment or inflammatory diseases, research into how aurosomes immunomodulate the microenvironment through macrophage polarization or inflammation, with and without light activation, holds promise for our understanding of their immunomodulatory effects and potential in therapy.

Another category of iron-based NPs investigated in this study are bacterial magnetosomes manufactured by the company Nanobactérie. These NPs are nanostructures that are naturally found inside magnetotactic bacteria named Magnetospirillum magneticum strain AMB-1. Magnetosomes are composed of aligned magnetic particles surrounded by a bacterial membrane. They typically have a size range of 30-37 nm and are primarily composed of magnetite (Fe3O4) or maghemite (_Fe2O3). Following extraction from bacteria to render them biocompatible and nontoxic, magnetosomes are coated with substances such as carboxymethyl dextran (CMD) or citric acid (AC) [Alphandéry, Front. Bioeng. Biotechnol. 2, (2014).]. Magnetosomes have interesting properties for magnetic and light-induced hyperthermia. These nanoparticles are distinguished by their high crystallinity and chain-like organization, endowing them with superior heating capabilities until the optimal temperatures of 41-50 °C under the influence of an alternating magnetic field. The surface coating of poly-L-lysine further enhances their stability and reduces toxicity risks. Notably, preclinical models have shown that magnetosomes can completely eradicate tumors in mice, illustrating their effectiveness as a cancer treatment [Le Fèvre, R. et al. Theranostics 7, 4618-4631 (2017)]. Finally, clinical grade RCL iron oxide NPs, supplied by Resonant Circuits Limited Company, were used in our investigation. These RCL nanoparticles are currently under clinical evaluation for magnetic hyperthermia associated with standard of care chemotherapy to treat patients with locally advanced pancreatic cancer. They belong to a class of multicore magnetic NPs characterized by the presence of multiple distinct magnetic cores, each enclosed within a non-magnetic matrix that physically isolates them from adjacent cores. The magnetic cores are primarily composed of magnetite (Fe3O4) and/or maghemite (Fe2O3), whereas the non-magnetic matrix comprises a low-molecular-weight polysaccharide known as dextran (H(C6H10O5) xOH), with an approximate molecular weight of 40 kDa.

To assess the immunomodulatory effect of the different NPs and gold salt treatments, M0, M1, and M2-like macrophages were treated for 24 hours and the different descriptors characterizing macrophage polarization and inflammation status were measured and analyzed by PCA, offering valuable insights into the nuances of cellular responses in comparison to the standard dexamethasone and LPS treatments (figure 5). Importantly, the exposure to NPs and Au salts was carried out at concentrations that were below the toxicity thresholds for macrophages, with the aim of reducing any potential confounding effects on gene expression or inflammation modulation resulting from cell death. The non-toxic concentration was set to 5µg / mL, based on the measurements of the mitochondrial metabolic activity of cells. Interestingly, when aggregating all the NPs and salt treatments to the "control" LPS and dexamethasone treatments, the two PC that emerged from the PCA were different from the PC with control treatments alone (figure 3), showing distinctive immunoreactome to the nanoparticles. PC1 demonstrates a gradient of polarization towards M2-like macrophages and PC2 suggests a gradient from anti-inflammatory to pro-inflammatory properties principally via the expression of iNOS, IL-1β and activation of NF-kappaB pathway. PCA reveals distinct clusters crossing these axes, each associated with specific treatments, highlighting the diversity of the immunological reactions to NPs and Au salts. M2 treated with dexamethasone are found in the cluster outlined in green at the bottom left of the axes signaling a deep polarization toward the M2-like phenotype. In contrast, the cluster outlined in red at the bottom right encompasses sequentially M0, M2 and M1 treated with LPS, indicating a gradient of proinflammatory response and a shift to M1-like phenotype. The clusters A, B and C outlined in black, encompass respectively the M2, M1 and M0 cells treated with various NPs or non-treated. M0 cluster "C" is located in the upper middle section displaying no polarization towards either M1 or M2-like macrophages, nor any activation of the NF-kB pathway or iNOS expression, which is consistent with the anticipated outcomes for M0 macrophages. Consistently, M2 cluster "A" and M1 cluster "B" are located on the left and right on the PC1 axis, respectively. The variability in the cellular responses to the different NPs can be observed within the clusters A, B and C, meaning that none of the NPs drastically reprogram the polarization of macrophage subsets. However, in comparison to the untreated M1 and M2 controls, we observe that most of the NPs treatment, especially the gold NPs, gold salt, IONF and magnetosomes AC tend to depolarize both the M1 and M2 subsets, making them closer to M0. Interestingly we observed distinct behavior of GIONF and magnetosomes CMD, that shift M1 towards M0 cluster, and displace M2 towards the cluster of M2 treated with dexamethasone. GIONF have the most prominent effect on M2 sharing a common cluster with M2 treated with LPS and dexamethasone. In conclusion, this analysis provides in-depth insights into the correlation between cellular inflammatory responses and applied treatments, shedding light on immunomodulating properties of the tested nanoparticles.

Nanoparticles-mediated immunomodulation of macrophages is differentially reprogrammed by photothermal activation.

In solid tumors, dense desmoplastic tumor presenting a stiff extracellular matrix are associated with poor prognosis due to resistance to conventional therapies. Therefore, novel strategies targeting ECM are envisioned to break down the physical barriers that restrain antibodies and immune cells to penetrate solid tumors and initiate immunogenic cell death [Korangath, P. et al. Sci. Adv. 6, 1601 (2020)]. We and other have shown that a less stiff ECM allows for the enhancement of immune cells infiltrating the tumor [Nicolas-Boluda, A. et al. eLife 10, e58688 (2021)]. In this context, NPs remotely activated by external near-infrared light (NIR) or alternating magnetic field can induce local and controlled mild hyperthermia in the tumor region, eliciting multifactorial effects on the TME, such as de-structuration of ECM, tumor softening, cell apoptosis or ferroptosis, lysosomal disruption and potential change of a cold towards a hot immune environment. NPs with different materials, sizes, shapes are often selected based on their light-to-heat energy conversion properties and biocompatibility [Lucas, T. et al. Nanoscale, 2023, 15, 17085-17096] but few is known on their immunomodulatory properties, especially when activated by an external field. [Chanput, W. et al International Immunopharmacology 23, 37-45 (2014); Sica, A. et al European Journal of Cancer 42, 717-727 (2006).]. As many preclinical tumor models rely on human xenograft in immunodeficient mice, the endogenous immune response to NPs and PTT remains poorly investigated, even if recent studies pointed out very different biodistribution and outcomes of NPs in immunocompetent models.

To fill this gap, we used our screening model to study how NP-mediated hyperthermia affects the macrophage inflammatory status and polarization. As shown above, it was important to assess the immunoreactome to different NPs treatment first in absence of external activation. We next added a sub-toxic exposure to NIR light (photothermal treatment, PTT) to evidence the specific effect of photoactivation on macrophages (figure 6a). As before, the viability of cells was evaluated to ensure that the observed effects were not caused by cell death due to NPs or photoactivation. A concentration of 5µg/ml of NPs was chosen because it was found to be non-toxic without PTT, but still led to some cell death after light irradiation with the viability remaining above 70%. Additionally, we confirmed that the observed effects were due to nanoscale heating within the cells caused by photoactivation, rather than heating of the culture medium.

Upon examination of the PC score graphs (figure 6b), the ordinate axis PC2 displays a gradient of IRF pathway activation from top to bottom. Simultaneously, the abscissa PC1 illustrates a gradient of M2-like macrophage polarization from right to left. For each NPs treatment, results are indicated by dots with light color without PTT and dark color after PTT. So, one should pay attention to the shift from light to dark dots that emphasizes the outcome of PTT for specific NPs. As before, a distinct cluster outline in green encompasses M2 macrophages treated with dexamethasone. In contrast, a conspicuous cluster outlined in red comprised M1 cells treated with LPS but also M1 cells treated with gold salt and PTT, indicating a pronounced proinflammatory profile via IRF activation. M2 macrophages treated with NPs, without PTT, occupy the left side of PC1 axis, whereas M1 cells treated with NPs alone and with PTT are all located on the right side. The widespread cluster outlined in black contains M1 cells treated with NPs and PTT that align along a proinflammatory trajectory confirming the impact of NPs combined with PTT to enhance the inflammation state of the cells. Positions along this trajectory classify the NPs in descending order of their pro-inflammatory effect under light exposure: Gold salt, GIONF, RCL, magnetosome CMD, IONF, Gold NPs and magnetosome AC. Interestingly, the outcome of PTT on M2 macrophages treated with NPs is even more contrasted. PTT tends to shift the M2 clusters to the top right, sometimes beyond the M0 clusters, demonstrating M2 reprogramming into M1-like macrophages with an increase in TGF-β and iNOS expression. This reprogramming effect is the most pronounced for GIONF. As seen above, GIONF alone have the same effect as the combination of LPS and dexamethasone. However, when exposed to light, M2 are reprogrammed to the cluster outlined in blue at the ext3reme position on the top right. Therefore, the intriguing pro-M2 effect of GIONF alone is completely reversed by light activation inducing an overexpression of iNOS and TGF-β and bypassing the activation of IRF.

The other NPs treatments when combined with PTT also induce a shift of M2 macrophages to the top right of the pattern, although this effect is much less pronounced than for GIONF. It is interesting to note that M2 treated with NPs and PTT are located close to or beyond the cluster encompassing M0, which demonstrates the reversion of M2 polarization by PTT. On the other hand, in the case of M1 macrophages, the combination of NPs with PTT doesn't change the macrophage polarization. However, PTT induces a more pronounced M1-like polarization and increased the inflammatory status of these cells by activating the IRF pathway.

This multivariate analysis and automated clustering of macrophage immunoreactome to the different NPs shed light on the nuanced spectrum of macrophage responses and allows to compare, rank and classify the NP's and PTT treatments as function of their immunomodulatory outcome. While this statistical analysis gives a very general and integrated view of the differential effects of NPs combined to light activation, it also revealed some NPs with unique behaviour, among which we focused on GIONF, magnetosomes CMD and gold salts. In the following, we examine in more details the macrophage responses to these particular treatments.

GIONFs: photoactivable nanoparticles with distinctive properties, reversing polarization and inflammation status.

To better characterize the effect of GIONFs on macrophage polarization and inflammation, we isolated data from macrophages treated with GIONF +/- PTT and controls to perform the PCA. With these restricted data, the first three principal components are PC1, a polarization gradient to M2-like macrophages, PC2, mostly representing iNOS and TGF-β activation and PC3, representing IRF pathway activation. Scatter plots are shown in Figure 6a (PC3 versus PC1) and Figure 6b (PC3 versus PC2). As before, the cluster of M2 treated with dexamethasone is at the extreme left along the PC1 axis. M2 treated with GIONF without PTT share the same next cluster as M2 treated with dexamethasone + LPS, demonstrating the pro-M2 effect of GIONF alone compared to the non-treated M2. However this M2 polarization induced by GIONF is observed without any activation of IRF pathway on PC3 (unlike treatment with dexamethasone, LPS or both that induce a basal level of IRF activation). In contrast, iNOS and TGF-β are overexpressed during M2 polarization by GIONFs (right shift on PC2). GIONF have few effect on M1 and M0 macrophages in absence of PTT.

As shown before in comparison to the other NPs, the light activation on GIONFs has drastic consequences both on M1 and M2 macrophages, but in different directions. M2 treated with GIONF and PTT are at the extreme right along PC1 axis, showing M2 depolarization without activation of IRF, but with strong overexpression of iNOS and TGF-β right position on PC2). Besides, the PTT accentuates the M1 profile of M1 treated with GIONFs along with a strong activation of IRF pathway but without overexpression of iNOS. In the context of the tumor microenvironment, these results are very promising, as they suggest the potential of GIONF combined with PTT treatment to reprogram protumoral M2 macrophages into antitumoral and immunogenic M1. Simultaneous, M1 macrophages are push to a more inflammatory state through the activation of the IRF pathway, which can be beneficial to promote a hot tumor microenvironment. Further investigations are needed to understand the mechanisms by which M2 macrophages reverse to an M1-like phenotype without IRF activation, but with activation of iNOS expression in response to GIONF and PTT, and the role of this new subtype of M1-like macrophages in the TME or in other context.

The activation of iNOS in macrophages is regulated by nuclear factor kappa B (NF-kB), which serves as a crucial transcription factor in the regulation of inflammatory and immune responses. NF-kB activation occurs in response to various inflammatory stimuli, such as tumor necrosis factor (TNF) or transforming growth factor beta (TGF-β), or in response to pathogens and other inflammatory signals. Once activated, NF-kB translocates to the cell nucleus, where it regulates the expression of several genes including iNOS. The activation of iNOS leads to the synthesis of nitric oxide (NO) in macrophages, which plays a crucial role in the immune response against pathogens and is involved in inflammatory processes and M1 macrophage differentiation [Xue, Q. et al IJMS 19, 3805 (2018).]. In our study, M2 macrophages treated with GIONFs + PTT tended to exhibit an M1-like profile, including early activation of the NF-κB pathway and late activation of iNOS, together with inhibition of the IRF pathway. Further investigation of the modulation of NF-κB and iNOS at shorter intervals after PTT, such as 1, 3, 6, and 12 h, would provide valuable insights into the temporal dynamics of these pathways and their roles in macrophage responses.

Moreover, recent studies have shown that photodynamic therapy can activate iNOS-derived NO in macrophages and has a protective effect in killing cancer cells [Bhowmick, R. et al Free Radical Biology and Medicine 57, 39-48 (2013)]. Our results also align with studies demonstrating that ferumoxytol, an iron oxide NP approved by the FDA for treating anemia, is capable of inducing tumor growth inhibition in mice, macrophage polarization towards an M1 phenotype, and eliciting a more inflammatory and anti-tumoral response [Zanganeh, S. et al. Nature Nanotech 11, 986-994 (2016)]. Additionally, the M1 macrophage profile induced by ferumoxytol triggered a Fenton reaction, leading to the apoptotic death of adjacent tumor cells. This initiates a feedback loop resulting in the production of tumor necrosis factor-alpha (TNF-α) and nitric oxide (NO), as evidenced in this study.

Importantly, the immunomodulatory outcomes of GIONF that is predicted in our in vitro screening model are fully consistent with our previous observations in vivo in a preclinical model of triple negative breast cancer. GIONF were injected intratumorally and the tumor was exposed to NIR light, generating mild hyperthermia at 43°C for 15 min. Although PTT did not induce significant changes in tumor growth, it resulted in an increase of CD8+ T lymphocytes and pro-inflammatory cytokines in the tumor contributing to the transition of a "cold" tumor devoid of T cells into a favorable pro-inflammatory, T-cell infiltrated anti-tumoral microenvironment. Importantly, the PTT strongly transformed the tumor immune microenvironment already shaped by GIONF, in line with our in vitro results. Hence the combination of a timely controlled immune response to GIONF and to mild hyperthermia at the best favorable time-window could be used as a remotely triggered adjuvant treatment to precisely alter the tumor microenvironment and enhance the efficacy of immunotherapeutic approaches, such as immune checkpoint inhibitors or CAR T cell therapy.

In other contexts of inflammatory and auto-immune diseases, it would be of interest to test the effects of GIONF alone, which proved to favor M2 polarization in our screening test.

Magnetosomes CMD immunomodulation on THP-1 Dual macrophages.

We next isolate the immunostimulatory effects of magnetosomes CMD with and without PTT on macrophages. In figure 8a, we focused on principal components, PC1, a polarization gradient towards M2-like macrophages from right to left, and PC2, representing the activation of TNFα and IL-1β from top to bottom. In figure 8b, we added PC4 (versus PC1) mostly representing the activation of IRF pathway from top to bottom. In Figure 7a, without PTT, we observe that M1 treated with magnetosomes CMD belong to the cluster outlined in red close to control M1, and to the control M0, M2, and M1 treated with LPS, with or without PTT. In the absence of PTT, none of the conditions induce TNF-α or IL-1β activation. In this representation (Figure 7a), the most conspicuous effect is seen on the M1 macrophages treated with CMD magnetosomes and PTT, that show a shift to the right bottom due to a strong activation of pro-inflammatory genes, TNFα and IL-1β. The co-activation of TNF-α and IL-1β is known to amplify the macrophage's immune response against pathogens and to have double-edged sword in tumors with either pro-oncogenic and tumor-suppressive functions, for example by inhibiting the M2 phenotype or by normalizing tumor vasculature and interstitial pressure, with beneficial effects on drug delivery. When adding the PC4, we can see that this pro-inflammatory response to the combined treatment of magnetosomes CMD and light activation, is not dependent on IRF pathway activation. This is a significant difference with the pro-inflammatory response of M1 macrophages to GIONF and PTT which is activated through IRF pathway.

When looking at the M2 response, we also observed important differences between GIONFs and magnetosomes CMD. M2 cells treated with CMD magnetosomes without PTT remain on the same cluster than the control M2, while the GIONF favor a deeper M2 polarization. Nevertheless, these M2 treated with magnetosome CMD shift from left to right along the PC1 axis after light activation, indicating a less M2-like profile owing to PTT and a slight activation of TNF-α and IL-1β. This polarization shift after PTT is also associated with TGF-β activation and inhibition of IRF pathway as shown in Figure 7b. These results indicate that CMD magnetosomes coupled to PTT can inhibit IRF activation in M2 subsets but also in M1 macrophages under PTT inflammatory conditions. These findings provide an overview of the intricate immunomodulatory effects of magnetosomes on macrophages. While M1 macrophages treated with GIONF and PTT exhibit strong IRF activation, M2 macrophages inhibit it and activate the iNOS pathway. However, CMD magnetosomes elicit contrasting responses, with inhibition of IRF pathway in M1 and M2 macrophages even with PTT, whereas the PTT on control M0 and M1 treated with LPS (without NPs) significantly activate IRF pathway. The mechanism of action of CMD magnetosomes here passes by the initial co-activation of TNFα and IL-1β at the beginning of the immune response to the light exposure.

Gold salt-induced intracellular aurosome nanostructures can modulate the macrophages responses and are photoactivable by PTT.

Regarding the treatment with gold salt, the two principal components were again PC1, a gradient towards M2 polarization and PC2, representing the activation of IRF pathway. M0 and M1 macrophages, when treated with Au salts without PTT, remain in the same cluster on the right side of the PC1 axis together with M1 controls. However, when exposed to the laser, the M1 macrophages treated with gold salt are displaced to the bottom right, demonstrating significant PTT-inducible IL1-β and IRF pathway activation and shift towards a more inflammatory M1 phenotype. Regarding the M2, Au salt alone displace M2 to the right side on PC1 axis in comparison to the control. Under PTT, this right shift on PC1 is even more pronounced accompanied by an activation of IL1-β and IRF pathway. The combined Au salt + PTT treatment thus results in a reversion of the M2 polarization and the stimulation of inflammatory M1 macrophages. Au salt forms gold aurosome nanostructures within macrophages as soon as after day 1 of treatment, which, under the impact of light exposure, induce the reprogrammation of macrophages towards an inflammation state. This first confirmed the optical reactivity of aurosomes formed in macrophages, and second the potential of gold salt treatment combined with light activation to transform a cold towards hot tumor immune microenvironment.

### Conclusion

In this study, we shed light on the complex interplay between nanoparticle exposure, light irradiation generating nano-hyperthermia, macrophage polarization, and inflammatory state, offering insights into potential therapeutic strategies for cancer treatment and autoimmune diseases. By examining with automated clustering tools the multivariate impact of various nanoparticles on macrophage behavior, we gained a deeper understanding of how these treatments modulate inflammation and polarization dynamics. Additionally, we established a rapid and straightforward screening test for predicting macrophage immunomodulation in vitro, which holds promise for translating the findings into in vivo applications. The first step was to validate the THP-1 dual-cell differentiation and polarization protocol and automated clustering in response to standard LPS and dexamethasone treatments. This study provides a solid foundation for subsequent analyses of the complex gene expression patterns and inflammatory pathway activation in response to various nanoparticles, gold salts with or without light exposure. Our methodology can be extended to any other stimuli, treatment and exposure to external field to comprehensively assess their main effects on the broad spectrum of macrophage behaviour. Moreover, any supplementary descriptors of the cellular response (cytokine release, metabolic state, morphological features...) or any other recipient cells or tissues can be added to this screening test to enrich the description of the immune response to various conditions and perform automated clustering of the immune behaviour with reduced dimensionality. Among the demonstration of a powerful methodological approach for IA assisted immunological screening, our study shed light on the variety of the immune responses to different nanoparticles that should be evaluated case by case and in comparison, to each other. Moreover, it shows that external field application, such as laser exposure, might drastically change the immunomodulatory effect of the nanoparticles, and should be also evaluated in a predictive decision support test prior to in vivo experiments. Overall, our study provides valuable insights into the potential of nanoparticle-mediated hyperthermia as a therapeutic strategy for cancer. By elucidating the effects of nanoparticles and metallic treatments on macrophage polarization and inflammation, we contribute to the rational design and ranking of targeted treatments aimed at modulating the tumor microenvironment and enhancing anti-tumor immune responses.

Material and Method

### THP-1 dual cell culture and differentiation

THP-1 Dual cells (Sigma-Aldrich, St. Louis, MO, USA) are a type of monocytic cell line derived from a one-year-old male patient with monocytic leukaemia. These cells were seeded in 24 well plate at 0.5x10^6 cells/ml in RPMI 1640 medium (Invitrogen, 61870-01), supplemented with 10% heat-inactivated foetal bovine serum, 1 mM sodium pyruvate (REF), 1% penicillin/streptomycin (Life technology, 15070-063), 100 µg/ml Normocin (Invivogen, ant-nr-2), 10 µg/ml blasticidin (Invivogen, ant-bl-1), and 100 µg/ml Zeocin (Invivogen, ant-zn-1) at 37°C in a 5% CO2 environment. To differentiate monocytes into macrophages in an M0-like state, the cells were treated with 5 ng/ml phorbol-12-myristate-13-acetate (PMA) (Sigma: P1585-5MG) for 24 h. Subsequently, the macrophages were differentiated into M1- or M2-macrophages using two different cytokine cocktails for 24 h. Cocktail 1 consisted of LPS (100 ng/ml) and IFN-γ (20 ng/ml) for M1 polarization, whereas cocktail 2 consisted of IL-4 (25 ng/ml) and IL-13 (25 ng/ml) for M2 polarization. After 24 h, the cells were washed 1 time with PBS1X and incubated in complete RPMI medium.

### Nanoparticles, gold salts, LPS and dexamethasone treatment

After differentiation, cells were incubated for 24 h with different NPs or gold salt (aurothiomalate) at a concentration 5µg/ml of iron or gold in RPMI complete medium. lipopolysaccharide (LPS, 100ng/ml) and dexamethasone (Dexa, 10µg/ml) were used as positive and negative controls for inflammation, respectively. These two controls were added at the same time as the NPs treatment. After treatment, the cells were washed 1 time with PBS 1X and incubate in complete RMPI medium. The PTT session started 1 h after washing with PBS.

### Photothermal therapy (PTT)

The cells were exposed to near infrared irradiation (NIR) 808 nm at 1 W/cm2 for 10 min. Laser model: F-808-3W (PhotonTec Berlin GmbH)) Optical fiber: 400µm/0.22NA, 1m.

### NF-kappaB and IRF monitoring with THP-1 dual macrophages.

For NF-κB pathway detection, SEAP secreted in the medium was measured using 180µl of QUANTI-Blue^{™} solution (rep-qbs1) detection reagents (preparation followed by the manufacturer's instructions) mixed with 20 µL of cell supernatant for each well. SEAP secretion in the supernatant induces a turn of the purple/pink QUANTI-Blue^{™} solution to blue and allows indirect monitoring of NF-kB pathway activation by measuring absorbance at 620-655 nm. For the IRF pathway detection, luciferase production was measured using luciferin. Luminescence detection was performed using 50 µL of QUANTI-Luc^{™} (rep-qlc1) solution detection reagent mixed with 10µl of cells supernatant for each well. LPS (100 ng/ml) was used as a positive control to activate the IRF and NF-kB pathways, and dexamethasone (10µg/ml) was used as a negative control to reduce the activation of both pathways. Absorbance and Luminescence measurements were performed using a spectrophotometer.

### Macrophage polarization confirmation by RT-qPCR

Total RNA was extracted from lysed cells using a RNeasy Mini Kit (Qiagen, #74136). The quantity and quality of RNA were measured using a NanoDrop spectrometer. The reverse transcriptase step was performed with 500 ng of each RNA sample using SuperScript II Reverse Transcriptase (Invitrogen, 18064-014) and RNAase (Promega, N251B). The qPCR reaction was performed with PowerUp SYBR Green Master Mix (Applied Biosystems, #A25742) and primers from Eurogentec using the following thermal cycles: one step of 2 min at 95°C and 40 cycles of 15 s at 95°C, followed by 1 min at 60°C. RPLPO was used as a reference gene for normalization. mRNA levels were quantified using the threshold cycle method.

### Alamar blue

Cellular metabolic activity was quantified using the redox indicator resazurin in the form of the Alamar Blue assay. THP-1 Dual-derived macrophages (M0, M1, and M2 phenotypes) were plated at a density of 5x10^5 cells per well in an opaque 24-well plate. Triton-treated cells (0.3%) served as a negative control, while untreated cells acted as a positive control. Nanoparticles were incubated for 24 hours in complete RPMI medium, and a PTT session was applied if necessary. 24 hours later the Alamar Blue reagent (Invitrogen, Carlsbad, CA, USA) was prepared in a 1:10 dilution with RPMI medium devoid of phenol red and supplemented with 1% penicillin/streptomycin. Subsequently, 200 µl of this diluted Alamar Blue solution was added to each well. The incubation period lasted for 3-4 hours at 37°C, ceasing once the solution in the positive control wells turned pink, indicating metabolic activity. Fluorescence quantification was performed using a microplate reader with an excitation of 560 nm and emission of 590 nm. To calculate the cell viability, blank readings from wells with only medium and Alamar Blue were deducted from those of the test wells, and the resultant percentage reduction was computed using the provided formula from the manufacturer, reflecting a direct relationship between fluorescence intensity and cell viability.

### Human cytokine quantification

The MSD cytokine assay (reference K15049D) served as a rapid sandwich immunoassay method for quantifying protein levels in the supernatants. Supernatant samples were securely stored at -80°C until required for cytokine measurements. Leveraging the principles of sandwich enzyme-linked immunosorbent assay (ELISA), the mesoscale multiplexing immunoassay employs commercially available capture. Antibodies were pre-coated on the conductive plates onto which the samples were applied. The concentrations of interferon gamma (IFN-γ), interleukin IL-1β, IL-2, IL-4, IL-10, IL-12p70 and IL-13, were quantified using MESO^{®} QuickPlex SQ 120mm system and data were analyszed using MSD DISCOVERY WORKBENCH analysis software, providing a comprehensive assessment of the cytokine profile.

### Transmission electron microscopy

Cells were fixed with 2 % glutaraldehyde (Sigma Aldrich) in a 0.1 mol/L sodium cacodylate buffer (Sigma Aldrich) for 1 h at ambient temperature, washed with cacodylate buffer (0.2M), and kept in this buffer at 4°C until inclusion. Cell was then contrasted with Oolong Tea Extract (0.5 % in cacodylate buffer), osmium tetroxyde (1 % in cacodylate buffer), and potassium cyanoferrate (1.5 % in cacodylate buffer). They were then gradually dehydrated in ethanol (25 to 100 %). The samples were then substituted gradually in propylene oxide, a mix of propylene oxide and Epon, and finally, embedded in pure Epon (Delta microscopie, Labège, France). Thin sections (70 nm) of samples were collected on 200-mesh grids. Observations were performed on a Hitachi HT7700 microscope.

### Statistical analysis

All results are expressed and plot as mean ± standard error of the mean SEM.Univariate statistical analyses were performed using an unpaired t-test followed by Kolmogorov-Smirnov test in non-parametric conditions. Differences among multiple groups were tested using one-way ANOVA followed by Holm-Sidak post-test or Kruskall-Wallis in non-parametric conditions. Significant differences between groups are expressed as follows: *P < 0.05, **P < 0.01, or ***P < 0.001. Multivariate statistical analysis was performed using principal component analysis (PCA) with GraphPad Prism 10.1.2 software (San Diego, CA, USA). PCA allowed to display automatically unsupervised data clustering.

## Claims

1. A method to control or change the polarization of at least one macrophage associated with a tumor or pathological site (TAM), from being predominantly a TAM with either no polarization (M0) or a polarization i (Mᵢ) before at least one step of the method, to a predominant polarization j (Mⱼ) after at least one step of the method, wherein the method comprises the following steps:
- Step 1 providing i) and ii):
i) At least one nanoparticle which is:
- A magnetosome synthesized by a magnetotactic bacterium or by at least one of its components, preferentially not comprising at least one non-denatured protein and/or lipid and/or LPS and/or DNA and/or RNA originating from or produced by or comprised in a magnetotactic bacterium or at least one of its components,
- A chemically synthesized nanoparticle, having at least one property in common with a magnetosome, preferentially selected in the group consisting of: i) a ferrimagnetic or ferromagnetic behavior or property, , ii) an arrangement in chain or in a geometric figure, iii) a percentage in iron larger than 50, 75, 90, 95 or 99%, , iv) the presence of a core, preferentially ferrimagnetic and/or metallic, and v) the presence of a coating surrounding partly or fully the core,
and/or
- A nanoparticle that has at least one property selected in the group consisting of:
1) It is composed of or comprises at least 2 metals or metal atoms, which are surrounded by or mixed with or bound through or interacting through or assembled by part of or the entire of an organelle or cellular vesicle or endosome or exosome or lysosome or cellular compartment, preferentially intracellular, most preferentially inside an eukaryotic cell, preferentially belonging to or originating from a pathological cell,
and
2) It forms a nanoparticle or a particle with a size smaller than 1000, 100, 50, 20, 10, 5, 2 or 1 nm, preferentially in at least one dimension or cross section of the nanoparticle or particle, through the composition of at least 1 or 2 metals or metal atoms, which is/are surrounded by or mixed with or bound through or interacting through or assembled by or transformed into a nanoparticle or particle that has at least one property that differs from that of a single atom, or made crystallized or magnetic, preferentially diamagnetic, paramagnetic, superparamagnetic, ferromagnetic or ferrimagnetic, by part of or the entire of an organelle or cellular vesicle or endosome or exosome or lysosome or cellular compartment, preferentially intracellular, most preferentially inside an eukaryotic cell, preferentially belonging to or originating from a pathological cell,
and
ii) At least one TAM, having at least one property selected in the group consisting of: i) it is a TAM with either no polarization (M0) or with polarization i (Mⱼ), ii) at least two TAM forming an assembly of TAM with predominantly polarization(s) (M0) and/or (Mᵢ) and/or with a minority of or no polarization (Mⱼ), ii) it is comprised in or is extracted from or originates from or is produced by or is differentiated in or by a pathological site or tumor or body part to be treated or its/their environment or microenvironment, partly or fully, iii) it is differentiated from a mast or stem or un-differentiated cell, iv) it is moving or in movement or is migrating, and v) it is provided, collected, harvested, detected, differentiated, isolated, activated, polarized;
- Step 2 comprising assembling and/or mixing, and/or conjugating, and/or complexing at least one nanoparticle of step 1 with at least one TAM of step 1 by:
i) Administering and/or assembling and/or mixing and/or conjugating and/or complexing the at least one nanoparticle of step 1 to or with a body part comprising at least one TAM of step 1;
ii) Bringing together the at least one TAM of step 1 and at least of nanoparticle of step 1;
iii) Internalizing at least one nanoparticle of step 1 in at least one TAM of step 1;
iv) Externalizing or expelling at least one nanoparticle of step 1 from at least one TAM of step 1; and/or
- Associating and/or assembling and/or mixing and/or conjugating and/or complexing at least one nanoparticle of **step 1** and at least of TAM of **step 1;**
- **Step 3** comprising exposing or having exposed or letting exposed, preferentially by the body part, or at least one compound or radiation in or outside the body part, the nanoparticle(s) and/or TAM(s) of **step 1** and/or assembly, and/or mixture, and/or conjugate, and/or complex and/or composite and/or compound and/or blend of nanoparticle(s) and/or TAM(s) of **step 2** and/or its/their environment to at least one of:
a. a variation of at least one radiation parameter;
b. a movement and/or displacement;
c. a temperature gradient;
d. a pH gradient;
and
e. a composition gradient;
wherein the environment of the nanoparticle(s) and/or TAM(s) is at least one region or volume comprising the nanoparticle(s) and/or TAM,
- Optionally **step 4** of:
i) Stopping the application of radiation after **step 3;**
or
ii) Exposing the assembly or mixture or conjugate or complex, or compound, or composite, or blend, resulting from **step 3** of the method to a different source of radiation than that applied in **step 3** or to the same source of radiation as that applied in **step 3,** but with at least one of its parameter with a different value than in **step 3.**
- Optionally **step 5** of detecting or measuring or differentiating at least one cell, being a monocyte or TAM (M0, M1, or M2), or property associated with the at least one state of the cell, such as the morphology, size, directional movement, production of type, size, number of immune entities or the cell,
- Optionally **step 6** of differentiating at least one monocyte into an unpolarized TAM by:
i) associating at least one monocyte with at least one entity that differentiates the monocyte into a TAM, and/or
ii) associating at least one nanoparticle of step **1** with at least one monocyte, and of exposing the assembly of at least one nanoparticle and monocyte to at least one radiation,
wherein Mᵢ = M₁ and Mⱼ = M₂ or Mᵢ = M₂ and Mⱼ = M₁.

2. The method according to claim **1,** wherein at least two of its step, comprise at least one lapse of time and/or one location, during or in which part of or all of the at least two steps occur simultaneously or concomitantly in time and/or space.

3. The method according to claim **1,** wherein at least two of its step, comprise at least one lapse of time and/or one location, during or in which par of or all of the at least two steps occur non-simultaneously or non-concomitantly in time and/or space.

4. The method according to claim **1,** wherein M₂ has at least one property selected in the group consisting of:
a. It is anti-inflammatory;
b. It is or is associated with or comprises partly or fully tumor-promoting, pathogen-promoting, angiogenesis-promoting, neovascularization-promoting, stroma-activating, tumor or pathological or tumor site or environment or microenvironment remodeling, growth or protection, or increasing or activating or triggering pro-tumor or pro-pathological site inflammatory reactions or immune entity(ies) of first category or decreasing or de-activating anti-tumor or anti-tumor-site inflammatory reaction(s) or immune entity(ies) of second category,
c. it comprise(s) or generate(s) or is/are associated with or activate(s) or is activated by or is polarized by or originate(s) from:
- at least one immune entity of first category;
- a larger number or size or concentration or different types of immune entity(ies) of first category than for M1 or in the presence than in the absence of at least one nanoparticle, preferentially exposed to at least one radiation, preferentially by a factor of at least 0, 0.1, 1, 2 or 5;
- a larger number or size or concentration or different immune entity(ies) of first than second category, preferentially by a factor of at least 0, 0.1, 1, 2 or 5;
- a larger number or concentration of immune entity(ies) of first category than 10²⁰, 10⁵, 10³, 100, 50, 20, 10, 5, 2 or 1,
- a size of immune entity(ies) of first category that is larger than 10²⁰, 10⁵, 10³, 100, 50, 20, 10, 5, 2 or 1 nm,
- a number of different types of immune entity(s) of first category that is larger than 10²⁰, 10⁵, 10³, 100, 50, 20, 10, 5, 2 or 1,
- at least one immune entity of first category that changes the polarization at least one TAM from an un-polarized state to M2 or from M1 to M2.

5. The method according to claim **1,** wherein M₁ has at least one property selected in the group consisting of:
i) it is pro-inflammatory;
ii) it is or is associated with or comprises partly or fully pathogen or tumor resistant preferentially immune entity(ies), anti-angiogenic, acting against the development of the neovasculature of a tumor or pathological site, anti-pathogenic or anti-tumoral or increasing or activating or triggering anti-tumor or anti-pathological site immune reaction, or deactivating the tumor or pathological site or its environment or micro-environment, or decreasing or de-activating pro-pathological site inflammatory reactions or immune entity(ies) of second category,
iii) it comprises or generates or is associated with or activate(s) or is activated by or is polarized by or originate(s) from:
- at least one immune entity of second category;
- a larger number or size or concentration or different types of immune entity(ies) of second category than for M2 or in the presence than in the absence of at least one nanoparticle, preferentially exposed to at least one radiation;
- a larger number or size or concentration or different types of immune entity(ies) of second category than for M2;
- a larger number or concentration of immune entity(ies) of second category than 10²⁰, 10⁵, 10³, 100, 50, 20, 10, 5, 2 or 1;
- a size of immune entity(ies) of second category that is larger than 10²⁰, 10⁵, 103, 100, 50, 20, 10, 5, 2 or 1 nm,
- a number of different types of immune entity(s) of second category that is larger than 10²⁰, 10⁵, 10³, 100, 50, 20, 10, 5, 2 or 1,
- at least one immune entity of second category that changes the polarization at least one TAM from an un-polarized state to M1 or from M2 to M1,

6. The method according to claim **1,** which is reversible, wherein at least one TAM first changes its polarization from being predominantly a TAM with either no polarization (M0) and/or a polarization i (Mi) before the method, to a predominant j polarization (Mj) after one step of the method, and second changes back its polarization from being predominantly polarized into Mj before at least one step of the method to being predominantly with no polarization (M0) and/or with a polarization i (Mi) after one step of the method.

7. The method according to claim **1,** wherein the radiation is not a laser or magnetic field or an electromagnetic wave.

8. The method according to claim **1,** wherein the radiation is selected in the group consisting of: i) wave(s) such as electromagnetic, sound, acoustic or particle wave(s), ii) electromagnetic radiation, iii) acoustic radiation forces, iv) radiation forces, v) radiation pressures, vi) irradiation, vii) a magnetic or electric field, viii) light not produced by a laser, ix) light produced by a lamp, x) light emitted at a single wavelength, xi) light emitted at multiple wavelengths, xii) a ionizing radiation, xiii) microwave, xiv) radiofrequencies, xv) alpha particles or radiation, xvi) beta particles or radiation, xvii) gamma particles or radiation, xviii) X-ray particles or radiation, xix) neutron particles or radiation, xx) proton particles or radiation, xxi) electron particles or radiation, xxii) ion particles or radiation, xiii) neutrino particles or radiation, xiv) muon particles or radiation, xv) meson particles or radiation, xvi) photon particles or radiation, xvii) infra-sounds, xviii) sounds, xix) ultra-sounds, xx) hyper sounds. xxi) heating radiation, xxii) a cooling radiation, xxiii) a ionizing radiation, xxii) a light not being a laser, xxiii) an alternating magnetic field, xxiv) a uniform radiation, and xxv) radiation with a gradient or variation, preferentially spatial and/or temporal.

9. The method according to claim 1, wherein part or the entire of the at least one or two nanoparticle(s) has or have at least one property selected in the group consisting of:
1) Part or the entire of the at least one nanoparticle comprises a metallic, crystalline, plasmonic, super-paramagnetic, ferromagnetic and/or ferrimagnetic composition or property;
2) Part or the entire of the at least two nanoparticles form an assembly within a geometric figure such as a chain, circle, rectangle, or square,
3) Part or the entire of the at least one nanoparticle does not comprise at least one photosensitizer or ROS or radical species generator or radiation-generator, radiation-amplifier, or photogenerator;
4) Part or the entire of the at least one nanoparticle differentiates or polarizes at least one cell or TAM; and
5) Part or the entire of the at last one nanoparticle comprises at least one compound such as at least one or two metal(s) and/or part or the entire of an organelle or cellular vesicle or endosome or exosome or lysosome or cellular compartment.

10. At least one TAM polarized in a M1 TAM or at least one TAM polarized in a M2 TAM, as obtained by at least one step of the method according to claim 1, to destroy or inactivate a pathological site such as a tumor or bacterial site or stimulate at least one immune response or immune entity of first category, or to defend a host or individual.
